(19) **Europäisches Patentamt**
**European Patent Office**
**Office européen des brevets**

(11) **EP 2 767 275 A1**

(12) **EUROPEAN PATENT APPLICATION**

(43) Date of publication:
**20.08.2014 Bulletin 2014/34**

(21) Application number: **13155069.1**

(22) Date of filing: **13.02.2013**

(51) Int Cl.:
*A61K 31/135* (2006.01)   *A61K 31/4515* (2006.01)
*A61K 31/5415* (2006.01)   *A61P 31/06* (2006.01)
*G01N 33/569* (2006.01)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **Max-Planck-Gesellschaft zur Förderung der Wissenschaften e.V.**
**80539 München (DE)**

(72) Inventors:
• **Sundaramurthy, Varadharajan**
**01307 Dresden (DE)**

• **Barsacchi, Rico**
**01277 Dresden (DE)**
• **Zerial, Marino**
**01309 Dresden (DE)**
• **Bickle, Marc**
**01307 Dresden (DE)**
• **Kalaitzidis, Ioannis**
**01277 Dresden (DE)**
• **Samusik, Nikolay**
**01099 Dresden (DE)**

(74) Representative: **Vossius & Partner**
**Siebertstrasse 4**
**81675 München (DE)**

(54) **Nortriptyline, haloperidol or prochlorperazine edisylate for use in the treatment of tuberculosis as well as two screening methods**

(57) The present invention relates to a compound capable of killing mycobacteria by activating host cellular mechanisms for use in treating tuberculosis, wherein the compound is selected from the group consisting of nortriptyline, haloperidol and prochlorperazine edisylate (PE). Furthermore, the present invention relates to a pharmaceutical composition comprising the compound of the invention, and optionally a pharmaceutically acceptable carrier. The invention further relates to a method of treating tuberculosis as well as a method of identifying a lead compound for the treatment of tuberculosis.

EP 2 767 275 A1

**Description**

The present invention relates to a compound capable of killing mycobacteria by activating host cellular mechanisms for use in treating tuberculosis, wherein the compound is selected from the group consisting of nortriptyline, haloperidol and prochlorperazine edisylate (PE). Furthermore, the present invention relates to a pharmaceutical composition comprising the compound of the invention, and optionally a pharmaceutically acceptable carrier. The invention further relates to a method of treating tuberculosis as well as a method of identifying a lead compound for the treatment of tuberculosis.

In this specification, a number of documents including patent applications and manufacturer's manuals are cited. The disclosure of these documents, while not considered relevant for the patentability of this invention, is herewith incorporated by reference in its entirety. More specifically, all referenced documents are incorporated by reference to the same extent as if each individual document was specifically and individually indicated to be incorporated by reference.

Tuberculosis (TB) is a common, and in many cases lethal, contagious bacterial infection caused by various strains of mycobacteria, usually *Mycobacterium tuberculosis (M. tuberculosis)*. The disease involves the lungs, but may also spread to other organs. Primary TB infection typically occurs by breathing in air droplets from a cough or sneeze of an infected person. Normally, most people recover from primary TB infection without further evidence of the disease, but the infection may stay inactive (dormant) for many years. In some people, the disease can reactivate or the disease may become active within weeks after the primary infection. In particular elderly people and infants but also people with a weakened immune system, e.g. due to AIDS, chemotherapy, diabetes or certain medications are at higher risk for active TB. Established treatment regimes require the administration of multiple antibiotics over a long period of time, however, antibiotic resistance increasingly presents a problem in multiple drug-resistant tuberculosis (MDR-TB) infections.

TB is characterized by an extensive array of interactions between mycobacteria and the host. To maintain their intracellular niche, mycobacteria influence a variety of fundamental host cell processes including apoptosis (Behar et al., 2010; Chen et al., 2006; Keane et al., 2000), autophagy (Gutierrez et al., 2004; Kumar et al., 2010), trafficking (Armstrong and Hart, 1971; Russell, 2001) and signalling (Koul et al., 2004). Therefore, a pharmacological modulation of the host cell mechanisms that are hijacked by the bacteria could, on the one hand, be used to explore the processes altered during the infection and, on the other hand, lead to development of novel classes of anti-TB compounds. However, exploiting host processes to fight against infectious diseases is currently an under-explored approach. Yet, it presents several advantages. First, the host processes exploited by pathogens are well studied from both a mechanistic and physiological perspective (Dyer et al., 2008). Second, it could better prevent the emergence of drug resistant strains (Schwegmann and Brombacher, 2008). Additionally, critical host cellular responses are conserved across diverse mycobacterial strains, including drug-resistant ones (Kumar et al., 2010).

Thus, despite the fact that a lot of effort has been invested into identifying antibacterial compounds for the treatment of tuberculosis, there is still a need to identify further compounds. Preferably, such compounds should target mycobacteria via mechanisms different from the mechanisms of action of established tuberculosis drugs. This need is addressed by the provision of the embodiments characterized in the claims.

Accordingly, the present invention relates to a compound capable of killing mycobacteria by activating host cellular mechanisms for use in treating tuberculosis, wherein the compound is selected from the group consisting of 3-(10,11-dihydro-5H-dibenzo[a,d] cyclohepten-5-ylidene)- N-methyl-1-propanamine (nortriptyline), 4-[4-(4-chlorophenyl)-4-hydroxy-1-piperidyl]-1-(4-fluorophenyl)-butan-1-one (haloperidol) and 2-chloro-10-[3-(4-methyl-1-piperazinyl)propyl]-10*H*-phenothiazine (prochlorperazine edisylate;PE).

In accordance with the present invention, compounds for the treatment of tuberculosis are provided that were not previously known to be suitable as active agents for the therapy of tuberculosis. These compounds are selected from the group consisting of nortriptyline, haloperidol and prochlorperazine edisylate (PE), all three of which have been shown in the appended examples as being capable of killing mycobacteria by activating host cellular mechanisms, in particular by restoring mycobacteria-arrested phagosome maturation.

Nortriptyline is a tricyclic antidepressant, the systematic (IUPAC) name of which is 3-(10,11-dihydro-5H-dibenzo[a,d] cyclohepten-5-ylidene)- N-methyl-1-propanamine. It is also marketed as the hydrochloride salt under the trade names Sensoval, Aventyl, Pamelor, Norpress, Allegron, Noritren and Nortrilen. Nortriptyline is used to treat symptoms of depression as well as childhood nocturnal enuresis. Sometimes it is also used for the treatment of chronic illnesses such as chronic fatigue syndrome, chronic pain and migraine, and labile affect in some neurological conditions.

Haloperidol is a compound from the group of medications called conventional antipsychotics and has the systematic (IUPAC) name 4-[4-(4-chlorophenyl)-4-hydroxy-1-piperidyl]-1-(4-fluorophenyl)-butan-1-one. Haloperidol works by decreasing abnormal excitement in the brain and is used to treat psychotic disorders, but also to control motor tics and verbal tics in adults and children who have Tourette's disorder. Haloperidol is further used to treat severe behavioural problems such as explosive, aggressive behaviour or hyperactivity in children who cannot be treated with psychotherapy or with other medications. Haloperidol is sold under the trade names Aloperidin, Bioperidolo, Brotopon, Dozic, Duraperidol (Germany), Einalon S, Eukystol, Haldol (common tradename in the US and UK), Halosten, Keselan, Linton, Peluces, Serenace, Serenase, and Sigaperidol.

Prochlorperazine edisylate (PE) has the systematic (IUPAC) name 2-chloro-10-[3-(4-methyl-1-piperazinyl)propyl]-10H-phenothiazine. It is also known under the names Compazine, Stemzine, Buccastem, Stemetil and Phenotil. PE is a dopamine (D2) receptor antagonist that is used for the antiemetic treatment of nausea and vertigo and is also a highly potent typical antipsychotic. It is also used to treat migraine headaches.

Nortriptyline, haloperidol and prochlorperazine edisylate (PE) are commercially available and can for example be purchased from Sigma. The chemical synthesis of these compounds is well described, e.g. the synthesis of haloperidol has been described in US 3,438,991, GB 895309 or BE577977; the synthesis of prochlorperazine edisylate has been described in US patent 2,902,484 and the synthesis of Nortriptyline has been described in Peters and Hennion, J. Med. Chem., 1964, 7 (3), pp 390-392.

In accordance with the present invention, novel anti-mycobacterial properties for three existing drugs were revealed. Nortriptyline is known in the art and is employed as a second generation tricyclic antidepressant while PE and haloperidol, which are also well known in the art, are used as anti-psychotics. Neither of these compounds was previously known in the art to be suitable for the treatment of tuberculosis. The finding that three neuro-modulators are endowed with membrane trafficking and anti-mycobacterial activity suggests that compounds can exert diverse biological effects depending on the cellular context analyzed. Such influences are typically neglected in target-based screenings. Repurposing existing drugs for new applications could thus open new avenues and lead to rapid therapeutic advances (Huang et al., 2011).

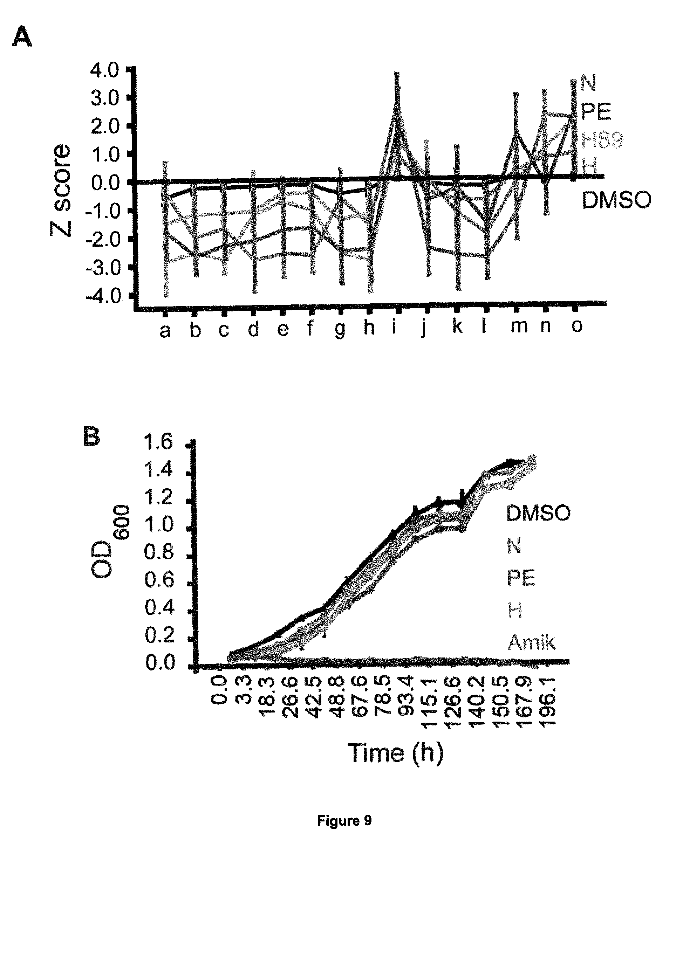

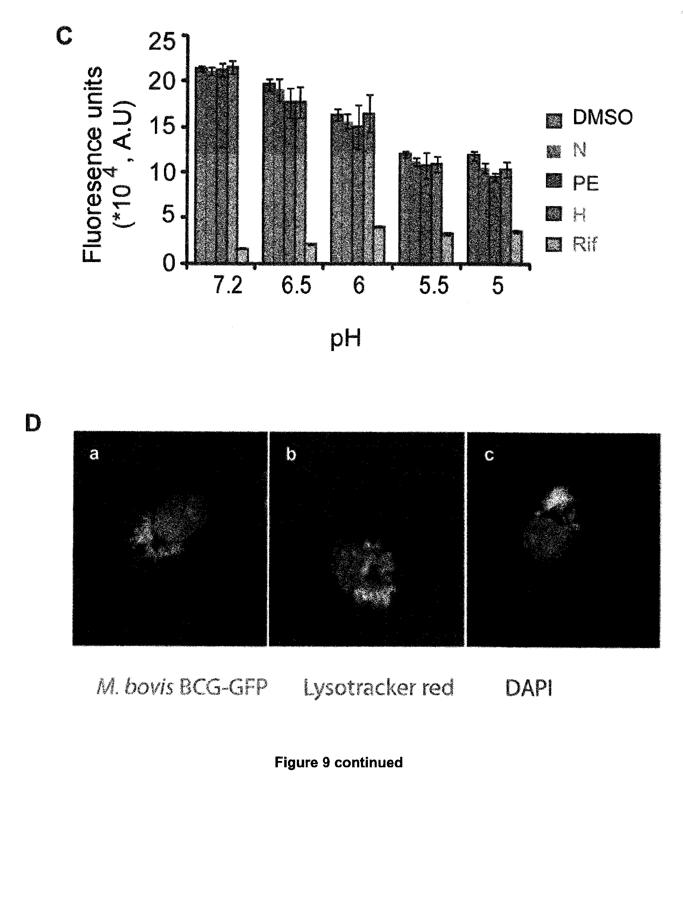

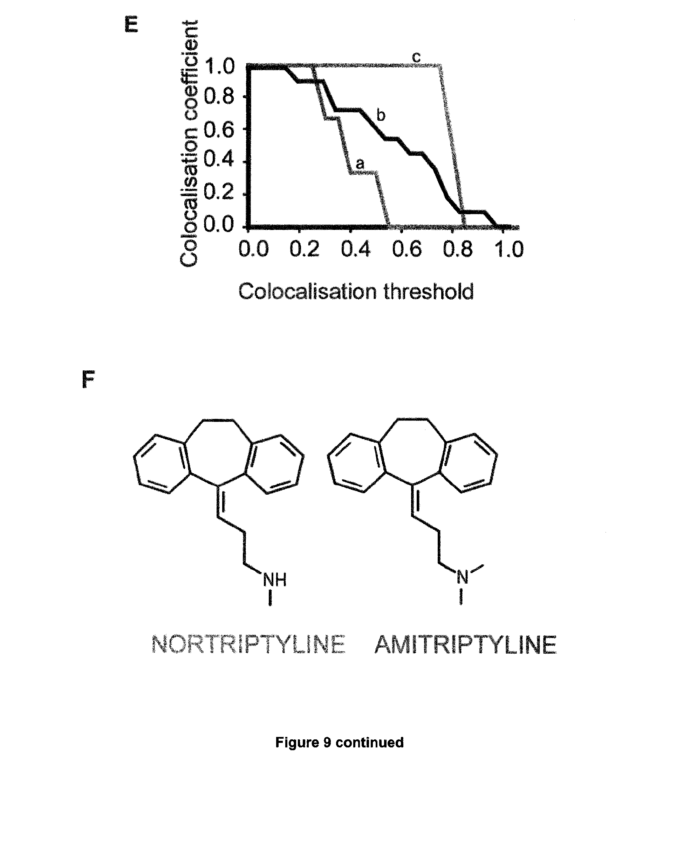

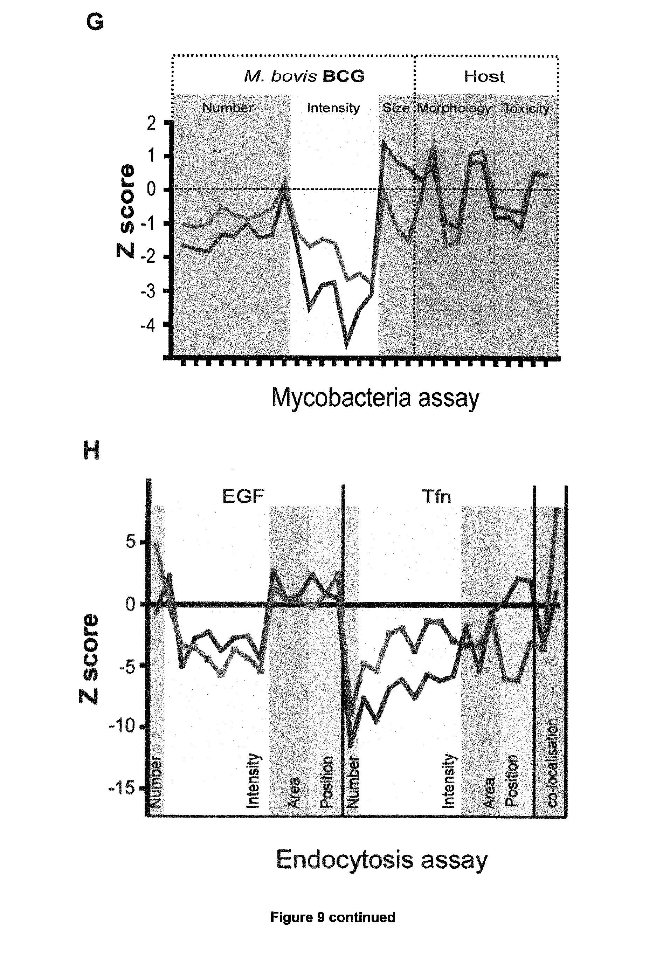

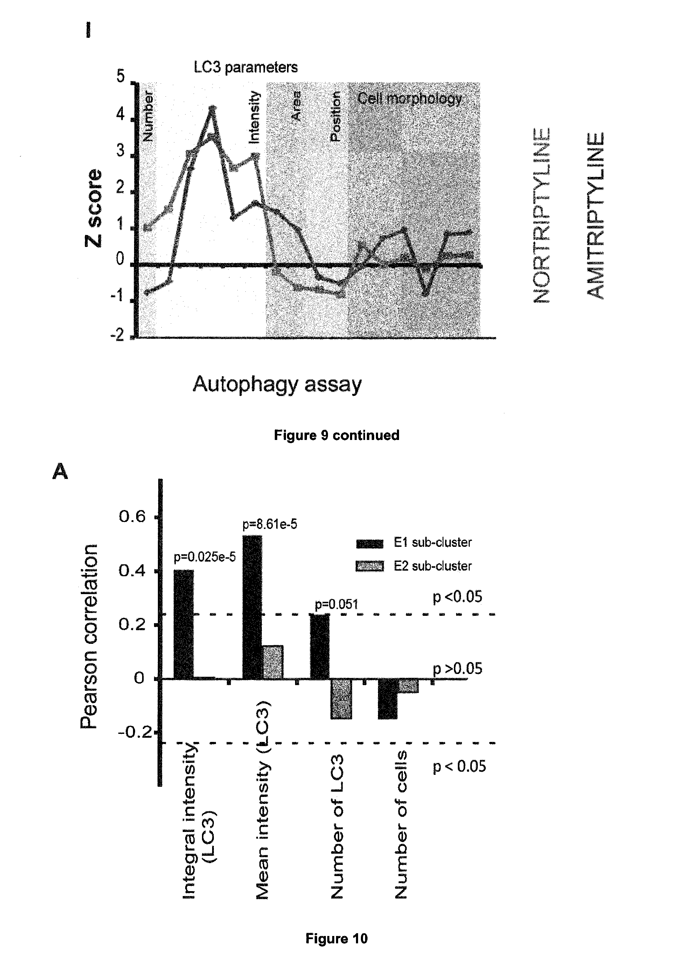

As detailed herein above, upon infection, mycobacteria alter host cell mechanisms in order to survive and proliferate. One of these host cell mechanisms is cellular phagosome maturation, which is blocked in the presence of mycobacteria (Armstrong and Hart, 1971; Pieters, 2008; Russell, 2001). Here, novel pharmacological modulators of host cellular processes were identified that trigger killing of intracellular mycobacteria via restoring of the host cell phagosome maturation. Accordingly, these compounds act on (and restore) host cell mechanisms instead of targeting and killing mycobacteria directly, as is shown in the appended examples; see e.g. Figure 9, in particular Figures 9b showing the growth curve of mycobacteria in the presence of the compounds as well as Figure 9c showing an alamar blue assay for mycobacterial growth in the presence of compounds at different acidic pH. These data therefore confirm that the compounds identified herein influence intracellular mycobacterial survival by modulating cellular processes at the host-pathogen interface rather than acting directly on the bacteria.

Accordingly, in a preferred embodiment, the present invention relates to a compound selected from the group consisting of nortriptyline, haloperidol and prochlorperazine edisylate (PE) for use in treating tuberculosis, wherein the compound is capable of restoring mycobacteria-arrested phagosome maturation.

The present invention further relates to a pharmaceutical composition comprising the compound of the invention, and optionally a pharmaceutically acceptable carrier. Preferably, the pharmaceutical composition comprises the compound of the invention as the single active agent.

In accordance with the present invention, the term "pharmaceutical composition" relates to a composition for administration to a patient, preferably a human patient. The pharmaceutical composition of the invention comprises at least one of the compounds of the invention, i.e. one or more of nortriptyline, haloperidol and prochlorperazine edisylate (PE), i.e. either nortriptyline, haloperidol or prochlorperazine edisylate (PE) alone or a combination of either nortriptyline with haloperidol, nortriptyline with prochlorperazine edisylate (PE) or haloperidol with prochlorperazine edisylate (PE) or a combination of all three.

The pharmaceutical composition of the present invention may, optionally and additionally, comprise a pharmaceutically acceptable carrier. By "pharmaceutically acceptable carrier" is meant a non-toxic solid, semisolid or liquid filler, diluent, excipient, encapsulating material or formulation auxiliary of any type. Examples of suitable pharmaceutical carriers are well known in the art and include sodium chloride solutions, phosphate buffered sodium chloride solutions, water, emulsions, such as oil/water emulsions, sterile solutions, organic solvents etc. Conventional excipients include binding agents, fillers, lubricants and various types of wetting agents. Preferably the carrier is a parenteral carrier, more preferably a solution that is isotonic with the blood of the recipient. The term "parenteral" as used herein refers to modes of administration, which include for example intravenous injection. These modes of administration, together with intranasal, oral, buccal, sublingual or intrabronchial administration, are preferred modes of administration in accordance with the present invention. Most preferred modes of administration in accordance with the present invention are oral and intravenous administration. The carrier optionally contains minor amounts of additives such as substances that enhance isotonicity and chemical stability. Such materials are non-toxic to recipients at the dosages and concentrations employed, and include buffers such as phosphate, citrate, succinate, acetic acid, and other organic acids or their salts; antioxidants such as ascorbic acid; low molecular weight (less than about ten residues) (poly)peptides, e.g., polyarginine or tripeptides; proteins, such as serum albumin, gelatin, or immunoglobulins; hydrophilic polymers such as polyvinylpyrrolidone; amino acids, such as glycine, glutamic acid, aspartic acid, or arginine; monosaccharides, disaccharides, and other carbohydrates including cellulose or its derivatives, glucose, mannose, or dextrins; chelating agents such as EDTA; sugar alcohols such as mannitol or sorbitol; counterions such as sodium; and/or nonionic surfactants such as polysorbates, poloxamers, or PEG.

Compositions comprising such carriers can be formulated by well known conventional methods. Generally, the formulations are prepared by contacting the components of the pharmaceutical composition uniformly and intimately with liquid carriers or finely divided solid carriers or both. Then, if necessary, the product is shaped into the desired formulation.

The pharmaceutical compositions can be administered to the subject at a suitable dose. The dosage regimen will be determined by the attending physician and clinical factors. As is well known in the medical arts, dosages for any one patient depend upon many factors, including the patient's size, body surface area, age, the particular compound to be administered, sex, time and route of administration, general health, and other drugs being administered concurrently. The therapeutically effective amount for a given situation will readily be determined by routine experimentation and is within the skills and judgment of the ordinary clinician or physician. The pharmaceutical composition may be for administration once or for a regular administration over a prolonged period of time. Generally, the administration of the pharmaceutical composition should be in the range of for example 10μg/kg of body weight to 2g/kg of body weight for a single dose. However, a more preferred dosage might be in the range of 100μg /kg to 1.5g/kg of body weight, even more preferably 1 mg/kg to 1 g/kg of body weight and even more preferably 5mg/kg to 500mg/kg of body weight for a single dose. The typically recommended dose for Haloperidol is between 0.5 to 20 mg/day (Oosthuizen et al. (2001). Journal of Psychopharmacology 15 (4): 251-5.), for Nortriptyline between 10 to 150 mg/day (according to the world wide web at drugs.com/dosage/nortriptyline.html) and for Prochlorperazine edisylate between 5 to 40 mg/day (according to the world wide web at drugs.com/dosage/prochlorperazine.html). Adjustment of these dosages in case of combination of the agent of the present invention with further pharmaceutically active compounds, such as those described elsewhere herein, is within the skills and judgment of the ordinary clinician or physician.

The components of the pharmaceutical composition to be used for therapeutic administration must be sterile. Sterility is readily accomplished by filtration through sterile filtration membranes (e.g., 0.2 micron membranes), however, the present invention is not confined to this method of obtaining sterility. The pharmaceutical composition of the invention is formulated in accordance with national laws and regulatory requirements according to GMP standards.

The components of the pharmaceutical composition ordinarily will be stored in unit or multi-dose containers, for example, sealed ampoules or vials, as an aqueous solution or as a lyophilized formulation for reconstitution. As an example of a lyophilized formulation, 10-ml vials are filled with 5 ml of sterile-filtered 1% (w/v) aqueous solution, and the resulting mixture is lyophilized. The infusion solution is prepared, for example, by reconstituting the lyophilized compound(s) using bacteriostatic water-for-injection. Preservatives and other additives may also be present such as, for example, antimicrobials, anti oxidants, chelating agents, and inert gases and the like.

The pharmaceutical composition may comprise further agents depending on the intended use of the pharmaceutical composition. For example, such further agents may be selected from agents known in the art to be suitable in the treatment of tuberculosis (as described below) or in the treatment of side effects associated with tuberculosis. Since the pharmaceutical composition of the present invention relies on the above mentioned active compounds, it is preferred that these further agents are only used as a supplement, i.e. at a reduced dose as compared to the recommended dose when used as the only drug, so as to e.g. reduce side effects conferred by the further agents.

In a preferred embodiment of the invention, the compound or the pharmaceutical composition have admixed thereto or associated in a separate container one or more agents selected from the group consisting of antibiotic compounds, arginine, vitamin D and IFN-γ.

Antibiotic compounds, in accordance with the present invention, are antimicrobial compounds, i.e. compounds capable of killing microbes or inhibiting their growth. Preferably, the antibiotic compounds are antibacterial compounds. Non-limiting examples of antibiotics suitable for use in accordance with this preferred embodiment include existing first line anti-tuberculosis drugs such as isoniazid, ethambutol, rifampin, pyrazinamide; second line anti-tuberculosis drugs including aminoglycosides, such as e.g., amikacin (AMK), kanamycin (KM); polypeptides such as e.g., capreomycin, viomycin, enviomycin; fluoroquinolones such as e.g., ciprofloxacin (CIP), levofloxacin, moxifloxacin (MXF); thioamides such as e.g. ethionamide, prothionamide; cycloserine such as e.g., closerin, terizidone as well as third-line anti- tuberculosis compounds like rifabutin macrolides such as e.g. clarithromycin (CLR); linezolid (LZD); thioacetazone (T); thioridazine; arginine or R207910. Combination of the compound(s) of the present invention with antibiotic compounds is expected to provide the advantage of increasing positive therapeutic effects of different treatment approaches while minimizing the unwanted side effects by lowering the dose or treatment duration of each individual treatment approach.

Arginine has been shown to be an effective adjuvant in the treatment against tuberculosis, possibly by enhancing NO levels (Schön et al. (2003) Eur Respir J 2003;21:483-8). Later work by the same group suggested that this effect may be restricted to a sub-group of patients who are also HIV positive (Schon et al. (2011), Tuberculosis (Edinb). Sep;91 (5):370-7). Thus, arginine may be of particular interest in a combination therapy for this patient group.

Vitamin D, in accordance with the present invention, refers to the Vitamin produced by sunlight. Tuberculosis patients given high doses of Vitamin D together with anti-tuberculosis drugs showed accelerated resolution of inflammatory responses as well as smear conversion in a randomized clinical trial (Coussens et al. (2012), PNAS 109(38):15449-54).

Interferon-gamma (IFN-γ), the only member of the type II class of interferons, is a dimerized soluble cytokine that is critical for innate and adaptive immunity against viral and intracellular bacterial infections and for tumor control. The amino acid sequence of IFN-γ can be found in the UniProt database under P01579 or in the NCBI database under NP_000610.2. IFN-γ is an established physiological inducer of autophagy (Gutierrez et al., 2004; Matsuzawa et al., 2012) and, as is shown in Example 6, Nortriptyline synergized strongly with IFN-γ, supporting its role in autophagy induction. Additive or even synergistic activities of the pharmaceutical compounds with such antibiotic compounds, arginine, vitamin D and/or IFN-γ may be exploited to yield more potent pharmacological interventions.

The present invention also relates to a method of treating tuberculosis comprising administering a pharmaceutically effective amount of a compound capable of killing mycobacteria by activating host cellular mechanisms to a subject in need thereof, wherein the compound is selected from the group consisting of nortriptyline, haloperidol and prochlorperazine edisylate (PE).

All the definitions as well as the preferred embodiments provided herein above with regard to the compound or the pharmaceutical composition of the invention apply *mutatis mutandis* also to the embodiments relating to the method of treating tuberculosis as outlined above.

For example, in a preferred embodiment of this method of the invention, the method further comprises the administration of one or more agents selected from the group consisting of antibiotic compounds, arginine, vitamin D and IFN-γ.

The pharmaceutically effective amount of the respective compound may be formulated as a pharmaceutical composition, as defined above.

In another preferred embodiment of the method of the invention, the subject is a mammal. More preferably, the subject is a human subject.

The present invention further relates to a method of identifying a lead compound for the treatment of intracellular bacterial infections, the method comprising the steps of:

(a) incubating cells infected with bacteria with a test compound;
(b) staining the cells with (a) detectable marker probe or introducing into the cell a detectable marker probe;
(c) obtaining a multi-parametric phenotypic profile of host cell parameters and bacterial parameters; and
(d) clustering the multi-parametric phenotypic profile by using a clustering algorithm based on density estimate and modified Gabriel graph neighbourhood, which comprises:

(i) computing multi-dimensional Gabriel neighbourhood graph over dataset, where nodes correspond to phenotypic profiles and edges determine the neighbourhood of nodes;
(ii) filtering out spurious as well as 'long-range' edges from the Gabriel graph;
(iii) computing the density estimate for each node of the Gabriel graph using a kernel function, wherein the kernel function is chosen according to the selected multi-parametric profile dissimilarity measure;
(iv) converting the modified Gabriel graph into weighted directed acyclic graph (WDAG) by assigning directionality to edges such that their point from nodes with lower density estimate to nodes with higher density estimate and edge weights are set proportional to the distances (dissimilarity) between neighbouring nodes, or, in the ambiguous case of equal density estimates of two neighbour nodes, one node is randomly chosen and a small arbitrary value (0.0000001) is added to its density estimate;
(v) marking the nodes of the graph with no out-coming edges, i.e. nodes having local maxima of density, as cluster centers; and
(vi) assigning the remaining nodes to the cluster centers based on the shortest weighted path in the directed neighbourhood graph;

wherein an alteration in bacterial parameters and a lack or substantial lack of alteration of host cell parameters in the presence of the test compound is indicative of a compound suitable as a lead compound for the treatment of the intracellular bacterial infection.

Preferably, the intracellular bacterial infection is a mycobacterial infection, even more preferably *M. tuberculosis.*

In accordance with the present invention, the term "lead compound", which is used herein interchangeably with the term "hit compound", refers to a compound discovered by the method of the invention as being suitable for the treatment of an intracellular bacterial infection and which can e.g. be further optimized, in particular to be pharmaceutically more acceptable.

Essentially any compound can be assayed as a test compound in this method according to the present invention. Compounds include small molecules, such as organic or inorganic molecules. Organic molecules relate or belong to the class of chemical compounds having a carbon basis, the carbon atoms linked together by carbon-carbon bonds, including biological entities such as e.g. proteins, sugars, nucleic acids, lipids. The original definition of the term organic related to the source of chemical compounds, with organic compounds being those carbon-containing compounds obtained from plant or animal or microbial sources. Organic compounds can be natural or synthetic. Small organic molecules preferably have a molecular weight of about 500 Da or below. Inorganic compounds are derived from mineral sources and include all compounds without carbon atoms (except carbon dioxide, carbon monoxide and carbonates). There are many suppliers of such compounds, including Sigma (St. Louis, Mo.), Aldrich (St. Louis, Mo.), Sigma-Aldrich (St. Louis, Mo.), Fluka Chemika-Biochemica Analytika (Buchs, Switzerland), for example. In addition, compounds to be analysed may be synthesized by methods known in the art, or obtained from fractionating extracts obtained from natural sources, such as e.g. plants. The compounds to be tested as potential agents suitable for the treatment of tuberculosis can be dissolved in aqueous or organic, such as e.g., DMSO-based, solutions.

Test compounds may be comprised in compound libraries of diverse or structurally similar compounds (e.g, combinatorial chemistry synthesized libraries). A plurality of test compounds in a library can be assayed simultaneously. Optionally, test compounds derived from different libraries can be pooled for simultaneous evaluation. A library can comprise a random collection of molecules. Alternatively, a library can comprise a collection of molecules having a bias for a particular sequence, structure, or conformation. Methods for preparing libraries containing diverse populations of various types of molecules are known in the art (Brenk, R. et al., Lessons Learnt from Assembling Screening Libraries for Drug Discovery for Neglected Diseases, ChemMedChem 2008, 3, 435 - 444, Quinn J.R. et al., Developing a Drug-like Natural Product Library, J. Nat. Prod. 2008, 71, 464-468). Numerous libraries are also commercially available.

In a first step of this method of the invention (i.e. step (a) recited above), cells that are infected with bacteria, preferably mycobacteria, are incubated with said test compound(s). Cells infected with bacteria may be obtained in a preceding step by infecting cells with a bacterial strain, such as e.g. a mycobacteria strain using methods well known in the art. For example, cells can be infected by incubating them with cell culture medium that contains a defined amount of bacteria. After a specified period of time (such as e.g. 1 hour), the bacteria containing media are removed and cells are washed extensively to remove the remaining bacteria. A non-pathogenic *M.bovis* expressing GFP (BCG-GFP) can for example be employed, as described in the appended examples. Alternatively, pathogenic *M. tuberculosis* may be employed, such as e.g. the clinical strains CS1 or CS2, as also described in the appended examples with regard to the validation experiments. Further mycobacteria suitable for use in the methods of the present invention include, without being limiting *M. marinum, M. ulcerans, M. avium, M. africanum, M. fortuitum, M. smegmatis.*

The cells suitable for use in the method of the present invention include all cells typically infected by bacteria, and in particular mycobacteria, such as e.g. macrophages, monocytes or dendritic cells. The cells may be primary cells, i.e. cells directly obtained from an organism, as well as stable, immortalized cell lines.

Incubation with the test compound is performed under cell culture conditions suitable for the respective cell type. Such cell culture conditions are known in the art. Preferably, the cells are incubated with the test compound for at least 6 to 72 hours, such as e.g. 12 to 62 hours, more preferably 24 to 52 hours and most preferably for about 48 hours, as shown in the appended examples. Incubation can e.g. be carried out in high-throughput assays which are capable of screening up to several thousand compounds in parallel. High-throughput assays generally may be performed in wells of microtiter plates, wherein each plate may contain 96, 384 or 1536 wells. Handling of the plates, including incubation at temperatures other than ambient temperature, and bringing into contact of test compounds with the assay mixture is preferably affected by one or more computer-controlled robotic systems including pipetting devices. In case large libraries of test compounds are to be screened and/or screening is to be effected within short time, mixtures of, for example about or exactly 10, 20, 30, 40, 50 or 100 test compounds may be added to each well. In case a well exhibits biological activity, said mixture of test compounds may be de-convoluted to identify the one or more test compounds in said mixture giving rise to said activity.

Subsequently, the cells may be fixed by methods well established in the art, using e.g. fixatives commonly used in histo-chemical or fluorescence studies such as for example paraformaldehyde, such as 3.7% PFA.

Then, in step (b), the cells are stained with a suitable detectable marker probe or a detectable marker probe is introduced into the cell(s) in order to enhance the visibility of cellular and/or bacterial features. Detectable marker probes for staining or which can be introduced into the cell(s) are not particularly limited. They include, without being limiting, markers selected from the group consisting of fluorescent compounds, bioluminescent compounds, chemiluminescent compounds, radioisotopes, markers which can be detected by colorimetric methods, metal chelates and enzymes or compounds, such as proteins, labelled therewith. In a preferred embodiment of the method of the invention, the detectable marker probe introduced into the cell(s) is a compound labelled with a fluorescent dye. A fluorescent dye in accordance with the present invention includes compounds which emit light of a particular wavelength when exposed to electromagnetic radiation of a shorter wavelength. Non-limiting examples of fluorescent dyes are fluorescein, naphtofluorescein, the various known versions of GFPs, Rhodamin, Texas Red, Pacific Blue, Oregon Green, Alexa Fluor Dyes, Cy3 or Cy5. In another preferred embodiment of the method of the invention, the cells are stained with a fluorescent dye. For example, cells may be stained with 4',6-diamidino-2-phenylindole (DAPI), a fluorescent stain that binds strongly to A-T rich regions in DNA, for staining the nucleus and Cell Mask Blue (Invitrogen), which labels the cytoplasm of the cell.

Means and methods for introducing a detectable marker probe into the cell are well known in the art. For example, a fluorescently labelled probe may be introduced into the cell via a transport protein, such as e.g. transferrin (Tfn), as described e.g. in Example 4 below. Alternatively, antibodies for a specific target within the cell can be introduced, wherein said antibodies are detectably labelled, for example with a fluorescent dye. Also envisaged herein is the use of bi-specific antibodies, wherein one antibody specificity is directed to a cellular compartment/target while the second antibody specificity is directed to a detectable protein or dye. Alternatively, a detectable marker probe may be introduced into the cell in form of an expressible nucleic acid molecule using any of the methods known in the art for introducing nucleic acid molecules into cells. Such methods include for example calcium phosphate-DNA coprecipitation, DEAE-dextran-mediated transfection, polybrene-mediated transfection, electroporation, microinjection, liposome fusion, lipofection, protoplast fusion, retroviral infection, and biolistics. Preferably, said nucleic acid molecule(s) encoding the detectable marker probe is contained in a vector expressible in the cell. Suitable expression vectors are well known in the art.

Next, in step (c), a multi-parametric phenotypic profile of host cell parameters and bacterial parameters is obtained. "Multi-parametric phenotypic profiles" are well known in the art and have been described in e.g. Collinet et al, Nature 2010. Such profiles reflect the phenotype of e.g. a cell or population of cells based on a number of parameters, such as the parameters detailed herein below.

This step includes acquiring images of the cells, for example using the automated spinning disk confocal Opera (Perkin Elmer) and analysing said images. Preferably, image analysis is carried out automatically.

Finally, in step (d), the multi-parametric phenotypic profile is clustered by using a new density-estimate-based clustering algorithm. As high-content screening datasets have a complex distribution of multi-dimensional phenotypes, new clustering and hit-selection methods are required. Using a new density-based method for robust clustering of multi-dimensional phenotypes is employed herein.

The density-based clustering algorithm of the present invention is similar to mean-shift, wherein the move along the steepest gradient over a continuous density estimate function is replaced by a discrete gradient ascent over a sequence of Gabriel graph neighbouring data points (Gabriel, K. R.; Sokal, R. R., A new statistical approach to geographic variation analysis, Systematic Zoology (1969) v.18(3), pp.259-270). Such a design provides two main advantages. First, peculiar dependency of number of clusters as function of clustering free parameter (kernel bandwidth) which provides intrinsic criteria for free parameter selection and, second, it only needs to compute a density estimate at each data point and does not depend on the smoothness of density estimate function between data points, which decreases over-fragmentation in multi-dimensional space.

The clustering algorithm of the present invention begins in step (i) with computing a set of multi-parametric phenotype profiles into nodes of Gabriel graph where edges connect neighbouring nodes. Subsequently spurious as well as 'long-range' edges are filtered out from the graph in step (ii). Then the density estimate for each node is computed in step (iii) using a kernel function that is chosen according to the selected distance measure (as detailed below). Then the modified Gabriel graph is converted in step (iv) into weighted directed acyclic graph (WDAG) by directing the edges between neighbour nodes such that they point towards the node with higher density estimate. In the ambiguous case of equal density estimate of two neighbour nodes, one node is randomly chosen and a small arbitrary value (0.0000001) is added to its density estimate. Edge weights are set proportional to the distance between neighbouring nodes. The sinks of the graph (nodes with no out-coming edges) are marked as modes (local maxima) of density in step (v). Those data points become cluster centres, and the remaining data points are assigned in step (vi) to the cluster centres based on the shortest weighted path in the directed neighbourhood graph.

Kernel density estimate of the multivariate probability density function $\hat{f}(x)$ of a random variable x from an independent and identically-distributed sample $x_1, x_2, ., xn$ is given by

$$\hat{f}(x) = \sum_{i=0}^{n} K(D(x, x_i), h),$$

where $K$ is a kernel function that depends on the distance D between two points and bandwith $h$. Kernel function can be any function that fits the following criteria:

1) $K$ is a function of distance $D(x, x_i)$ and is defined over the whole argument space;
2) $K$ is monotonously non-increasing;
3) $K$ has unity integral on the argument space.

The simplest case is the so-called flat kernel, where $K$ equals constant when $D(x, x_i) < h$ and 0 otherwise. In this case, kernel density estimate simply is equal to the number of data points that fall into the hyper-sphere of radius h around the point x, normalized by the volume of this sphere. The bandwidth parameter h determines the degree of density smoothing. In each particular case, the choice of kernel function is primarily guided by the measure of distance between data points. In the case of Euclidian distance, a commonly chosen kernel is a multivariate Gaussian function in which the covariance matrix parameterizes the bandwidth.

In case of directional data, meaning that the similarity between two feature vectors is given by cosine of angle (uncentered Pearson correlation), von Mises-Fisher probability function may be used as a kernel

$$p_{vMF}(\vec{x} \mid \vec{\mu}, k) = \frac{k^{d/2-1}}{(2\pi)^{d/2} I_{d/2-1}(k)} e^{k \cdot \cos\theta}, \text{ where } \cos\theta = \frac{\vec{x} \cdot \vec{\mu}}{\|x\| \cdot \|\mu\|}$$

Von Mises-Fisher probability distribution is an analogue of normal distribution for directional data and defines the probability of a random vector $\bar{x}$ that deviates from the central vector $\bar{\mu}$ by cos $\theta$. The distribution has one concentration parameter $K$ (kappa) that is analogous to inverse of variance in Gaussian. Hence $K$ defines the concentration (inverse bandwidth) of von Mises-Fisher kernel. The normalization constant is dependent on modified Bessel function of the first kind $I$ of the order $\frac{d}{2} - 1$, where d is the number of dimensions.

In order to define data point neighbourhood in a nonparametric and locally adaptive way Gabriel graph is defined such that two points are connected by edges if and only if centre of the edge is closer to these points than to any other point in the set. According to the invention the Gabriel neighbours $x_i$ and $x_j$, that connected by edge which central point distance to the third point of set ($x_K$) is smaller than $\frac{1}{2}(1+\alpha) \cdot D(x_i, x_j)$ are filtered out as a spurious edges. The parameter $\alpha$ was set to 0.05 for all clustering shown in the appended examples.

In some cases the density modes of two clusters could occur almost on the edge of clusters and in multidimensional space they may happen to be Gabriel neighbours of each other through 'long-range' connections. In this case the present algorithm cannot separate them. To rescue that situation, for every data point the set of its Gabriel neighbours were additionally filtered by taking intersection with sets of its nearest neighbours of an equal size. This procedure removes 'long-range' links that spans across empty regions of the feature space while keeping most of the links within the clusters.

Further details of the algorithm in accordance with the present invention are provided in Example 8 below.

In accordance with this method of the invention, (an) alteration(s) in bacterial parameters and a lack or substantial lack of alteration(s) of host cell parameters in the presence of the test compound indicate that the compound is suitable as a lead compound for the treatment of tuberculosis. Alterations can for example be observed by comparing the host cell and bacterial parameters in the presence of the test compound with the corresponding host cell and bacterial parameters in the absence of the test compound.

The key feature of this intracellular mycobacterial survival assay is the multiplicity and reliability of parameters capturing various features of bacteria and host at high resolution. Interestingly, most compounds in the selected hit cluster (M5) were not directly detrimental to mycobacteria. Although it cannot be ruled out completely that bacteria may be killed directly within infected cells, the inventors hypothesize - without wishing to be bound by theory - that the compounds act via activating the host's own defence mechanisms for the following reasons. First, the M5 cluster showed very little overlap with the compounds which act directly on bacteria. Second, the three selected compounds do not kill mycobacteria outside the host cell under the different conditions tested. Third, they show similar effects on uninfected cells. Fourth, the effect of Nortriptyline and PE can be reverted by inhibiting a host cellular process (autophagy). Fifth, Nortriptyline showed strong phenotypic correlation with a structural analogue, Amitriptyline, in mycobacteria as well as in the endocytosis and autophagy screens (Figure 9F-I). Sixth, Nortriptyline and PE enhance the acidity of lysosomes. This correlates with delivery of mycobacteria to lysosomes and their survival. Additionally, standard anti-mycobacterial drugs showed weaker phenotypes compared to the selected compounds. Most likely, this is because the assay as exemplified herein measures GFP fluorescence from mycobacteria that decays at different rates, depending on whether the host degradation machinery can be accelerated or not. In support of this, Rifampicin did not show enhanced lysosomal acidity. Thus, this screening platform has a bias for compounds that boost host defence mechanisms. Importantly, these data argue that such compounds are likely to be missed in a screen for anti-mycobacterial compounds outside the context of infected cells.

In a preferred embodiment of the method of the invention, the cells are infected with mycobacteria that express a fluorescent protein marker. Preferably, the fluorescent protein marker is green fluorescent protein (GFP).

As is shown in the appended examples, using mycobacteria that express green fluorescent protein (GFP) render it easier to analyse and image bacterial parameters inside the cell.

In a more preferred embodiment of the method of the invention, the bacterial parameters are selected from parameters related to the number of bacteria, parameters related to signal intensity of the bacteria and/or parameters related to the shape of the bacteria.

Parameters related to the number of bacteria include, without being limiting:

(i) number of bacteria within the infected cell (mean, median and/or mode),
(ii) total number of bacteria per image,
(iii) number of bacteria per cell (mean, median and/or mode),
(iv) total number of bacteria in infected cells and
(v) number of infected cells.

The term "number of bacteria within the infected cell", as used herein, refers to the total number of bacteria that are present within one infected cell, and averaged over all the infected cells for a given condition.

As used herein, the terms "mean, median and/or mode" refer to standard measures of central tendencies of a dataset, i.e. "average" values that are well known in the art. "Mean" refers to the arithmetic mean of a dataset, "median" refers to the value that divides the dataset into upper and lower halves, while "mode" is the most frequently occurring value in the dataset. In case of normal distribution of a parameter, the mean, median and mode coincide. However, this is not always the case. Parameter distributions are often skewed and have heavy tails. By using a combination of mean, median and mode, it is possible to summarize the main features of the distribution in a few values without making any assumption about distribution *per* se. For example, mode of the distribution provides information about the parameter value of the most abundant objects while mean and median reflect on different features of distribution. Accordingly, including all of mean, median and mode, rather than only mean, provides a better descriptive power for the entire distribution of the parameter. This allows describing more aspects, such as the possible presence of a second population of infected cells, or asymmetry in the distribution, long tails, etc.

The term "total number of bacteria per image", as used herein, is an absolute value that reflects the number of all bacteria identified in the image that is analysed, irrespective of the amount of host cells present. Only intracellular bacteria, i.e. bacteria found inside of host cells, are taken into consideration.

The term "number of bacteria per cell", as used herein, refers to the number of all bacteria in relation to the total number of host cells (infected and uninfected) present. Thus, whereas the measure "(i) number of bacteria within the infected cell" is an averaged measure of the number of bacteria in a single infected cells, the measure "(iii) number of bacteria per cell" is a measure of the overall number of bacteria in relation to the overall number of all host cells present, both infected and uninfected.

As used herein, the term "total number of bacteria in infected cells" refers to an absolute value representing the number of all bacteria found in infected cells, i.e. without correlating said number to the number of host cells present (as done in (iii)) or the number of host cells infected (as done in (i)).

The term "number of infected cells" refers to an absolute value representing the number of all infected cells within the image taken. Where several images are taken and analysed, the data obtained are combined.

Parameters related to signal intensity of the bacteria include, without being limiting:

(i) integral vesicle intensity of bacteria within infected cell,
(ii) mean intensity (e.g. of the GFP signal in case of bacteria expressing GFP) (non-weighted median, weighted (intensity) and/or weighted (integral intensity)) and
(iii) mean integral intensity (e.g. of the GFP signal in case of bacteria expressing GFP) (non-weighted median, weighted (intensity) and/or weighted (integral intensity)).

Intensity-related parameters compute the average as well as integral intensity of bacteria within infected cells. Integral intensity is defined as the integral (the sum pixel by pixel) of the intensity calculated on the fitted object, such as for example an individual bacterium. The term "integral vesicle intensity of bacteria within infected cell", as used herein, refers to the integral intensity of bacteria within all infected cells, i.e. it is the sum of all individual integral intensities. The term "mean intensity", as used herein, refers to the average intensity within each identified object (i.e., bacteria) while the term "mean integral intensity" refers to the mean value of integral (i.e sum) intensity within the same identified object (i.e individual bacteria).

The integral vesicle intensity of bacteria within infected cell is the total (i.e., sum) of all bacterial integral intensities, normalized by the area covered by cells.

The terms "weighted (intensity)" or "weighted (integral intensity)" refer to the fact that the respective parameter has been corrected for the factor recited in brackets, i.e. the term "mean integral intensity (weighted (integral intensity))" refers to the parameter "mean integral intensity", wherein the measured mean integral intensity has been adjusted by the integral intensity . These adjustments are well known in the art and can be carried out by the skilled person without further ado. For example, if a

certain parameter p is measured for two distinct bacteria, two measures ($p_1$ and $p_2$) are obtained. Two additional parameters are also measured for the same objects, the weighing parameters $i_1$ and $i_2$. In that case, the simple mean of the parameter p would be $p_{avg} = (p_1 + p_2) / 2$, whereas the weighted mean would be $p_{avg\_w} = (p_1 * i_1 + p_2 * i_2) / (i_1 + i_2)$.

Weighting can reveal important biological features: measuring the mean parameter value gives information about whole population behaviour, including both bright (presumably intact) and dim (presumably partially degraded) bacteria at the same time; parameter weighted by intensity give information mostly about brighter (and presumably) bacteria. This approach extracts more comprehensive description of the phenotype from the same biological assay.

Parameters related to the shape of the bacteria include, without being limiting, the mean area, either non-weighted median, or weighted (intensity) and/or weighted (integral intensity).

The term "mean area", as used herein with regard to the shape of bacteria refers to the area in square micrometres enclosed by the contour of the segmented object.

In another preferred embodiment of the method of the invention, the host cell parameters are selected from parameters related to the morphology of the cell and/or parameters indicative of toxicity.

Parameters related to the morphology of the host cell include, without being limiting:

(i) area,
(ii) compactness,
(iii) dispersion,
(iv) eccentricity,
(v) solidity, and
(vi) extent.

The term "area", in this regard, relates to the average area of the host cells in square micrometers.

As used herein, the term "compactness" relates to a measure of region non-smoothness, defined as the ration between the actual area and the area a circle with the same perimeter would have. Compactness is measured on the contour of the segmented host cell.

The term "segmented", as used herein, is a standard term used in the art. It refers to an image divided on segments, which correspond either to objects of interest or to the background.

The term "dispersion", as used herein, relates to a measure of region irregularity, defined as the ratio of the major chord length (maximum circle enclosing the region) to the area and is measured on the contour of the segmented host cell.

The term "eccentricity", as used herein, is a measure of the elongation of the region, defined as:

$$e = \sqrt{1 - \left(\frac{b}{a}\right)^2}$$

where a is the semi-major axis and b the semi-minor axis of the best fitting ellipse. The axes can be directly calculated through the eigenvector decomposition of the region contour. The corresponding eigenvalues are equal to the length of the ellipse axes. The terms eigenvalues and eigenvectors are well known to the skilled person and are standard mathematical terms introduced in the early 19th century. Both eigenvalues and eigenvectors are widely used in linear algebra and geometry, and also find application in image analysis.

As used herein, the term "solidity" is a measure of segmented region tightness, defined as the ratio between the region area and the area of the convex hull of the same region. Solidity is measured on the contour of the segmented region which corresponds to the host cell..

The term "extent", as used herein, is a measure of region extension defined as the ratio between the area of the minimal rectangle (bounding box) that encloses the region and the actual region area. Extent is measured on the contour of the segmented host cells.

Parameters indicative of toxicity include, without being limiting:

(i) nucleus size (median, median (weighted) and/or mean),
(ii) number of nuclei, and
(iii) number of cells.

"Nucleus size", as used herein, relates to the average nuclei size in the image. Measurement of the size of nuclei is indicative of toxicity, since nuclear condensation is an initial signature of apoptotic cell death and a general sign of loss of cellular fitness (Galluzzi et al., 2009).

As used herein, "number of nuclei" relates to the number of nuclei per image.

"Number of cells", as used herein, relates to the number of cells per image. As all other parameters, this value is then averaged across all the images of the same experimental condition.

These host cell parameters capture the dynamics of alterations in the host cell as a consequence of perturbation.

Additionally, quality control and toxicity parameters are included. For example, "nuclei contrast" is used as a quality control parameter, as it estimates the correctness of focal position in the given image by measuring the difference in contrast between nuclear signal DAPI and nearby cytoplasmic pixels.

A comprehensive description of several of these parameters is provided in chapter 7 of the book "Feature Extraction & Image Processing for Computer Vision", Mark Nixon and Alberto S Aguado, ISBN: 978-0-12-396549-3.

Preferably, at least six bacterial parameters and at least one host cell parameter are analysed. The term "at least", as used herein, refers to the specifically recited amount or number but also to more than the specifically recited amount or number. For example, the term "at least six bacterial parameters" encompasses also at least 7, at least 8, at least 9, at least 10, at least 11, at least 12, at least 13, at least 14 parameters, such as at least 15, at least 16, at least 17, at least 18 parameters as well as all 19 bacterial parameters.

In a particularly preferred embodiment of the method of the invention, the bacterial parameters comprise at least

(i) number of bacteria per cell (mean);
(ii) total number of bacteria (per image);
(iii) integral vesicle intensity of bacteria within infected cell;
(iv) mean intensity of bacteria (non-weighted median);
(v) mean area of bacteria (non-weighted median); and
(vi) number of infected cells;

and the host cell parameters comprise at least

(a) number of cells.

Even more preferably, all 19 bacterial parameters and all 11 host cell parameters recited herein above (and shown in Table 1) are analysed.

As is shown in the appended examples, measuring different parameters gives a wealth of information related to bacteria as well as host cells in a high content screen, thereby describing the multiple facets of the biology of bacterial infection. Although one could consider only the integral intensity of bacteria within infected cell as a hallmark of infection, this would only be a very crude estimate of the intracellular bacteria status. It cannot distinguish between the possibilities where the bacteria are unchanged by perturbations from situations where the integral intensity of bacteria within the infected cell is unchanged, but the marker (GFP in the present examples) is distributed in many more, smaller objects. Hence, by considering several parameters, such as integral intensity of bacteria within infected cell, mean area, mean intensity and number of bacteria, or preferably the entire ensemble of bacterial parameters, one can better interpret the phenotype induced by perturbation. Moreover, since it is relevant to observe the effect on mycobacteria infection in the cellular context, it is also very important to monitor the status of the cell. In summary, using this approach, the multiple facets of phenotype space that are induced by the application of a test compound can be captured and compounds suitable for the intracellular targeting of bacterial infections can be identified.

In a preferred embodiment, the method further comprises optimizing the pharmacological properties of the test compound identified as lead compound, thereby enabling the development of further modified and potentially improved compounds.

The identified lead compounds may for example be optimized by modifying the compound to achieve: i) modified spectrum of activity, organ specificity, and/or ii) improved potency, and/or iii) decreased toxicity (improved therapeutic index), and/or iv) decreased side effects, and/or v) modified onset of therapeutic action, duration of effect, and/or vi) modified pharmacokinetic parameters (resorption, distribution, metabolism and excretion), and/or vii) modified physico-chemical parameters (solubility, hygroscopicity, colour, taste, odour, stability, state), and/or viii) improved general specificity, organ/tissue specificity, and/or ix) optimised application form and route by (a) esterification of carboxyl groups, or (b) esterification of hydroxyl groups with carboxylic acids, or (c) esterification of hydroxyl groups to, e.g. phosphates, pyrophosphates or sulfates or hemi-succinates, or (d) formation of pharmaceutically acceptable salts, or (e) formation of pharmaceutically acceptable complexes, or (f) synthesis of pharmacologically active polymers, or (g) introduction of hydrophilic moieties, or (h) introduction/exchange of substituents on aromates or side chains, change of substituent pattern, or (i) modification by introduction of isosteric or bioisosteric moieties, or (j) synthesis of homologous compounds, or (k) introduction of branched side chains, or (l) conversion of alkyl substituents to cyclic analogues, or (m) derivatisation of hydroxyl groups to ketales, acetales, or (n) N-acetylation to amides, phenylcarbamates, or (o) synthesis of Mannich bases, imines, or (p) transformation of ketones or aldehydes to Schiff's bases, oximes, acetales, ketales, enolesters, oxazolidines, thiazolidines or combinations thereof.

The various steps recited above are generally known in the art. They include or rely on quantitative structure-action relationship (QSAR) analyses (Kubinyi, "Hausch-Analysis and Related Approaches", VCH Verlag, Weinheim, 1992), combinatorial biochemistry, classical chemistry and others (see, for example, Holzgrabe and Bechtold, Deutsche Apotheker Zeitung 140(8), 813-823, 2000).

The present invention further relates to a method of identifying the cellular pathway targeted by a test compound, the method comprising

(a) treating a cell with the test compound and obtaining a multi-parametric phenotypic profile of the cell after said treatment;
(b) providing a phenotypic profile obtained for the same cell type by introducing directed genetic perturbations to target genes of said cell;
(c) comparing the multi-parametric phenotypic profile obtained in (a) with the phenotypic profile provided in (b) and identifying the genes whose perturbation leads to an identical or substantially identical phenotypic profile as the test compound; and
(d) identifying the cellular pathways involving the genes identified in (c).

In accordance with this method of the invention, the cellular pathway that is targeted by a test compound is identified. Accordingly, it is investigated which intracellular pathway(s) is/are affected in the presence of the test compound. The test compound may be any test compound, such as the test compounds defined herein above. Moreover, the test compound may be a compound identified by a method of the present invention of identifying a lead compound for the treatment of tuberculosis.

In a first step of this method of the invention, a cell is treated with the test compound and a multi-parametric phenotypic profile of the cell after said treatment is obtained. Typically, the cell is simply contacted with the compound, which then either exerts its effects via binding and/or activation of surface molecules, such as e.g. receptors, or is internalised into the cell, for example by active transport through the cell membrane or endocytosis. However, it will be appreciated that the compound may also be introduced directly into the cell, such as e.g. by micro-injection.

The cell, in accordance with this method, may be any cell typically employed in research, such as e.g. prokaryotic or eukaryotic cells, including the cells defined herein above with regard to the method of obtaining a multi-parametric phenotypic profile of a cell infected with mycobacteria. Additionally, suitable prokaryotic cells comprise e.g. bacteria of the species *Escherichia, Bacillus, Streptomyces* and *Salmonella typhimurium and Mycobacteria sp.* Suitable eukaryotic cells are e.g. fungal cells, inter alia, yeasts such as *Saccharomyces cerevisiae* or *Pichia pastoris* or insect cells such as Drosophila S2 and Spodoptera Sf9 cells and plant cells as well as mammalian cells. Appropriate culture media and conditions for the above-described cells are known in the art. Mammalian cells that could be used include, human HeLa, HEK293, H9 and Jurkat cells, mouse NIH3T3 and C127 cells, Cos 1, Cos 7 and CV1, quail QC1-3 cells, mouse L cells and Chinese hamster ovary (CHO) cells. Also within the scope of the present invention are primary cells or primary cell lines. Primary cells are cells which are directly obtained from an organism. Suitable primary cells are, for example, human primary monocytes, human primary monocyte derived macrophages, mouse bone marrow macrophages, mouse peritoneal macrophages, mouse embryonic fibroblasts (MEF), mouse primary hepatocytes, cardiomyocytes and neuronal cells as well as mouse muscle stem cells (satellite cells) and stable, immortalized cell lines derived thereof. Preferably, the cells are human HeLa cells.

The multi-parametric phenotypic profile may be a profile of the entire cell or of specific cellular compartments or pathways. Multi-parametric phenotypic profiles and methods of obtaining same are well known in the art, as described herein above.

One exemplary method of obtaining a multi-parametric phenotypic profile of a cell is described in detail herein above for the phenotypic profile of a cell infected with mycobacteria. Accordingly, all of the definitions and preferred embodiments recited herein above with regard to said method of obtaining a multi-parametric phenotypic profile of a cell apply *mutatis mutandis* also to this method of the invention. An alternative method of obtaining a multi-parametric phenotypic profile of a cell is shown in Example 4 below, where the effect of the tested compounds on endocytosis was analysed. In short, the cells of interest, such as e.g. HeLa cells, are pre-treated with the test compounds prior to the internalization of fluorescently-labelled marker suitable to show the effect of the test compound on the cellular function to be analysed. In this example, the effect of the test compounds on endocytosis was analysed and, thus, the fluorescently-labelled markers were epidermal growth factor (EGF) and Transferrin (Tfn), which are known to be internalised in these cells by endocytosis.

The multi-parametric profile may encompass any cellular parameters of interest, such as e.g. the host cellular parameters recited herein above. Moreover, pathway-specific parameters can be analysed, such as e.g. the parameters shown in Table 3 below for endocytosis and autophagy pathways.

The multi-parametric phenotypic profile of the cell after treatment with a test compound is then compared to a phenotypic profile obtained for the same cell type by introducing directed genetic perturbations to target genes of said cell.

Means and methods of introducing genetic perturbations to target genes of cells are well known in the art and include, without being limiting, the use of interfering nucleic acid molecule (RNAi) such as e.g. siRNA or miRNA as well as the targeted inactivation by methods such as homologous recombination, gene trapping or transposon-mediated mutagenesis. Such a phenotypic profile obtained by genetic perturbations of target genes can be established by the skilled person without further ado, for example as described in Collinet *et al.* 2010. Moreover, previously established data or published results of such phenotypic profiles obtained by genetic perturbations of target genes can be relied on.

The term "same cell type", as used herein, refers to a cell being of the same origin as the cell of step (a) and that is - prior to treatment with any compounds, i.e. under normal culture conditions - identical in its phenotypic characteristics to the cell of (a). Furthermore, this term also encompasses cell populations, such as for example homogenous cell populations consisting of cells having identical characteristics, and, thus, is not restricted to single cell analyses.

By comparison of the multi-parametric phenotypic profile obtained in the first step with the phenotypic profile obtained by genetic perturbations of target genes in the same cell type it is possible to identify those phenotypes that are identical or substantially identical in both data sets.

The term "substantially identical" relates to phenotypes that are not identical (i.e. do not correspond to each other to 100%) but do not differ significantly from each other. In order to determine whether two profiles are substantially identical, any known statistical approach may be employed. For example, the p-value of the null-hypothesis that the two profiles are the result of the same perturbation can be calculated. Means and methods for calculating the p-value are well known in the art. For example, if the number of parameters is higher than 10 and the measured correlation is below 0.9, then the chi-square approximation can be used for p-value calculation. In those cases where the number of parameters is less than 10 and/or for very high correlation (i.e. above 0.9), the calculation for the p-value can be done as for example described in the classical works of Fisher (see e.g. R. Fisher, "On the probable error of correlation coefficient deduced from a small sample set," Metron, v.1, pp.3-32 (1921) and (R. Fisher, "Frequency Distribution of the Values of the Correlation Coefficient in Samples from an Indefinitely Large Population," Biometrika, v.10(4), pp.507-521 (1915)).

Means and methods of comparing the phenotypic profiles in step (c) include, without being limiting, automated comparison e.g. using a computer program. For example, profiles can be treated as vectors in multi-dimensional space and measure of similarity could be a cosine of angle between them. This cosine is equivalent to non-centred correlation of profiles. Another possible measure of phenotype similarity could be as described in Mahalanobis Distance (Mahalanobis, P. C. "On the generalised distance in statistics". Proceedings of the National Institute of Sciences of India 2 (1): 49-55 (1936), http://en.wikipedia.org/wiki/Mahalanobis_distance). The procedures for such computation are included in MatLab, R, and other standard mathematical and statistical packages.

Based on the thus identified identical (or substantially identical) phenotypes, it can then be analysed which genetic perturbations led to the said phenotype. The gene products affected by these genetic perturbations can then be analysed for their involvement in cellular pathways, for example based on KEGG pathways and GO terms as described in Example 4 below. These pathways are well known in the art.

In accordance with the present invention, an integrated experimental and computational pipeline was developed to compare the multi-parametric profiles from the screen of the pharmacologically active compounds with the profiles from a previously established genome-wide siRNA screen for endocytosis (Collinet et al., 2010) to identify the cellular mechanisms underlying the anti-bacterial activities of the compounds identified herein. As is shown in the appended examples, the predictions derived from said comparison on the three selected compounds could be verified and it was established that two of them modulate autophagy by distinct mechanisms and the third one accelerates endocytic trafficking. These results demonstrate that trafficking pathways can be modulated pharmacologically to stimulate intracellular mycobacteria killing.

Whereas cell-based high-content screens have several advantages over traditional target-based high-throughput screens, understanding the mechanism of action of the identified compounds is a critical bottleneck (Swinney and Anthony, 2011). One possible approach is to identify genes that when silenced produce a phenotype similar to that of small inhibitory molecules (Eggert et al., 2004). However, there are significant caveats to this approach: For example, small molecules usually act rapidly whereas the RNAi effect is gradual and requires longer times, possibly allowing the system to adapt to a new steady-state. Moreover, the biological activity of small molecules may result from the modulation of multiple target molecules (Imming et al., 2006) rather than specific targets as in RNAi, although these may be contaminated by off-target effects. Although it was considered difficult to identify targets of small molecules by comparing the phenotypic profiles of chemicals and silenced genes, this approach nevertheless allowed the identification of the cellular pathways targeted by small molecules. Accordingly, as shown in the appended examples, phenotypes were captured through QMPIA and, by integrating chemical and functional genomics datasets (Collinet et al., 2010), hit compounds of the mycobacteria screen described above were differentiated into distinct groups on the basis of their phenotypes on endocytosis. Using this strategy, it was shown both computationally and experimentally that one of the groups is significantly enriched for modulators of autophagy, a well known anti-mycobacterial cell defence mechanism (Gutierrez et al., 2004; Kumar et al., 2010). Remarkably, autophagy phenotypes were thus identified using a general endocytosis assay.

Nortriptyline and PE influence autophagy differently. Whereas Nortriptyline induces the formation of autophagosomes, PE slows down autophagic flux. The basal rate of autophagy may be sufficient to accumulate autophagosomes in the presence of PE. These autophagosomes progressively acidify over time and become competent for Mycobacteria degradation (Ponpuak et al., 2010). PE may act at different stages, e.g. on the fusion with lysosomes.

The identification of Haloperidol as anti-mycobacterial compound provides proof of principle for the pharmacological modulation of endocytic trafficking as a means of boosting the host defence system to clear intracellular pathogens. Most importantly, these data imply that such an approach is feasible without inducing overt toxicity to cells. Haloperidol leads to faster degradation of cargo by accelerating transport from early to late endocytic compartments. Previous work has identified both general design principles and molecular details of the machinery underlying endosome biogenesis and progression that are relevant to the pharmacological properties of Haloperidol. First, transport of cargo along the degradative route (LDL) entails a conversion from Rab5- to Rab7-positive endosomes (Rink et al., 2005). Such conversion is based on a cut-out switch (Del Conte-Zerial et al., 2008) that requires the endosomal levels of Rab5 to pass a threshold value prior to triggering a feedback loop causing its removal and substitution with Rab7. Second, the inventors' recent systems survey on endocytosis has revealed that the centripetal movement of endosomes previously observed by quantitative live cell imaging (Rink et al., 2005) is a master parameter that regulates several other properties of the early endosome network, including cargo progression (Collinet et al., 2010). Mycobacterium interferes with such a switch, keeping the phagosome in an arrested state. Remarkably, Haloperidol exerts a positive influence on both centripetal movement of endosomes and membrane recruitment of the Rab5 machinery. By shifting the molecular composition of the mycobacterial phagosome, Haloperidol counteracts the fine balance between phagosome activity and maturation arrest, restoring its maturation. Thus, relatively subtle alterations in the general endocytic trafficking machinery are sufficient to cause dramatic effects on intracellular mycobacterial survival.

In a preferred embodiment of the method of the invention of identifying the cellular pathway targeted by a test compound, step (a) further comprises introducing into the cell a detectable marker probe. Detectable marker probes which can be used in the context of the invention are not particularly limited, and have been defined herein above. In a preferred embodiment of the method of the invention, the detectable marker probe is a compound labelled with a fluorescent dye, as defined herein above. For example, for the study of endocytosis as shown in the appended examples, the detectable marker probe may be selected from e.g. fluorescently labelled epidermal growth factor (EGF), transferrin or Low density lipoprotein (LDL).

Means and methods for introducing a detectable marker probe into the cell are well known in the art and have been described herein above.

In a preferred embodiment of the method of the invention of identifying the cellular pathway targeted by a test compound, the directed genetic perturbations to target genes of the cell are introduced by use of RNAi. Even more preferred is that the directed genetic perturbations to target genes of the cell are introduced by use of siRNA.

In a more preferred embodiment, the RNAi-mediated genetic perturbation is a genome-wide endocytosis RNAi screen. More preferably, the RNAi-mediated genetic perturbation is a genome-wide RNAi screen, wherein RNAi directed to each gene is introduced individually.

In a preferred embodiment of the methods of the present invention, the method is carried out as a high-content assay.

High-content assays, generally may be performed in wells of microtiter plates, wherein each plate may contain, for example 96, 384 or 1536 wells. Handling of the plates, including incubation at temperatures other than ambient temperature, and bringing into contact of test compounds with the assay mixture is preferably affected by one or more computer-controlled robotic systems including pipetting devices. Where large libraries of test compounds are to be screened and/or screening is to be effected within short time, mixtures of, for example about or exactly 10, 20, 30, 40, 50 or 100 test compounds may be added to each well. In case a well exhibits biological activity, said mixture of test compounds may be de-convoluted to identify the one or more test compounds in said mixture giving rise to the observed biological activity.

As regards the embodiments characterized in this specification, in particular in the claims, it is intended that each embodiment mentioned in a dependent claim is combined with each embodiment of each claim (independent or dependent) said dependent claim depends from. For example, in case of an independent claim 1 reciting 3 alternatives A, B and C, a dependent claim 2 reciting 3 alternatives D, E and F and a claim 3 depending from claims 1 and 2 and reciting 3 alternatives G, H and I, it is to be understood that the specification unambiguously discloses embodiments corresponding to combinations A, D, G; A, D, H; A, D, I; A, E, G; A, E, H; A, E, I; A, F, G; A, F, H; A, F, I; B, D, G; B, D, H; B, D, I; B, E, G; B, E, H; B, E, I; B, F, G; B, F, H; B, F, I; C, D, G; C, D, H; C, D, I; C, E, G; C, E, H; C, E, I; C, F, G; C, F, H; C, F, I, unless specifically mentioned otherwise.

Similarly, and also in those cases where independent and/or dependent claims do not recite alternatives, it is understood that if dependent claims refer back to a plurality of preceding claims, any combination of subject-matter covered thereby is considered to be explicitly disclosed. For example, in case of an independent claim 1, a dependent claim 2 referring back to claim 1, and a dependent claim 3 referring back to both claims 2 and 1, it follows that the combination of the subject-matter of claims 3 and 1 is clearly and unambiguously disclosed as is the combination of the subject-matter of claims 3, 2 and 1. In case a further dependent claim 4 is present which refers to any one of claims 1 to 3, it follows that the combination of the subject-matter of claims 4 and 1, of claims 4, 2 and 1, of claims 4, 3 and 1, as well as of claims 4, 3, 2 and 1 is clearly and unambiguously disclosed.

The figures show:

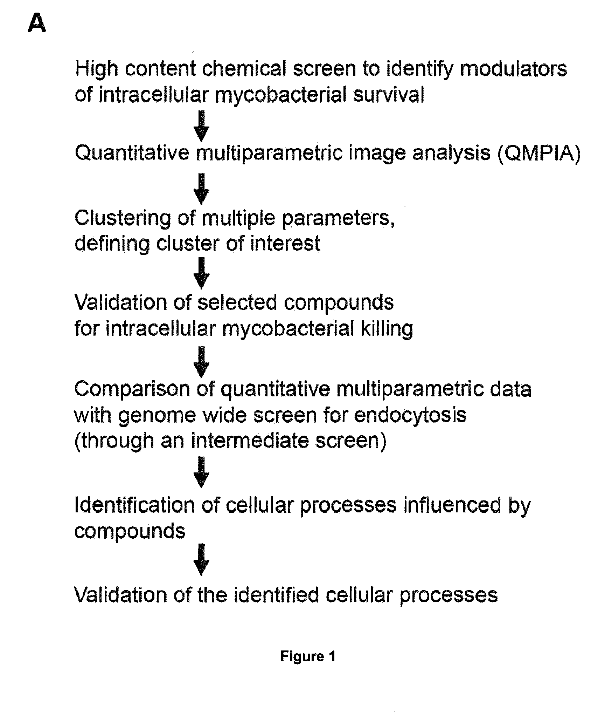

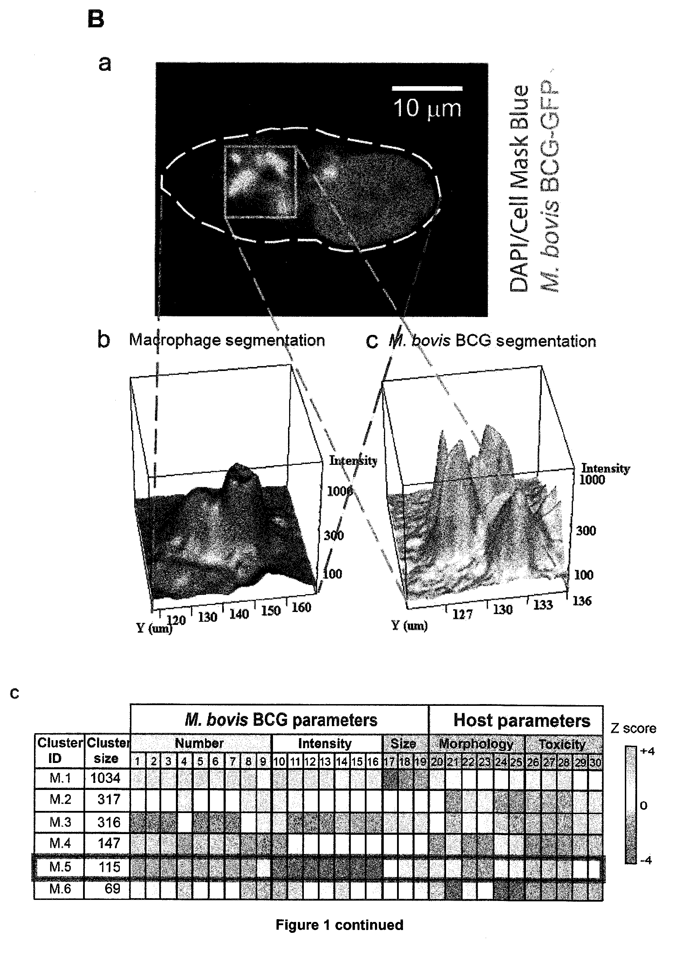

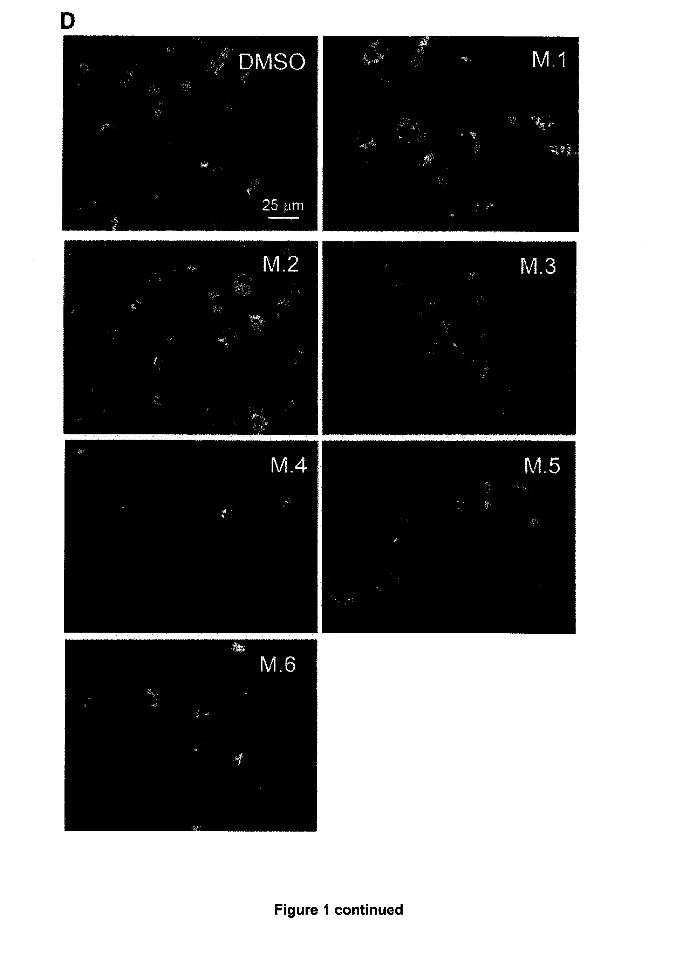

**Figure 1: High-content image-based screen for intracellular mycobacteria survival**

**A.** Flowchart of the experimental and computational pipeline used in this study.

**B.** a) Representative example of a human primary monocyte-derived macrophage infected with M. *bovis* BCG-GFP with cell contours (dashed line); b) Segmentation of macrophage: intensity distribution of nuclear and cytoplasmic regions used to draw the contours of the cell; c) Segmentation of bacteria: intensity distribution of bacteria in the region marked in the green square used for fitting the bacteria model;

**C.** Heat map representation of the mycobacteria high-content chemical screen after clustering. Rows denote cluster IDs and number of compounds. Columns are parameters of the high-content assay (see also Table 1, and experimental procedures). M5 is the cluster analyzed in this study;

**D.** Representative images from clusters M1-6.

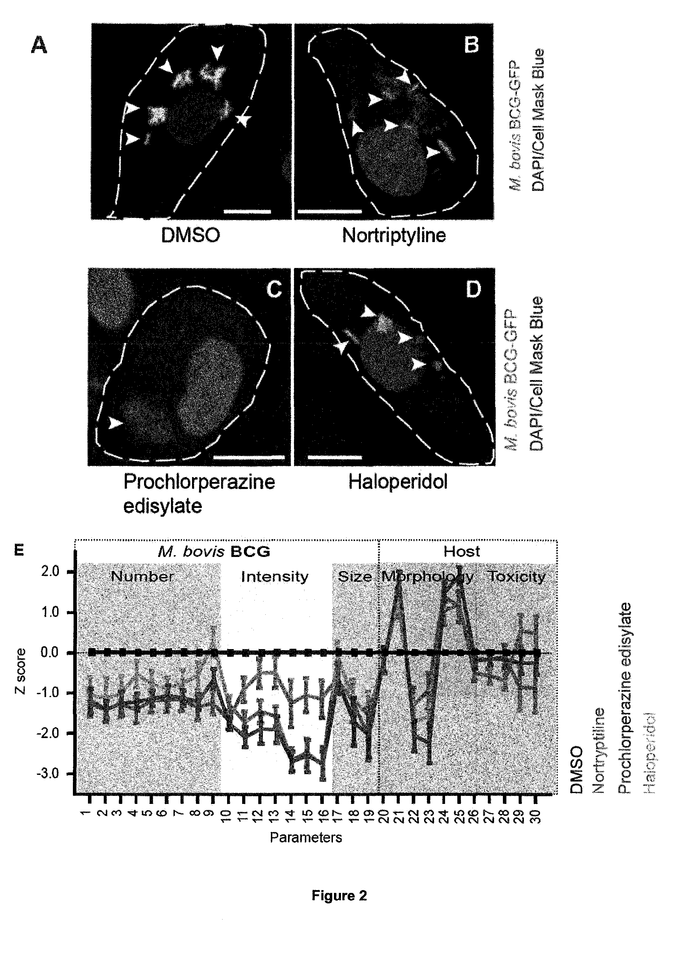

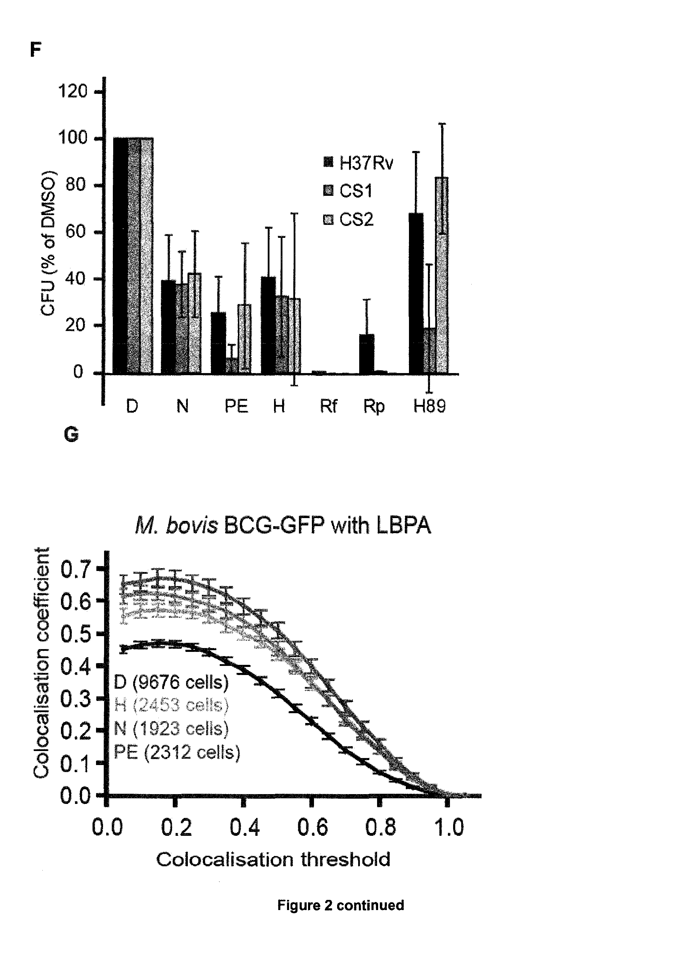

**Figure 2: Validation of three selected compounds for intracellular mycobacterial survival**

**A-D.** Representative images of infected cells treated with DMSO (A), Nortriptyline (B), Prochlorperazine edisylate (PE) (C) and Haloperidol (D) at a concentration of 10 $\mu$M for 48 hours after infection. Arrowheads indicate GFP-positive structures that are identified as 'bacteria' by the QMPIA. Scale bar=10 $\mu$m.

**E.** Quantitative multi-parametric profiles of the phenotypes in A-D with the Z score (y-axis) of the parameters (1-30, Table 1) showing e.g. decrease in number and intensity of intracellular mycobacteria. Error bars represent SEM.

**F.** CFU analysis of human primary monocyte-derived macrophages infected with virulent *M. tuberculosis* H37Rv and two different clinical isolates CS1 and CS2 and treated with the indicated compounds for 48 hrs (pooled data from two independent biological replicates with three technical replicates each). Error bars represent SD. D (DMSO 0.01%), Rf (Rifampin 2$\mu$M), Rp (Rapamycin, 5.5$\mu$M), PE (Prochlorperazine edisylate, 10$\mu$M), N (Nortriptyline, 10$\mu$M), H (Haloperidol, 10$\mu$M), H89 (H89, 10$\mu$M). p-values for all compounds tested (except H89) were < 0.0001 and < 0.01, for H37Rv and the two clinical strains, respectively.

**G.** Colocalization of *M. bovis* BCG-GFP and LBPA in cells treated with the indicated drugs. Number of cells analyzed per condition is indicated. Error bars are SEM. Data are representative of at least two independent experiments. Abbreviations and concentrations are as in F.

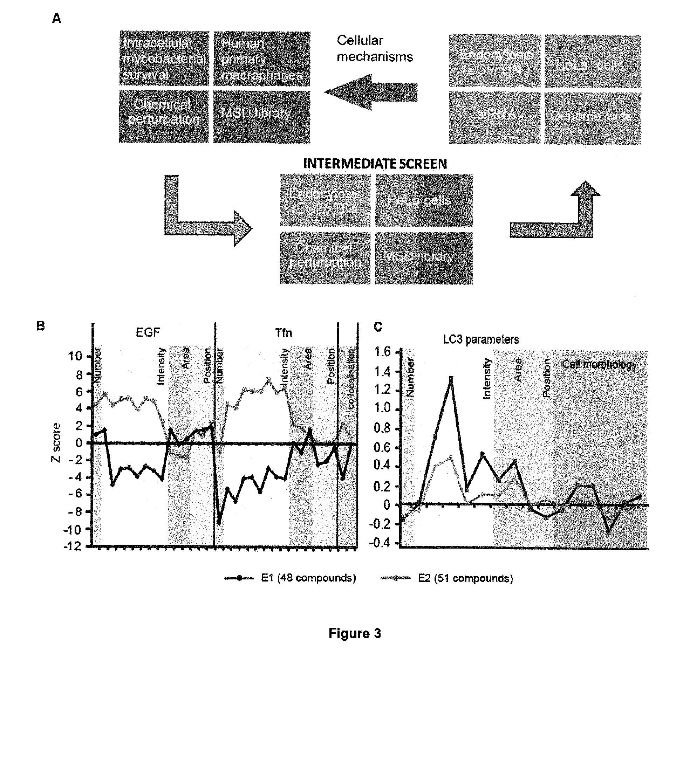

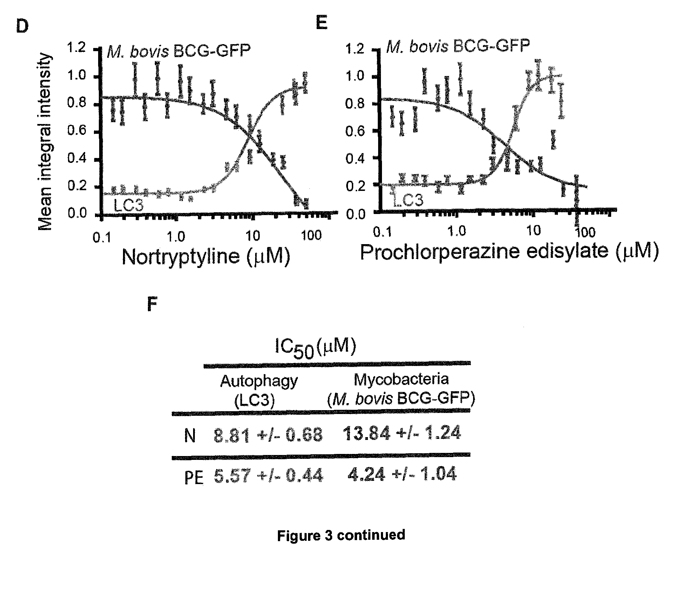

**Figure 3: Integration of chemical and RNAi screen data-sets.**

**A.** Scheme illustrating the integration of the primary chemical (left) and RNAi (right) screen data-sets via an intermediate screen on the chemicals from cluster M5.

**B.** and **C.** Endocytosis (B) and autophagy (C) profiles of sub-clusters E1 and E2 from cluster M5. The profiles are average representations of Z scores of the individual compounds within each sub-cluster. Parameters are grouped into different phenotype classes (Table 3).

**D.** to **E.** Dose response curves of mean integral intensities of *M. bovis* BCG-GFP (dark gray) and LC3 (light gray) for Nortryptyline (D) and Prochlorperazine edisylate (E). Error bars are SEM, number of images =25, data representative of at least two independent experiments.

**F.** Summary of IC$_{50}$ values calculated from curves D and E.

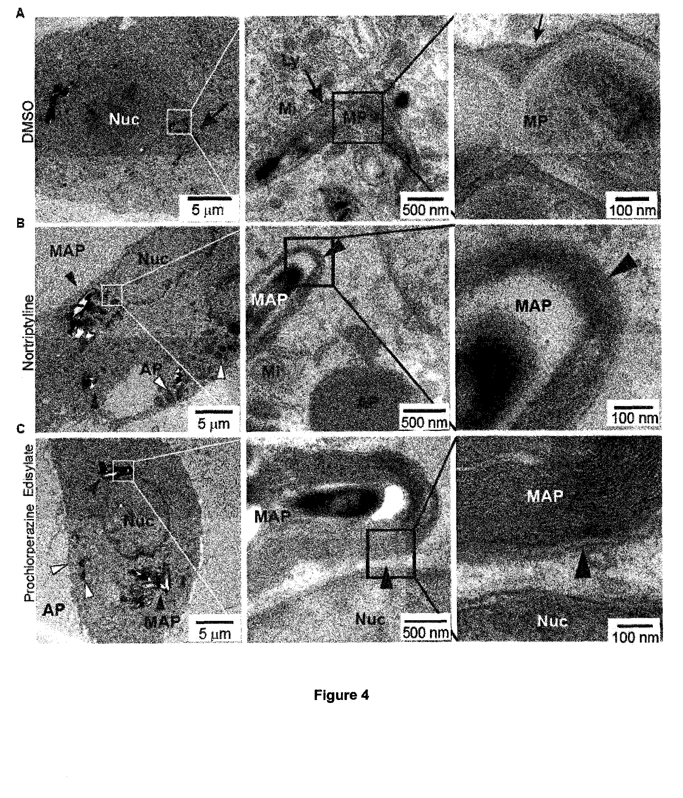

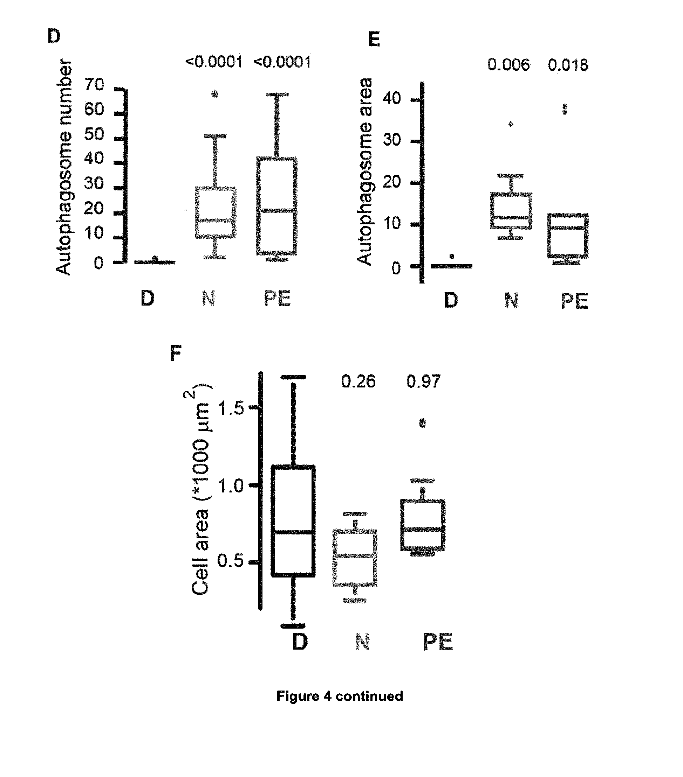

**Figure 4: Ultrastructural analysis of autophagy and intracellular mycobacterial survival.**

**A.** to **C.** Electron micrographs of *M. bovis* BCG-GFP infected human primary monocyte-derived macrophages treated with DMSO (A), 10 $\mu$M N (B) or PE (C) for four hr. Black arrows indicate mycobacterial phagosomes, black and white arrowheads indicate autophagosomes with and without mycobacteria, respectively. Mitochondria (Mi), nucleus (Nuc), mycobacterial phagosome (MP), autophagosomes (AP), mycobacteria containing autophagosome (MAP).

**D.** to **F.** Quantification of the number of autophagosomes (D) (n=30), autophagosome area as % of total cell area (E) and total cell area (F) (n=6) from the electron micrographs. D (DMSO, 0.01%), N (Nortriptyline, 10$\mu$M),

PE (Prochlorperazine edisylate, 10 $\mu$M). Numbers denote p values in comparison to DMSO.

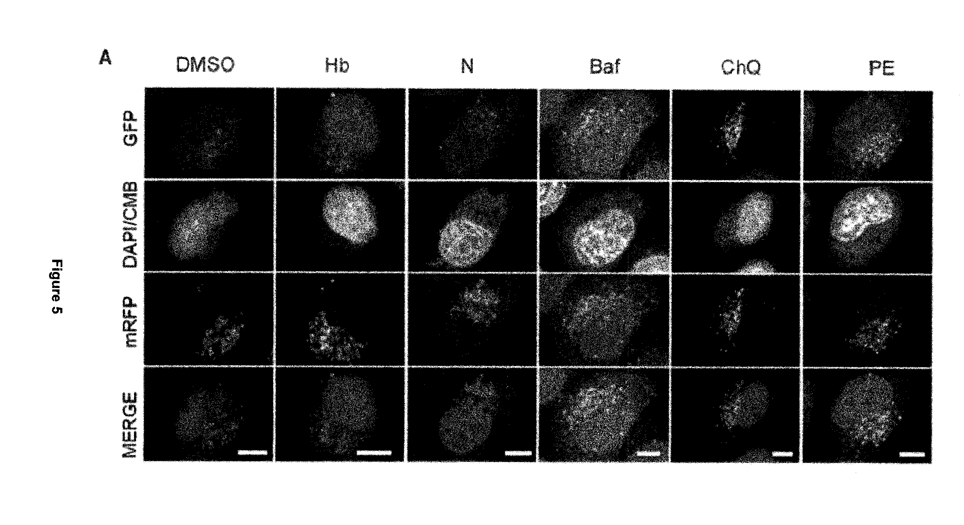

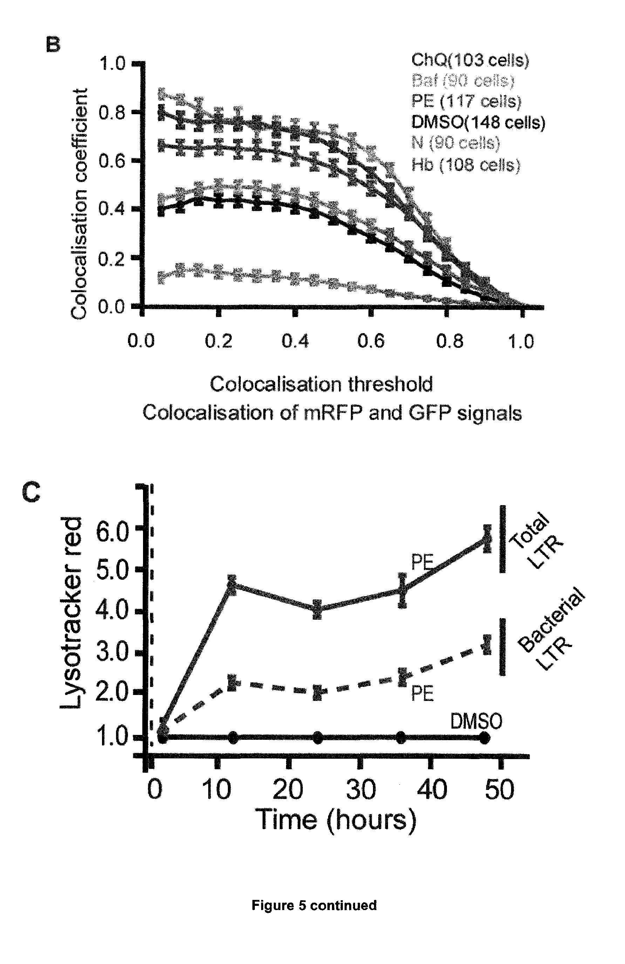

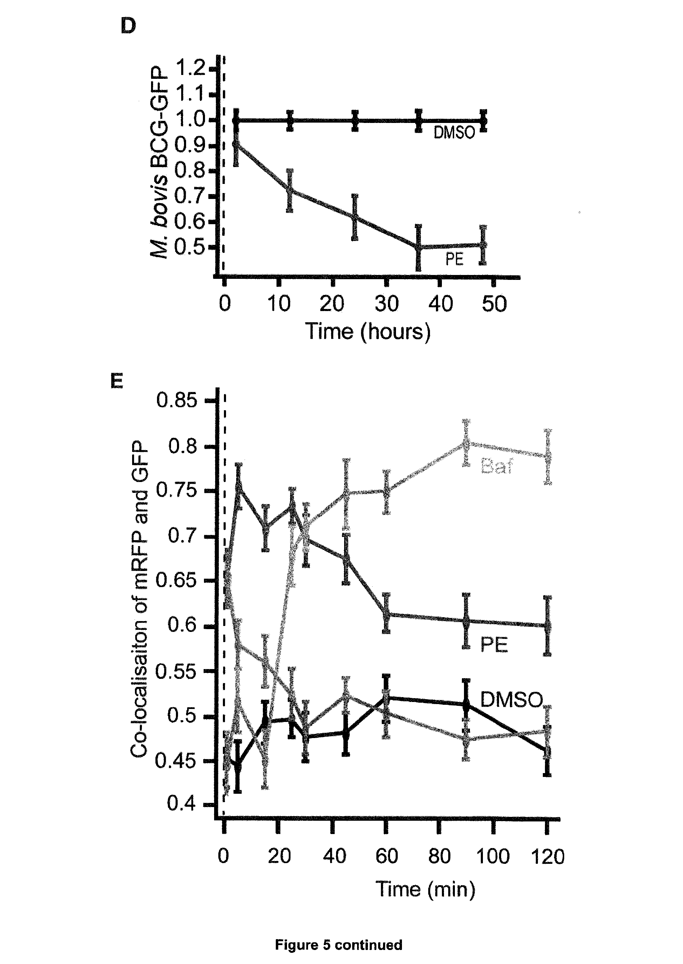

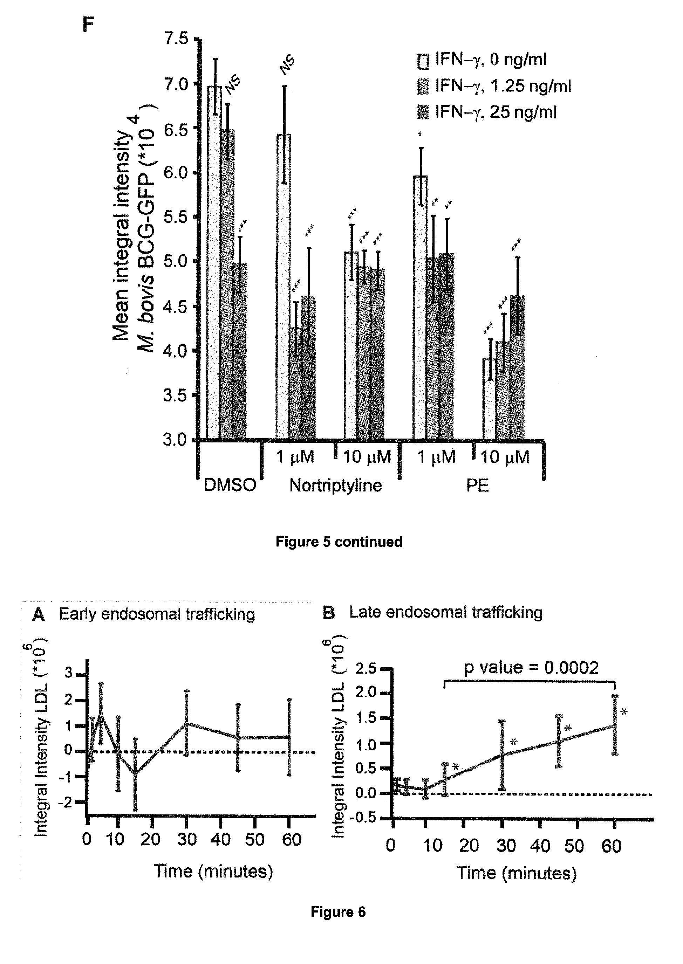

**Figure 5: Nortriptyline and PE modulate autophagy differently**

**A.** Representative images of HeLa cells stably expressing mRFP- GFP-LC3 treated with the indicated chemicals for 2 hr. DAPI/CMB denotes the cell nucleus and cytoplasm. Scale bar=10 $\mu$m.

**B.** Quantification of co-localization between the mRFP and GFP signals. The number of cells analyzed per condition is indicated. Results are representative of four experiments. Error bars are SEM. Abbreviations and concentrations as in A.

**C.** and **D.** Quantification of integral intensity of lysotracker red-labeled vesicles (LTR) (C), and *M. bovis* BCG-GFP (D) in infected human primary macrophages treated with either DMSO or PE (10$\mu$M) for the indicated periods of time. Total and bacterial LTR refer to integral intensity of all acidic compartments and those containing bacteria, respectively. Data are averaged from 45 images/condition/time point and normalized to DMSO to represent fold change (representative of two independent experiments).

**E.** Co-localisation between the mRFP and GFP signals in HeLa cells stably expressing mRFP-GFP-LC3 treated with the indicated drugs for different periods of time. Co-localization at a single threshold of 0.35 was used to generate the time response curves. Data are from 50 images per condition and aggregated from 4 experiments. For A, B and E, D (DMSO, 0.01 %); Hb (HBSS); N (Nortriptyline,10$\mu$M); ChQ (Chloroquine, 25 $\mu$M); Baf (BafilomycinA1,100nM); PE (10 $\mu$M).

**F.** Mean integral intensity of *M. bovis* BCG-GFP upon treatment of infected human primary macrophages with different concentrations of IFN-$\gamma$ for 4 hr prior to treatment with either 1$\mu$M or 10$\mu$M N or PE, for 48 hr. Significance was calculated by Student's t-test. NS p>0.05, *p<0.05, **p< 0.001, ***p<0.0001. Data are averaged from 16 images/condition and representative of 4 experiments. For B-F, error bars indicate SEM.

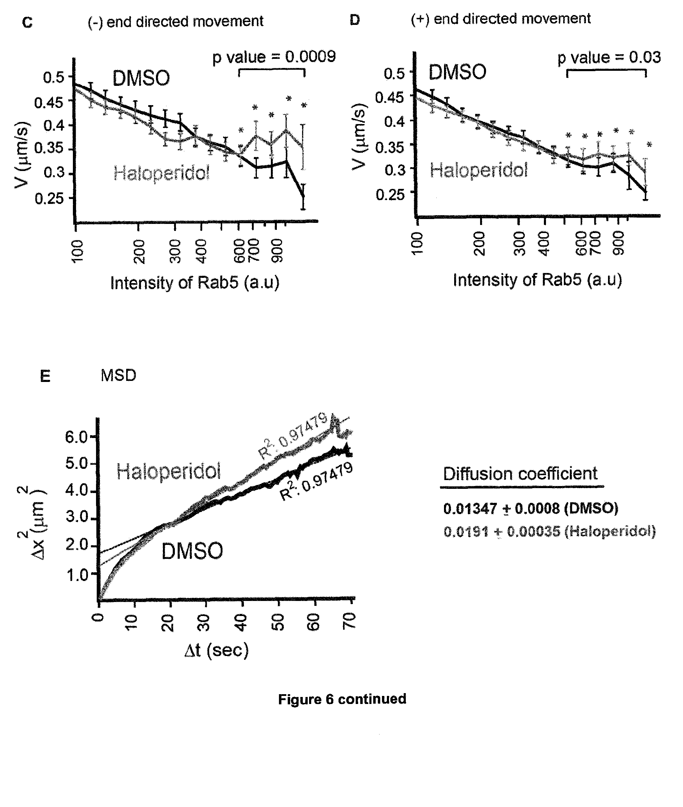

**Figure 6: Analysis of endocytosis trafficking by Haloperidol**

**A.** and **B.** The kinetics of endocytic trafficking were quantified using a cell-based LDL trafficking assay. (A) Integral intensity of LDL in early endosomes and (B) late endosomes. Values are H treatment (10$\mu$M) subtracted from DMSO and derived from an average of 1200 cells/time point. Error bars are SEM of number of images (average of 40 per condition). Data are representative of two experiments.

**C.** and **D.** A431 cells stably expressing Rab5-GFP were treated with either DMSO or H (10$\mu$M) for 2 hr and imaged live. Velocities of minus-end (towards cell centre) (C) and plus end-directed movement (away from cell centre) are shown for DMSO- (black line) and H-treated cells (grayline). For curves in B to D, the p value is for points denoted by asterisks (see Experimental Procedures). For C and D, error bars are standard deviations, n=37 (DMSO), n=29 (H).

**E.** Mean square displacement curves for the movies (Figures 6C, D) were computed as described in Experimental Procedures. The second part of the bi-phasic curve indicating long time-scale movements was fitted using linear least square fit. Diffusion coefficient is derived from the slope of the fitted curves.

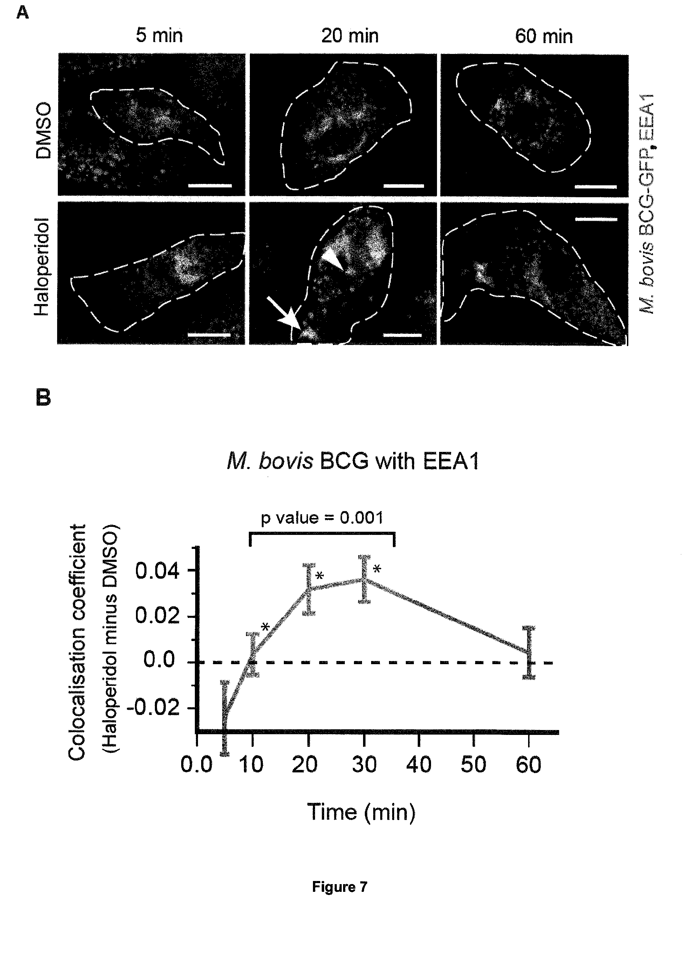

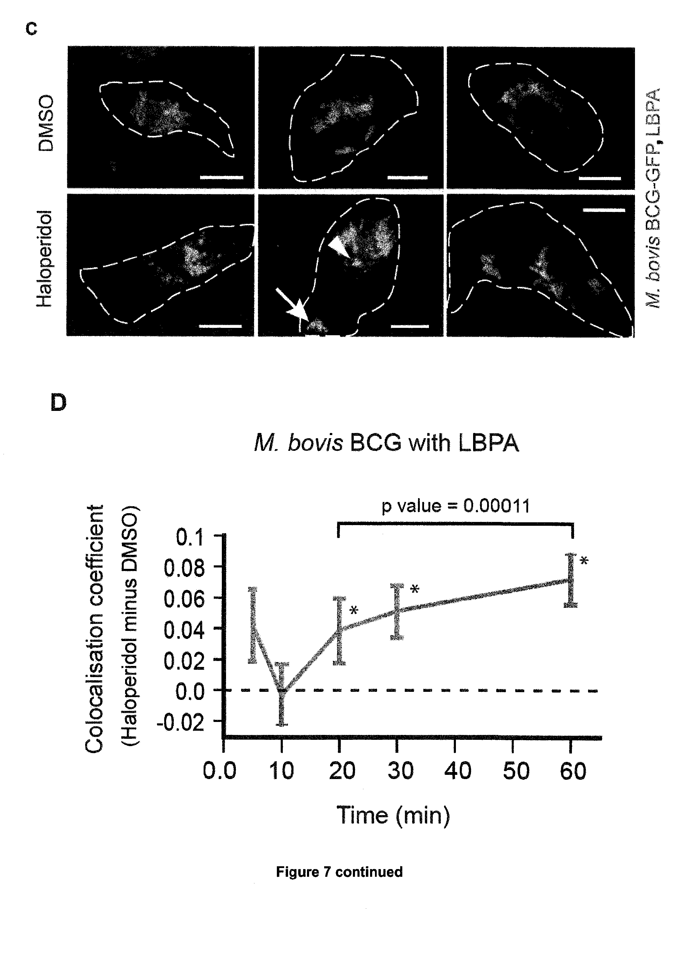

**Figure 7: Haloperidol induces alterations in intracellular mycobacterial trafficking**

**A.** and **C.** Representative images of *M. bovis* BCG-GFP-infected human primary macrophages treated with 10$\mu$M H for the indicated times and immunostained for EEA1 (A) and LBPA (C). The outline of infected cells is shown in dashed lines. Scale bar=10$\mu$m. The presence of EEA1 and LBPA on mycobacterial phagosome, as indicated by arrows and arrowheads (at 20 min) respectively, is mutually exclusive.

**B.** and **D.** Quantification of colocalization of *M. bovis* BCG-GFP with EEA1 (B) and LBPA (D). A stringency threshold of 0.35 was used. Data are averaged from about 1000 infected cells and are representative of at least two independent experiments. Error bars are SEM. The p value denotes significance for the consecutive points of the time series denoted by asterisks (see Experimental Procedures).

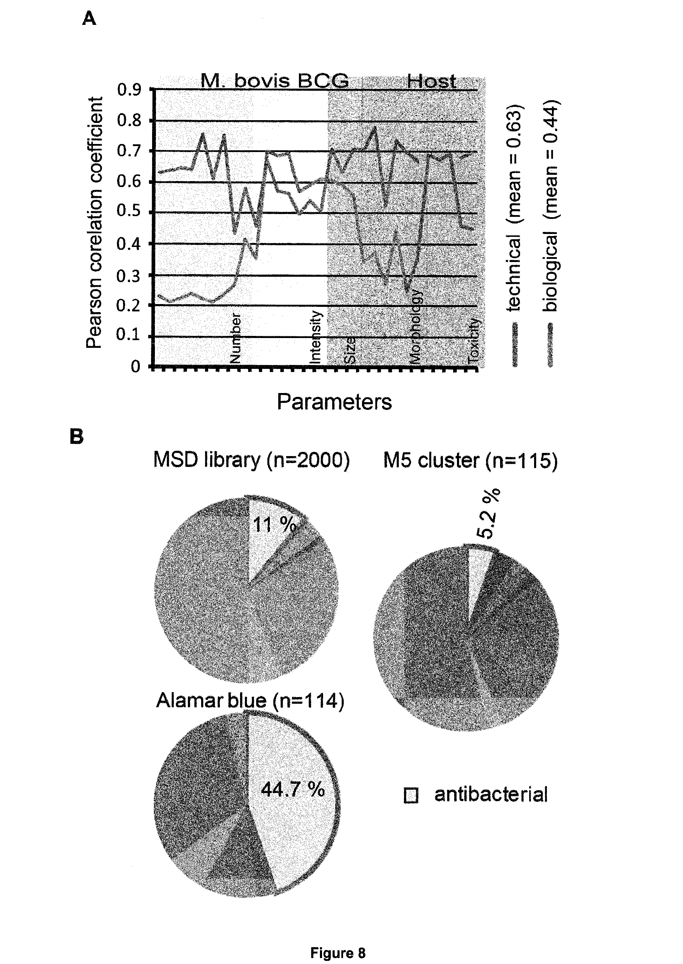

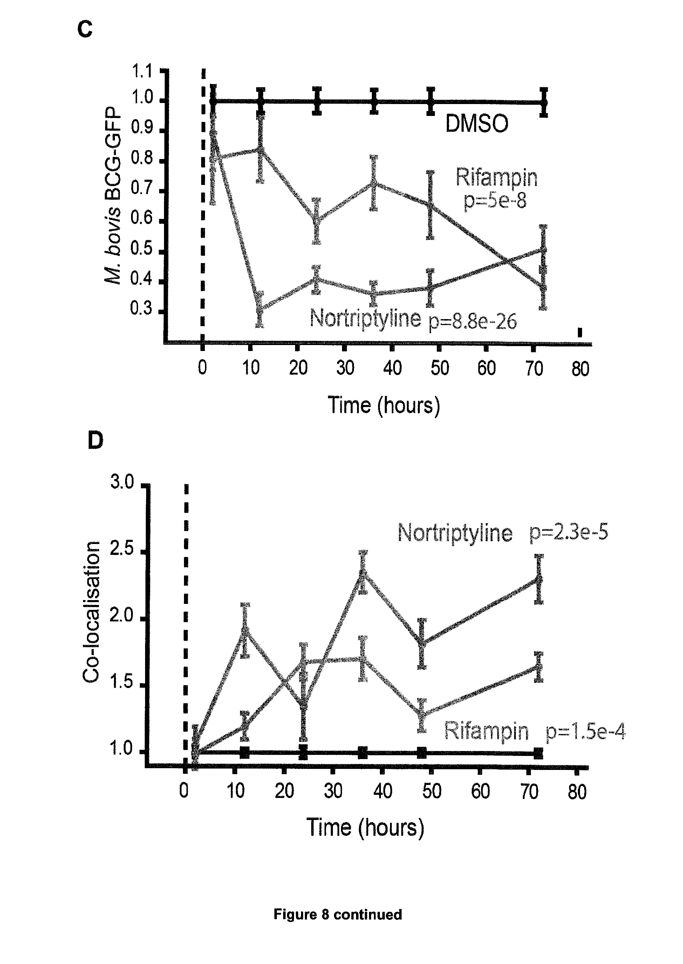

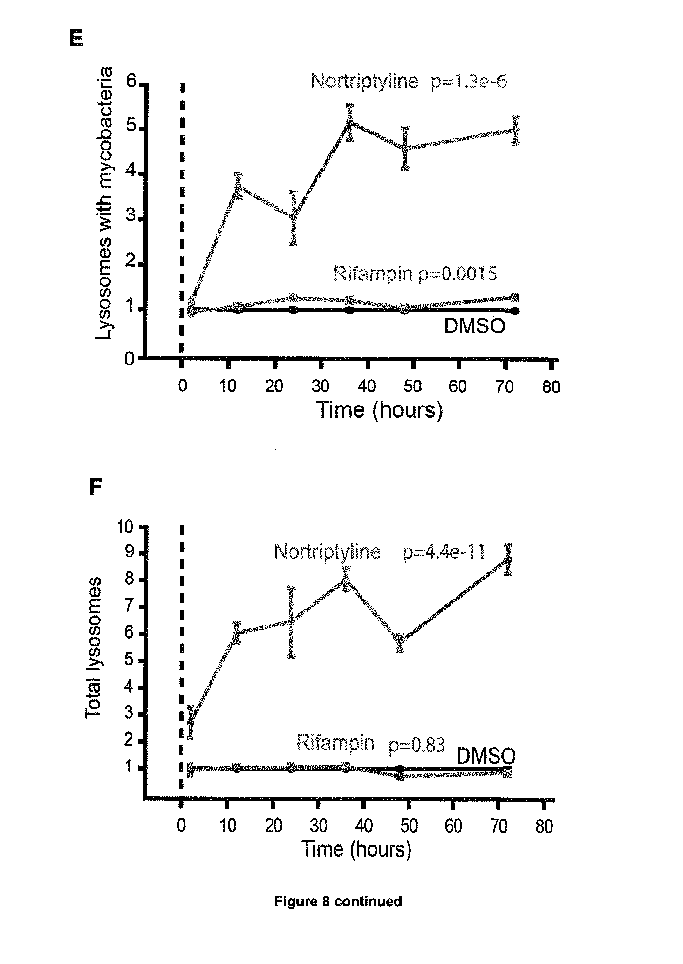

**Figure 8: Robustness of intracellular mycobacterial assay, enrichment analysis and standard anti-mycobacterial drugs in mycobacteria high content assay**

**A.** Robustness of intracellular mycobacterial assay: Pearson correlation coefficients for the parameters were computed between the biological and technical replicates of human primary monocyte derived macrophages that were infected with *M. bovis* BCG-GFP and screened with MSD spectrum chemical library. Multiple parameters were extracted as described.

**B.** Enrichment analysis: The MSD spectrum library collection is functionally annotated by the supplier (see the World Wide Web at msdiscovery.com/spectrum.html). These compounds were manually curated further to remove redundancies and to have uniform functional descriptors. Functional enrichment analysis of compounds from MSD spectrum chemical library was performed on the whole library as well as hits from the alamar blue screen and cluster M5 from the mycobacteria high content screen. The various functional categories are indicated in shades of grey in the chart. While compounds annotated as 'antibacterial' are represented by 11 % of the library compounds, they are enriched to 44.7 % in the hit list of Alamar blue screen. However, the mycobacteria cluster M5 showed a representation of only 5%.

**C. to F.** Standard anti-mycobacterial drugs in mycobacteria high content assay: Human primary monocyte derived macrophages were infected with *M. bovis* BCG and treated with the indicated compounds for the specified periods of time. Images were analyzed as described earlier. Quantification of integral intensity of mycobacteria (C), colocalisation of mycobacteria with lysosomes, (D), integral intensity of lysosomes that contain mycobacteria (E) and integral intensity of total cellular lysosomes (F). For all the graphs (C-F), data are scaled to indicate fold change over DMSO at each time point. Data are average from 45 images per condition per time point and representative of two independent experiments. For panels C-F, the concentrations used: DMSO, 0.01%, Nortriptyline, 10 $\mu$M, Rifampin, 10 $\mu$M.

**Figure 9: Bacterial morphological parameters are indicative of intracellular 'fitness', effect of selected compounds on extracellular mycobacterial growth, quantitative colocalisation scoring and host correlates of structural analogues of selected hit compounds**

**A.** Bacterial morphological parameters are indicative of intracellular 'fitness': The three selected hit compounds from the mycobacteria high content screen were analyzed for additional parameters reporting morphological features of bacteria (Table 3). Error bars are standard errors of measurement from an average of 20 images per condition.

**B.** and **C.** Effect of selected compounds on extracellular mycobacterial growth:

**B.** *M. bovis* BCG-GFP was grown in 7H9 liquid medium in the presence of indicated compounds (10 $\mu$M) and bacterial growth was monitored over time by measurement of OD$_{600}$. Amikacin (200 $\mu$g/ml) was used as a positive control. The error bars are SD (n=6) and the results are representative of three experiments.

**C.** *M. bovis* BCG-GFP was grown in liquid medium with different pH and incubated with indicated drugs at 10 $\mu$M concentration for 48 hours, followed by alamar blue assay. Error bars are SD of quadruplicate measurement and data representative of four experiments.

**D.** and **E.** Scoring colocalisation quantitatively: Human primary monocyte derived macrophages were infected with *M. bovis* BCG-GFP and stained with lysotracker red. Mycobacteria and lysosomal objects were segmented as described. Typically, cells show a range of co-localisation between *M. bovis* BCG-GFP and lysotracker red, from complete non-colocalisation (A), partial colocalisation (B) to complete colocalisation (C). Quantitative co-localisaiton profiles of the individual cells (A, B or C) are shown in the colocalisation curve (D). The colocalisation coefficient shows stable results and a correct hierarchy over a wide range of colocalisation threshold. Typically, threshold values from 0.4 to 0.6 are considered in the analysis.

**F.** to **I.** Host correlates of structural analogues of selected hit compounds:

**F.** Structures of Nortriptyline and a similar compound, Amitriptyline, which was also represented in cluster M5 from mycobacterial high content screen.

**G.** to **I.** Quantitative multi-parametric profiles of Nortriptyline (light gray) and Amitriptyline (dark gray) in mycobacteria (G), endocytosis (H) and autophagy (I) assays. Profiles are generated from high content data from the respective screens. See Tables 1 and 3 for description of parameters used in the different screens. Structural similarity was assessed by setting a Tanimoto distance threshold of 0.9 between compounds.

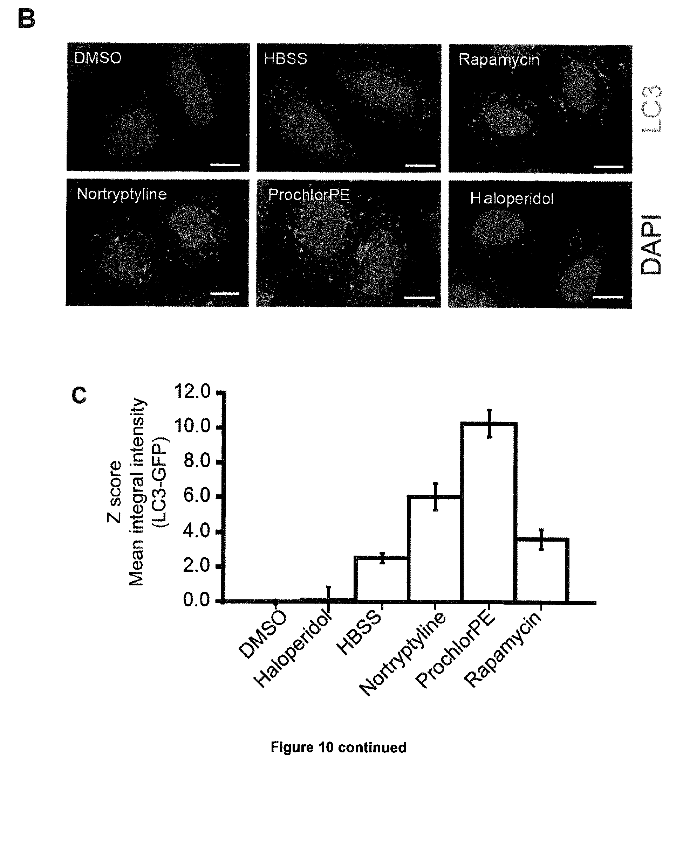

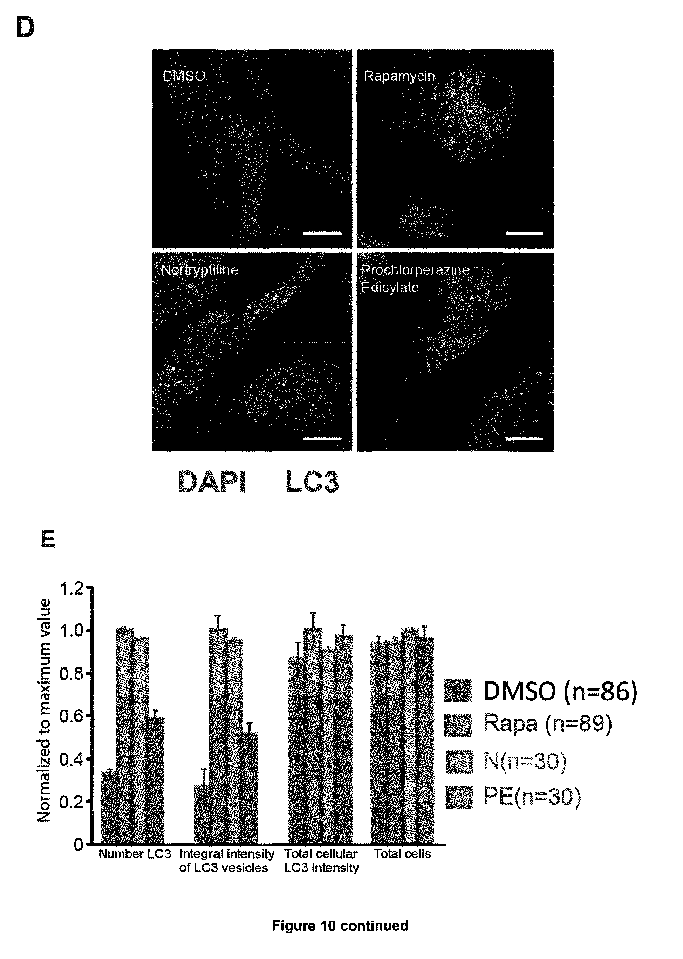

**Figure 10: Correlations between mycobacteria inhibition phenotype with autophagy parameters, Autophagy response of compounds validated for intracellular mycobacterial killing and Validation for autophagy in human primary macrophages**

**A.** Correlations between mycobacteria inhibition phenotype with autophagy parameters:

For the compounds in the endocytosis clusters, E1 and E2, the Pearson correlation coefficient was computed between the strength of the phenotype in the mycobacteria infection assay with three main autophagy parameters. The strength of the mycobacteria phenotype, which was calculated as an Euclidian length of the profile, showed a significant positive correlation to the autophagy parameters for E1, but not E2, cluster. This is consistent with the bioinformatics prediction that the compounds in E1 act predominantly by modulating autophagy. Absence of significant correlation to the number of cells indicates that mycobacteria inhibition is not result of cytotoxicity. p-values were computed using Fisher's z-transform.

**B. and C.** Autophagy response of compounds validated for intracellular mycobacterial killing:

**B.** Representative images from the autophagy screen showing HeLa cells treated with either controls for autophagy induction (Rapamycin, starvation induced by HBSS) or the compounds validated for intracellular killing of mycobacteria. Scale bar= 10 $\mu$m.

**C.** Quantification of mean integral intensity of LC3 vesicles from the high content screen for the compounds indicated. Data are represented as Z scores with DMSO as negative control. Error bars represent SEM. 25 images per condition are used. For A and B, Rapamycin was used at 5.5 $\mu$M and the other compounds were used at 10$\mu$M.

**D. and E.** Validation for autophagy in human primary macrophages:

**D.** Human primary monocyte derived macrophages were treated with the indicated compounds for four hours and immunostained for LC3. Scale bar is 10$\mu$m.

**E.** Quantification of the images as described earlier. Number and integral intensity of LC3 vesicles increase significantly with the addition of Nortryptiline and Prochloreperazine edisylate. The increase in LC3 intensity is specific to the vesicular population since the total cellular intensity of LC3 remains similar. The drugs are not toxic to cells at this concentration as seen in the stability of total cell numbers. The data are normalized to maximum value of each condition to bring them to the same scale. Error bars are SEMs. For panels B and D, Rapamycin was used at 5.5 $\mu$M, Nortriptyline and PE were used at 10$\mu$M.

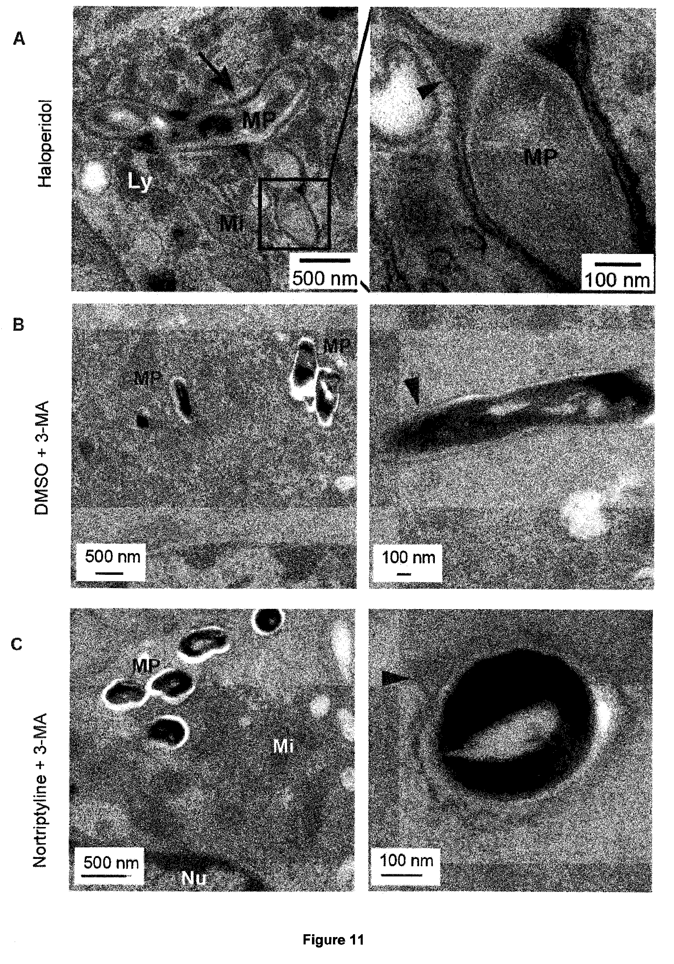

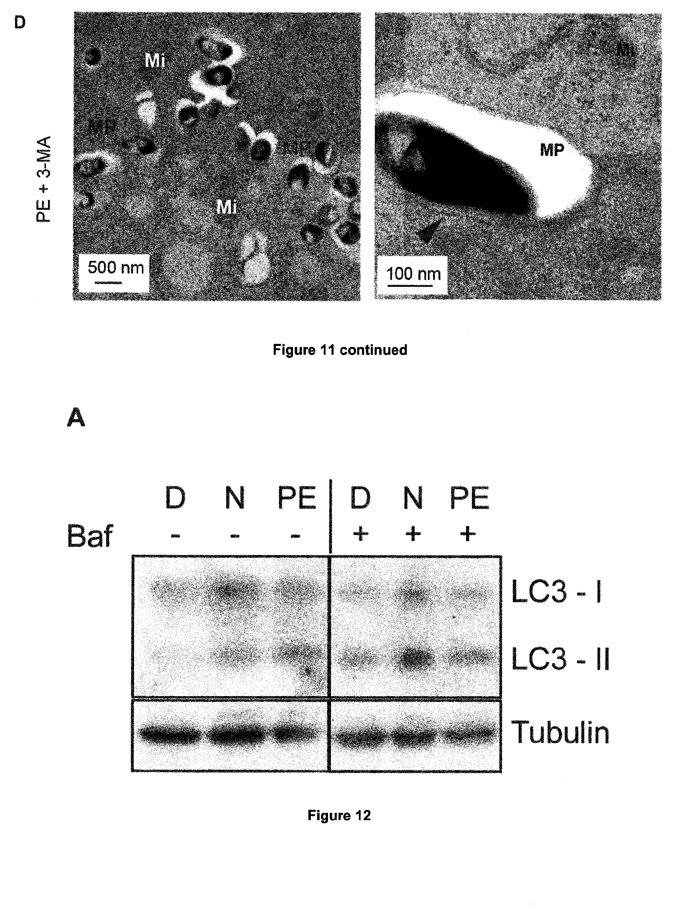

**Figure 11: EM for Haloperidol and effect of autophagy inhibitor 3-MA on action of Nortriptyline and PE**

**A.** EM for Haloperidol: Human primary macrophages were infected with *M. bovis* BCG-GFP and treated with Haloperidol (10$\mu$M) for 4 hours and processed for electron microscopy as described in methods. The images shown are representative of two independent experiments. Abbreviations: Nucleus (Nuc), mitochondria (Mi), lysosome (Ly), Mycobacterial phagosome (MP). Black arrow indicates mycobacterial phagosome and arrow head denotes phagosomal membrane.

**B. to D.** Effect of autophagy inhibitor 3-MA on action of Nortriptyline and PE: Human primary monocyte derived macrophages were infected with *M. bovis* BCG-GFP and treated with Nortriptyline (10$\mu$M) and Prochlorperazine edisylate (5$\mu$M) in the presence of 3-MA (10mM) for four hours and processed for electron microscopy, as described in methods. The images are representative of two independent experiments. Black arrow heads indicate mycobacterial phagosomal membrane. Abbreviations used: MP (Mycobacterial phagosome), Mi (Mitochondira), Nu (Nucleus).

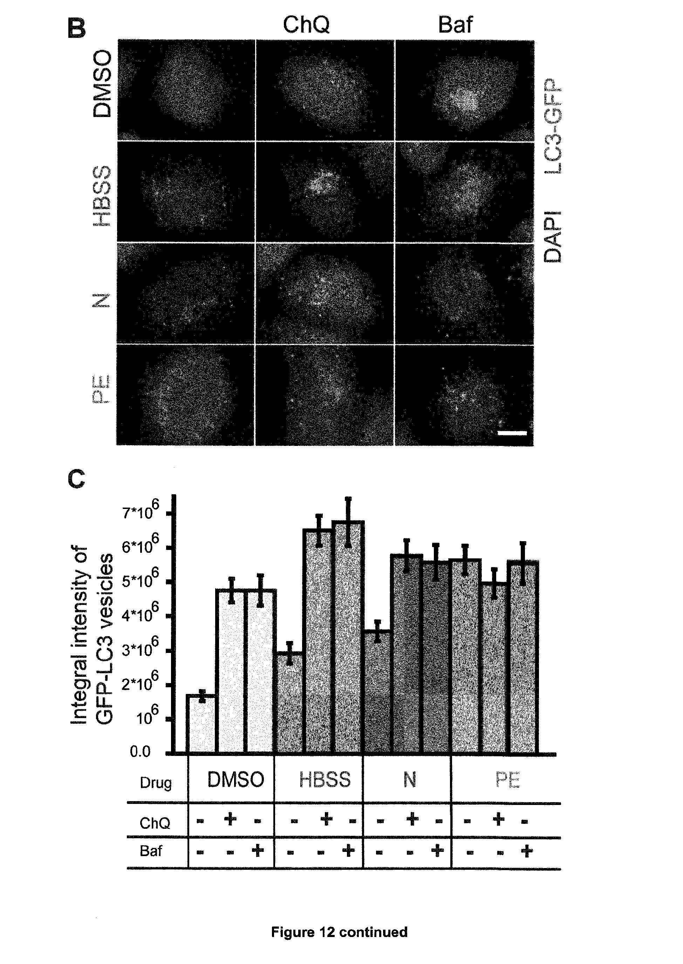

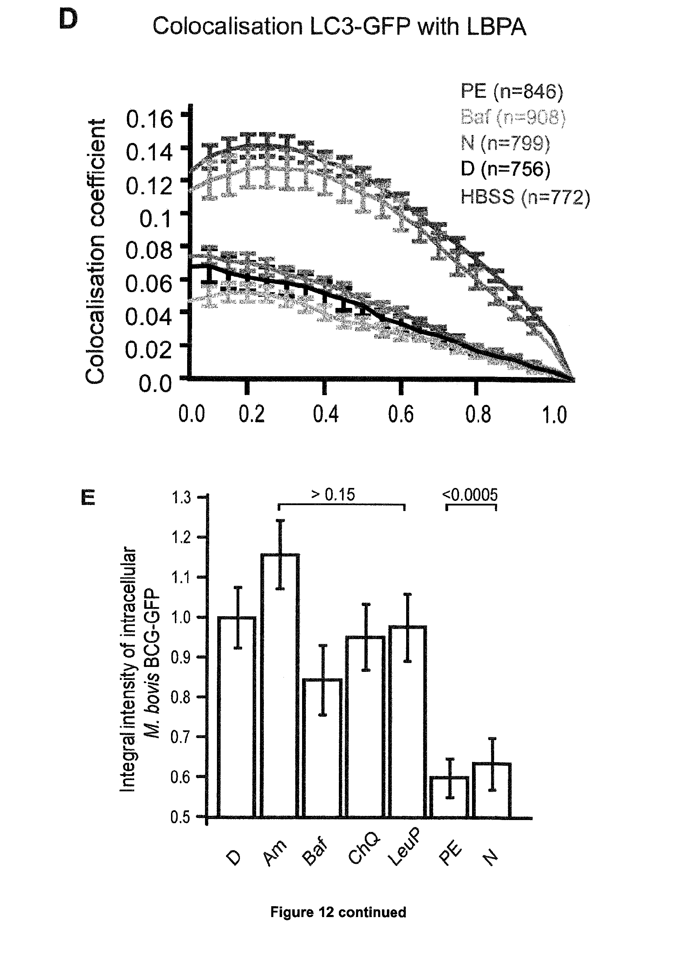

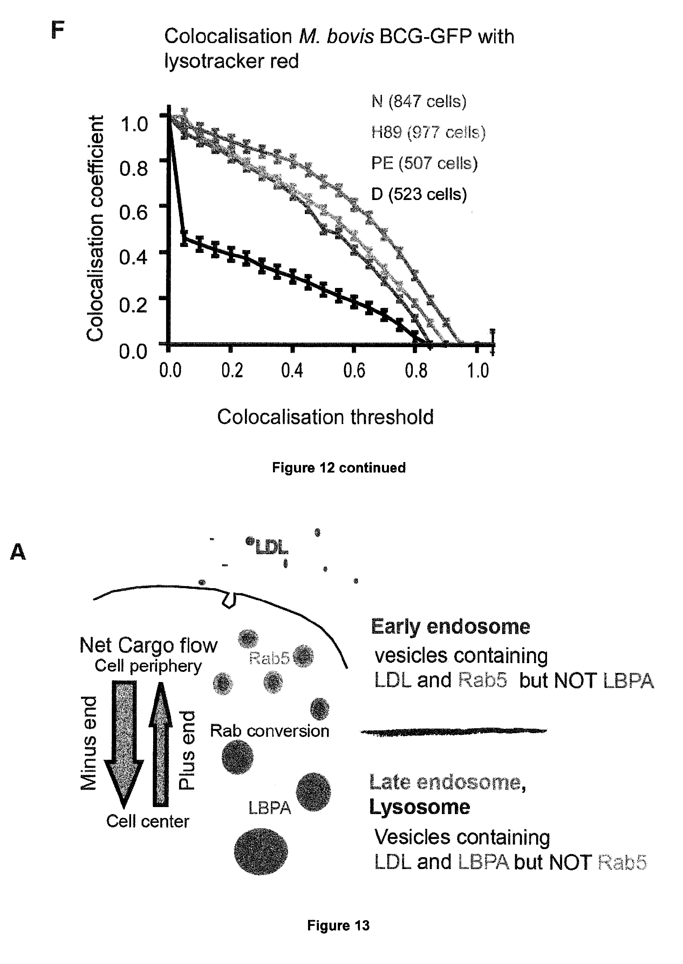

**Figure 12: Autophagy flux analysis, Colocalisation of autophagosomes with late endosomal marker LBPA and Effect of standard autophagy flux inhibitors on intracellular mycobacterial survival**

**A.** Autophagy flux analysis:

**A.** HeLa LC3-GFP BAC was treated with Nortriptyline (N) or Prochlorperazine edisylate (PE) in the presence or absence of BafilomycinA1 (Baf). DMSO (D) was used as negative control. Cells were lysed and immunoblotted for LC3. Tubulin was used as loading control. Result is representative of at least four independent experiments.

**B.** Representative images from HeLa LC3-GFP BAC cells treated with Nortriptyline (N), Prochlorperazine edisylate (PE) or starvation induced (HBSS) with or without Chloroquine (ChQ) and BafilomycinA1 (Baf) for 4 hours, fixed and imaged using automated confocal microscopy. Scale bar=10 $\mu$m.

**C.** Quantitation of autophagosome mean integral intensity for indicated conditions. Results are mean +/-SEM. (60 images per condition). Data are representative of two independent experiments.

For A, B and C, concentrations of compounds used were: DMSO, 0.01%, Nortriptyline, 10 $\mu$M, Prochlorperazine

edisylate, 10 μM, Chloroquine, 25 μM and BafilomycinA1, 100nM.

**D.** Colocalisation of autophagosomes with late endosomal marker LBPA: HeLa LC3-GFP BAC cells were treated with the indicated drugs for 4 hrs and immunostained for LBPA. Colocalisation of LC3-GFP with LBPA were quantified over a range of colocalisation thresholds, as described in methods. Number of cells per condition is given in parenthesis. Error bars are SEM. Data are representative of two independent experiments. PE (Prochlorperazine edisylate, 10 μM), Baf (BafilomycinA1, 100nM), N (Nortriptyline, 10μM), D (DMSO, 0.01%), HBSS (Hans Balanced Salt Solution).

**E.** Effect of standard autophagy flux inhibitors on intracellular mycobacterial survival: Human monocyte derived macrophages were infected with *M. bovis* BCG-GFP and treated with the indicated compounds for 48 hours. Cells were fixed, imaged and analyzed as described. Data are normalized to DMSO. Numbers above the bar graphs indicate p-values for the compounds. Data are aggregated from 60 images per condition and are representative of at least three experiments. Abbreviations and concentrations used are: D (DMSO, 0.01%), Am (Ammonium chloride, 10mM), Baf (Bafilomycin A1, 100nM), ChQ (Chloroquine, 25μM), LeuP (Leupeptin, 10μM), PE (Prochlorperazine edisylate, 10μM), N (Nortriptyline, 10μM).

**F.** Human primary macrophages were infected with *M. bovis* BCG-GFP, treated with the indicated chemicals for 48 hours and stained for the acidotropic marker Lysotracker red. Co-localisation of *M. bovis* BCG-GFP with lysotracker red was quantified over a range of thresholds. Numbers in parenthesis indicate the number of infected cells analyzed. Data are representative of at least three independent experiments. Abbreviations and concentrations used PE (Prochlorperazine edisylate, 10 μM).

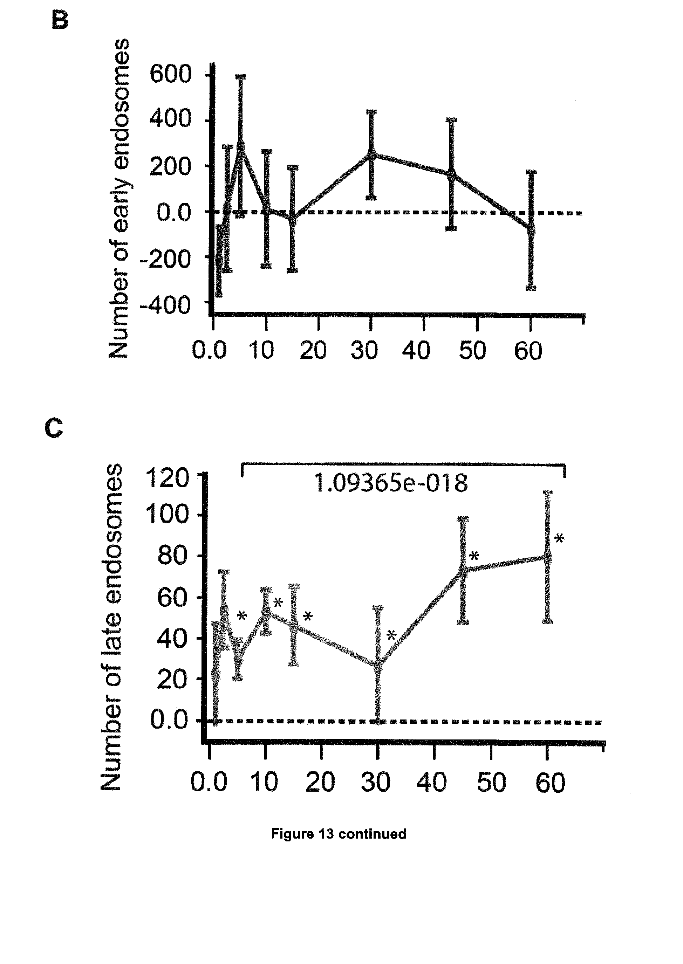

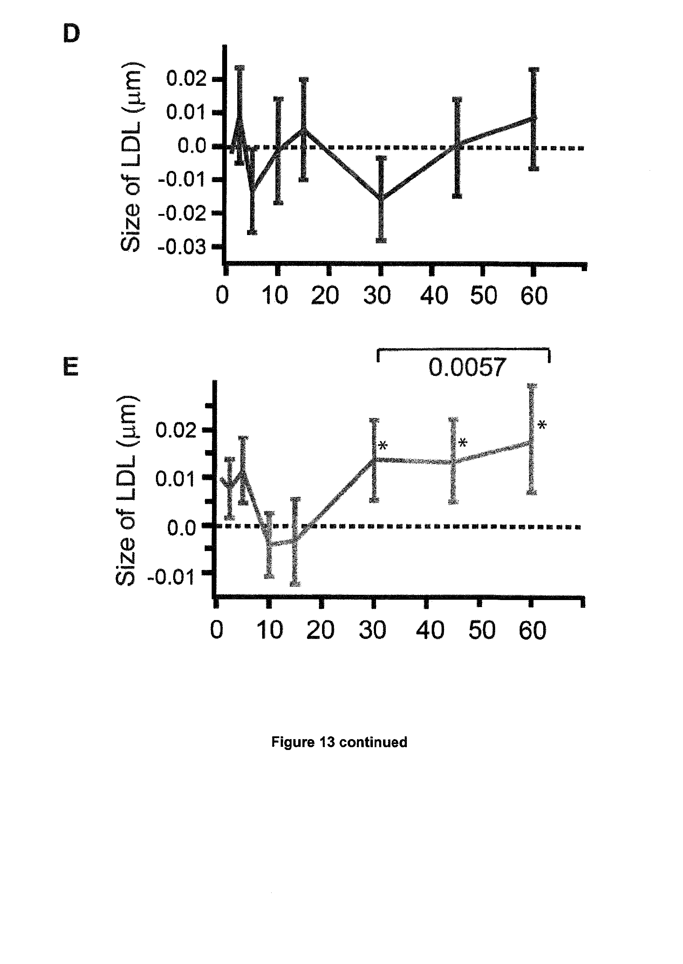

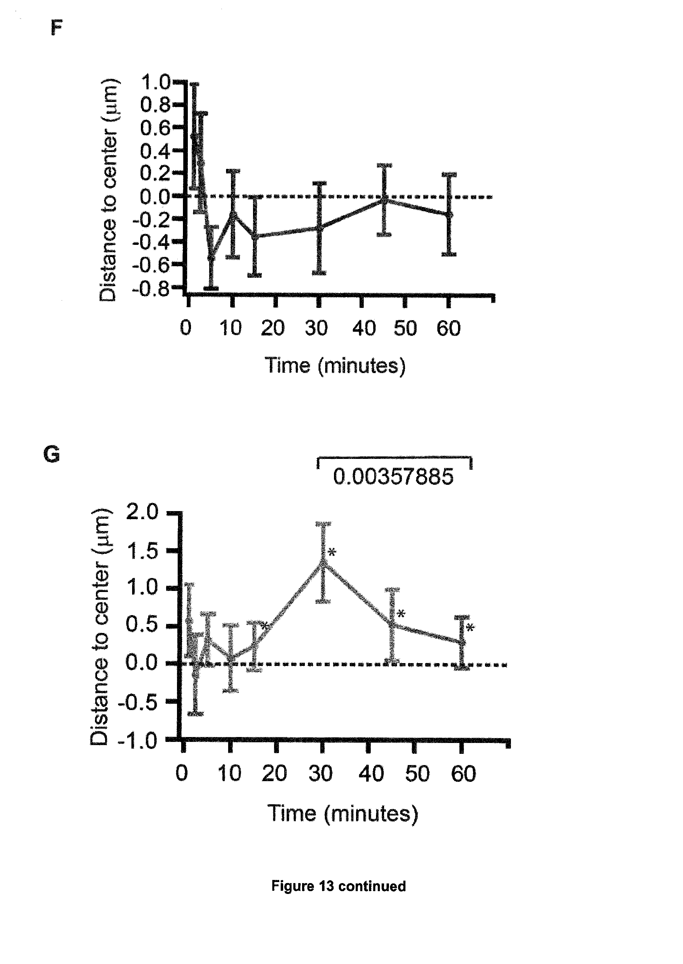

**Figure 13: Schematic of LDL trafficking assay and Additional parameters for LDL trafficking assay**

**A.** Schematic of LDL trafficking assay: HeLa-Rab5c-GFP-BAC cells were pre-treated with Haloperidol and pulsed with LDL for various times. Cells were then immunostained with ApoB and LBPA to visualize the cargo (LDL) and the compartment (late endosomes, lysosomes) respectively. Vesicles containing LDL and Rab5 but not LBPA were considered early endosomes, while vesicles containing LDL and LBPA but not Rab5 represented late endosomes in the following analysis.

**B. to G.** Additional parameters for LDL trafficking assay: HeLa Rab5c-GFP-BAC cells were pretreated with 10 μM Haloperidol and pulsed with LDL for various times and various parameters such as number (B,C), size (D,E) and distance to center (F,G) calculated for LDL containing early (dark gray curves) and late endosomes (light gray curves). Curves are represented as values for Haloperidol treated cells subtracted from DMSO. All parameters show significant increase in late, but not early, endosomes showing that trafficking of LDL containing endosomes is accelerated along degradative pathway by Haloperidol. Values are derived from an average of 1200 cells per condition. Error bars are standard deviations of number of images (average of 40 per condition). Data are representative of two experiments. The p value indicating significance is calculated for the time series as indicated in supplementary methods. Asterisks indicate the consecutive points used to calculate p value.

The examples illustrate the invention:

**Example 1:** Experimental Procedures

**1.1. High content screen for intracellular mycobacteria survival.**

Human primary monocyte-derived macrophages were infected with M. *bovis* BCG-GFP for 1 hr at MOI of 1:10, washed extensively (Biotec EL406). After adding the compounds (10μM) (Beckman Fxp automatic workstation), cells were incubated for 48 hr, fixed with 3.7% PFA (Sigma), stained with 1μg/ml DAPI, 3μg/ml Cell Mask Blue (Invitrogen) and imaged using the automated spinning disk confocal Opera (Perkin Elmer).

**1.1.1 Additional details on High content assay for mycobacterial survival**

**Cells :** Buffy coats were purchased from German Red Cross. Human primary monocytes were isolated from pooled buffy coats (3-4 donors per pool), seeded directly on assay plates (384 well, Greiner microclear) at a cell density of 15,000 per well and allowed to differentiate to macrophages by addition of 50 ng/ml hMCSF (peprotech). Media was exchanged after 3 days. Infection was done after 6 days. **Imaging:** Plates were sealed with aluminium tape and imaged using automated spinning disc confocal Opera (Evotec) with a 40x, 0.9 NA water objective. 30 images from each well (randomized intra well positions) were acquired in two channels with excitation filter at 405nm for host cell detection (DAPI/CMB) and 488nm to detect *M. bovis* BCG-GFP. Different cameras were used to image the different channels thus ensuring no bleed through of signals.

**Image analysis:** To correct for chromatic shifts due to the different cameras used, multi-colour beads were acquired with identical settings in each imaging session and were used to determine the correction factor for XY shift (Collinet *et al.* 2010). Chromatic and Illumination shift corrected images were then used for further analysis in a two step process. First, host cell nuclei and bacteria were identified from the images as described in Collinet *et al.*. Additionally, host cell boundaries were determined based on Cell Mask Blue staining by a watershed-based region growing algorithm (Parvati 2008; Roerdink 2000). Briefly, nuclei regions are used as seeds and expanded till a predefined masked area, representing the area covered by cells. The masked area is identified based on automatic maximum entropy-based thresholding. The boundaries of neighbouring cells touching each other are placed in the area of intensity minima between the two cells, according to the watershed principle. In order to avoid over-fragmentation, the watershed algorithm is preceded by maxima imposition, so that no new local maxima are considered outside the nuclear region. Segmentation of cells thus allowed for reliable and accurate quantitation of intracellular bacteria in addition to assessment of changes to host cells upon perturbation. In the second step of image analysis, multiple parameters relating to both bacteria and host cells were extracted (see Table 1 and details below).

### 1.1.2 Quality control metrics for Mycobacteria high content screen

Since the screening is based on quantitative image analysis, various quality control metrics were implemented to ensure the robustness of the results. The correlations of the results between replicates were measured. Towards this, a subset of MSD chemical library was screened in technical triplicates and the Pearson correlation coefficient was calculated for all parameters between the triplicates. The results (Figure 8A) show high level of correlation for all parameters groups with an average Pearson correlation coefficient of 0.631, indicating a high level of reproducibility. Since human primary monocyte derived macrophages were use, large variations between different preparations of the cells were anticipated. In order to quantify this, the MSD screen was performed in biological duplicates (different cells and infection) the Pearson correlation coefficient was calculated between the duplicates. The results (Figure 8A) show that parameters relating to intensity of bacteria as well as host toxicity are well conserved, while those relating to number of bacteria are lower.

### 1.1.3 Description of parameters of the mycobacterial high content assay

The image processing pipeline allowed to segment objects (bacteria and cells) from the images. Following this, quantitative multi-parametric image analysis algorithms calculated different statistical parameters describing multiple features for the two categories of objects (see complete list in Table 1). Parameters corresponding to bacteria were split into three groups, number-related, intensity-related and shape-related, giving information about different facets of intracellular bacteria. Number-related parameters report the numbers of bacteria within infected cells, number of bacteria within all cells, total number of bacteria in infected cells as well as the total number of infected cells. Intensity-related parameters compute the average as well as integral intensity of bacteria within infected cells. Integral intensity is defined as the integral (the sum pixel by pixel) of the intensity calculated on the fitted object, mean integral intensity is the mean value integral intensity of individual bacteria and total integral intensity is the sum of all bacterial integral intensities, normalized by the area covered by cells.

These parameters are measured in multiple ways with different statistics (mean, median or mode) and different weighting: parameters can be non-weighted, weighted by mean intensity or weighted by integral intensity. Weighting can reveal important biological features: measuring the mean parameter value gives us information about whole population behaviour, including both bright (presumably intact) and dim (presumably partially degraded) bacteria at the same time, parameter weighted by intensity gives us information mostly about brighter (and presumably) bacteria. This approach extracts more comprehensive description of the phenotype from the same biological assay.

In case of normal distribution of a parameter, the mean, median and mode coincide. However, this is not the case for most of the parameters in the present assay. Parameter distributions are skewed and have heavy tails. By using a combination of mean, median and mode, it is possible to summarize the main features of the distribution in a few values without making any assumption about distribution per se. For example, mode of the distribution tells us the parameter value of the most abundant objects while mean and median reflect on different features of distribution. Several of these parameters are closely related but computed in different ways (such as number of bacteria per infected cell and number of bacteria per cell), thereby also serving as measures of robustness and reliability of parameters and measurement.

Several host cell parameters were also measured (Table 1). These parameters capture the dynamics of alterations in the host cell as a consequence of perturbation. Additionally, quality control and 'toxicity' parameters were included. 'Nuclei contrast' estimates the correctness of focal position in the given image by measuring the difference in contrast between nuclear signal DAPI and nearby cytoplasmic pixels. 'Number of cells' served as a first level measure for toxicity. For the following analyses, only those conditions with at least 5 images, each image containing more than 4 cells, were considered. Therefore, extremely toxic compounds, those that lead to cell death and hence no measurable nuclei, are automatically filtered out from analysis. As an additional level of toxicity assessment, area and size of nuclei were measured, since nuclear condensation is an initial signature of apoptotic cell death and a general sign of loss of cellular fitness (Galluzziet al. (2009); Parvati and Mariya Das (2008); Roerdink (2000). Indeed these measurements for toxicity correlate well with standard toxicity assays such as PI exclusion (data not shown).

### 1.1.4 Bacterial morphology related parameters

In order to correlate the phenotypic profiles of selected hit compounds with the intracellular 'fitness' of mycobacteria, additional parameters were calculated (Table 2), reporting morphological features of bacteria. The profiles (Figure 9A) show significant influence of the selected hit compounds on bacterial morphological features. All the parameters are significantly altered by the selected compounds. Taken together with the phenotypic profile from the screen, these alterations can be summarized as: fewer bacteria that are smaller, less intense, less elongated, more compact and circular. This could be interpreted as partially degraded bacteria with remaining GFP signals. These parameters could thus offer a metric for assessment of intracellular 'fitness' of mycobacteria.

### 1.1.5 Standard anti-mycobacterial compounds in the high content mycobacteria assay

Standard anti-mycobacterial drugs did not fall into the hit cluster but were rather spread in different clusters. Additionally their phenotypes were weaker compared to the selected hit compounds. In order to understand this better, their activity was further characterized by improving the resolution of the assay, i.e. a detailed time course analysis of mycobacterial trafficking as well as survival was performed. The results (Figure 8C to F) show that the standard anti-mycobacterial drug Rifampin exhibited a phenotype with time. This phenotype corresponds to:

a) Decreased mean integral intensity of bacteria
b) Increased co-localisation of mycobacteria to lysosomes
c) Increased intensity of lysosomes that contain bacteria.

However, the phenotype is weaker than that of the hit compounds for most time points. One possible reason could be that the read out is dependent on the GFP fluorescence from mycobacteria, which could be reduced at different rates, depending on whether the lysosomal degradation machinery is accelerated or not by host acting mechanisms. In support of this, the hit compounds lead to a strong increase in the intensity of lysosomes, whereas Rifampin, that kills mycobacteria directly, does not show this effect (Figure 8F). This could lead to different rates of degradation of GFP from mycobacteria thus resulting in the different phenotypes seen.

### 1.2. Data analysis: clustering algorithm

Multi-parametric profiles from the chemical screen were clustered using an algorithm based on modified Gabriel neighbourhood and density estimate using von Mises-Fisher kernel. This clustering algorithm has one free parameter, namely kernel bandwidth, which is inversion of von Mises-Fisher concentration parameter $\kappa$. For every dataset, the number of clusters as function of kernel bandwidth has a pronounced plateau within a $\kappa$ range of 10-150. Further increase of $\kappa$ would not lead to an increase in the number of clusters. For each dataset, a cluster was manually selected at the first value of $\kappa$ that produced clusters that were stable in number and size. $\kappa$ values for mycobacteria and endocytosis screens were 30 and 90, respectively.

### 1.3. Integrating chemical and genetic screens

Representative profiles of the endocytosis sub-clusters were generated by averaging the profiles of individual compounds in each sub-cluster. For these profiles, we assembled a list of genes from the endocytosis screen (Collinet et al., 2010) that had a PSS > 0.95 and a Pearson correlation of 0.7. These lists were subjected to gene annotation enrichment analysis using a standard hypergeometric test. Gene annotations were obtained from Gene Ontology and KEGG Pathway databases.

### 1.4. Intracellular mycobacterial trafficking assay

Human primary monocyte-derived macrophages were infected with *M. bovis* BCG-GFP and stained for lysotracker red and LBPA as markers of late endosomes and lysosomes (Kobayashi et al., 1998; Schmid and Cullis, 1998). Co-localization was scored over a range of stringency thresholds varying from 0 (most relaxed) to 1 (most stringent).

### 1.5. Alamar blue screen for anti-bacterial compounds

To mid log phase *M. bovis* BCG-GFP seeded into 384 well plates in a volume of 90 µl 7H9 [Is the term 7H9 sufficiently established in the art so that the skilled person knows what to use here?], compounds were added to a final concentration of 10µM DMSO and Amikacin (200 µg/ml) were included as negative and positive controls, respectively. Plates were incubated at 37°C for 4 days, followed by addition of 10µl of 1:1 mix of alamar blue (serotec) and 10% Tween-80. After 24hr at 37°C, fluorescence was read in the Tecan Pro (Ex 535 nm; Em 590 nm). Analysis was done per plate. Compounds with a Z score > 3 were considered hits. The screen was repeated twice (Z' factors are 0.85 and 0.9 for each run). Hits from both runs were combined.

### 1.6. High content autophagy assay

HeLa cells were treated with the compounds at a final concentration of 10µM, incubated for 4 hr, fixed with 3.7% PFA and immunostained with anti-LC3 antibody (clone PM036, MBL labs). Cells were counterstained by DAPI and Cell Mask Blue, imaged and analyzed as described above.

### 1.7. Dose response curves

To calculate $IC_{50}$ for mycobacterial survival, human primary monocyte-derived macrophages were infected with M. *bovis* BCG-GFP and treated with different doses of Nortryptyline (D) and Prochlorperazine edisylate (E) for 48 hrs and fixed. For autophagy assays, the same preparation of cells was treated with the two compounds for 2 hrs, fixed and immunostained for LC3. Cells were imaged and analyzed as described.

### 1.8. *M. tuberculosis* CFU assay

Human primary macrophages were infected with virulent *M. tuberculosis* strain H37Rv and two different clinical isolates (CS1 and CS2) for one hr at MOI 1:10, washed, treated with Amikacin (200µg/ml) for 45 min, washed again and treated with the compounds. Cells were lysed after 48 hrs with 0.05% SDS and plated in multiple dilutions on the standard mycobacterial growth medium Middlebrook 7H11 agar plates. Colonies were counted after incubation at 37°C for 3 weeks.

### 1.9. Electron microscopy

Cells were fixed with 2.5% glutaraldehyde followed by post-fixation in Ferrocyanide reduced Osmium following standard protocols (Kamovsky, 1971) and embedded with epon. 70nm thin sections were cut on a Leica ultramicrotome, stained with Uranyl Acetate and Lead Citrate following classical procedures and imaged on a 100kV Tecnai 12 TEM microscope. For area measurements, several overlapping high magnification images covering the whole cell were acquired and a montage was generated using ImageJ.

### 1.10. Live cell imaging

A431 cells stably expressing GFP-Rab5 were treated with 10µM Haloperidol for 2 hr and imaged live using Zeiss Duo scan microscope at 0.098 sec /frame for 2 min. Individual endosomes were identified and tracked over time as described (Rink et al, 2005). To calculate mean square displacement, all endosomal tracks were first iteratively divided into non-overlapping equal time intervals from 0.1 to 70 sec and their displacements were computed for each interval. Next, the squares of displacement for all endosomal tracks for a given condition were averaged and plotted as a function of the time interval. In such a curve, early endosomes show a characteristic bi-phasic behavior, which is referred to as a short-time scale and long-time scale movements.

### 1.11 Chemical structural analogues

It was further investigated if structurally similar compounds also clustered together in the mycobacteria and endocytosis screens. The structures were delivered in Structure Data format by the vendor and converted to the Chemical Development Kit (CDK) format for further similarity analysis. Standard fingerprint was generated for all compounds, and fingerprint similarity for each compound was calculated iteratively as the Tanimoto similarity coefficient with every compound of the library, resulting in a 2000 by 2000 matrix of similarity measures. All pairs of compounds with Tanimoto similarity coefficient higher than 0.9 were considered structurally related compounds, as suggested by Pubchem (see the world wide web at pubchem.ncbi.nlm.nih.gov/help.html#sAssayclustering/).

Of the three validated compounds, only Nortriptyline had a structurally related compound in the hit cluster, namely Amitriptyline (Tanimoto distance of 0.96). Nortriptyline and Amitriptyline showed highly correlated profiles in mycobacteria high content screen, as well as in endocytosis and autophagy screens (Figure 9F to I). Thus structural analogs show similar host correlates, further confirming that the effect of these compounds on intracellular mycobacterial survival is by modulating the host.

**Estimation of p-value for time series**

The null-hypothesis tested is that deviation of a sequence of N measurements from zero is the result of normally distributed noise. Given that every measurement i in the series has a value $\Delta_i$ and variance $\sigma_i^2$, the probability of the null-hypothesis (p-value) was computed as

$$p_{value} = \frac{1}{2}\left(1 - erf\left(\frac{1}{\sqrt{2}}\left(\frac{\left(\sum_{i=1}^{N}\frac{\Delta_i}{\sigma_i^2}\right)^2}{\sum_{i=1}^{N}\frac{1}{\sigma_i^2}}\right)\right)\right).$$

**Example 2: High content chemical screen to identify intracellular modulators of mycobacteria**

An integrated experimental and computational strategy was developed for screening, selecting and validating compounds endowed with anti-mycobacterial activity by acting on the host machinery (Figure 1A). First, a high content assay was established in a human primary macrophage infection model. Briefly, cells were infected with *M. bovis* BCG expressing GFP (Dhandayuthapani et al., 1995), and incubated with chemicals for 48 hrs. Since the primary interest was to identify chemical modulators disrupting the host-mycobacteria equilibrium, chemical perturbations were introduced after the infection was established. Cells were then fixed, nuclei and cytoplasm stained, and imaged using an automated spinning disc confocal microscope. A QMPIA platform previously used was developed further to automatically identify cells, endosomes and nuclei (Collinet et al., 2010; Rink et al., 2005) by implementing automated algorithms to detect various features of intracellular bacteria and host cell simultaneously (Figure 1B, Tables 1, 2). A bioactive library (Microsource Discovery Inc., Spectrum collection, 2000 compounds) comprising most FDA approved drugs was screened. The compounds produced reproducible alterations of various parameters such as bacterial number, intensity as well as bacterial and host cell morphology (Figure 8A).

Cluster analysis was performed using the algorithm described above (see Example 8 for details). The compounds grouped into six distinct clusters, each with a distinct pattern, i.e. phenotypic profile (Figure1C,D). The strength of the phenotype of a compound is given by its *Pattern-specific score* (PSS) (not shown). Due to the multi-parametric nature of our screening output, each cluster was expected to capture distinct phenotypic features of mycobacteria and the host. Cluster M5 (containing 115 compounds shown in Table 4) was of particular interest as it was characterized by a strong reduction of bacterial parameters but relatively weak changes in host parameters reflecting toxicity, suggesting that the compounds in this cluster are detrimental to bacteria but not significantly toxic to the host.

Since the primary interest was to identify compounds that act upon the host, counter-selection was carried out for those compounds directly killing mycobacteria. The MSD library was screened using the alamar blue assay (see methods) and, interestingly, it was found that only 6 compounds were shared with cluster M5, suggesting that most compounds in this cluster are not bacteriostatic. Additionally, compounds functionally annotated as 'anti-bacterial' were not enriched in cluster M5 despite scoring in the alamar blue screen (Figure 8B). Standard anti-mycobacterial drugs were not enriched in any particular cluster and yielded weaker phenotypes than other compounds (Figure 8C to F). This is interpreted as being due to the specific characteristics of the cell-based assay that, by measuring the reduction in GFP fluorescence primarily reflects modulation of lysosomal degradation. Thus, the compounds of cluster M5 could modulate cellular processes at the host-pathogen interface rather than acting directly on the bacteria.

**Example 3: Validation of three compounds on M. *tuberculosis***

To validate the results from the screen, three compounds were selected from the cluster M5, namely Nortriptyline, Prochlorperazine edisylate (PE) and Haloperidol (Figure 2B-D), on the basis of

1) amplitude of the phenotypes (Figure 2E, 9A), showing reduced bacterial intensity, fewer bacteria and infected cells;
2) lack of effect in the alamar blue screen.

Additionally, these compounds did not show bacteriostatic activity on M. *bovis* BCG-GFP at neutral and acidic pH (Figure 9B,C), or plate assay of *M. tuberculosis* (data not shown).

To determine whether the phenotype observed on *M. bovis* BCG-GFP were reproducible on pathogenic *M. tuberculosis,* a colony forming unit (CFU) assay was performed on *M. tuberculosis* strain H37Rv as well as two clinical strains using the three selected compounds and the anti-mycobacterial drug Rifampin, Rapamycin (Gutierrez et al., 2004) and H89 (Kuijl et al., 2007) as controls. All three selected compounds caused a significant reduction in bacterial colonies in all the strains (Figure 2F), confirming that they impair intracellular survival of *M. tuberculosis.*

**Example 4: Integration of multi-parametric chemical and genetic data-sets to identify cellular processes modulated by the compounds**

Since the aim was to identify compounds restoring mycobacteria-arrested phagosome maturation, the effects of the selected compounds on mycobacterial trafficking was explored. The co-localization of *M. bovis* BCG-GFP with Lyso-bisphosphatidic acid (LBPA), a marker of late endocytic compartments (Fratti et al., 2001; Kobayashi et al., 1998) was quantified at the level of single infected cells, . Briefly, individual objects are fitted as described earlier, and their levels of co-localisation between each other quantified over a range of stringency thresholds. Figure 9D, E provides illustration of the variations observed in co-localisation between *M. bovis* BCG-GFP and lysosomal marker. Hence to obtain robust statistics, co-localisation events from a large number of individual infected cells are quantified. Infected cells treated with the selected compounds showed significantly higher colocalization than control cells (Figure 2G), indicating that the arrest in phagosome maturation is reverted.

A significant challenge in phenotypic chemical screens is to gain insights into the mechanism of action of the selected compounds. Multi-parametric profiles are specific signatures of either chemical or genetic perturbations because they capture multiple facets of a biological system. Since random matches between such profiles are unlikely, clues into the molecular processes targeted by the chemicals can be obtained in principle by systematically searching for genetic alterations that produce similar phenotypes. Hence, it was the aim to match the multi-parametric profiles of the compounds from the mycobacteria screen with those of the siRNAs from an genome-wide endocytosis screen (Collinet *et al.,* 2010). However, the two screens are not directly comparable due to their distinct characteristics, i.e. one being a chemical screen on intracellular mycobacterial survival in human primary macrophages and the other a functional genomics screen on endocytosis in HeLa cells. To overcome this problem the two data-sets were bridged via an intermediate screen, where the compounds from cluster M5 were screened on the endocytosis assay in HeLa cells (Figure 3A). HeLa cells were pre-treated with the compounds prior to the internalization of fluorescently-labeled epidermal growth factor (EGF) and Transferrin (Tfn) for 10 min (Collinet et al., 2010), imaged and multiple parameters extracted (Table 3).

Clustering separated the compounds into two sub-clusters E1 and E2 (Figure 3B, Table 5), suggesting that the compounds may act by at least two distinct mechanisms. To identify them, the multi-parametric profiles of the sub-clusters E1 and E2 were compared to a genome-wide RNAi screen data-set (Collinet *et al.,* 2010) and the sets of genes that are most likely to show similar phenotypes was identified (Experimental Procedures). Their representation in KEGG pathways and GO terms was computed and it was found that "mTOR regulation" and "Regulation of autophagy and mTOR" were significantly enriched in E1 ($p=1.2*10^{-4}$ and $9*10^{-4}$, respectively) but not E2 ($p=0.15$ and $0.4$, respectively) sub-clusters.

**Example 5: High content autophagy chemical screen**

Autophagy is a key process regulated by mTOR (Laplante and Sabatini, 2012) and a known mediator of intracellular mycobacterial survival (Gutierrez et al., 2004; Kumar et al., 2010). The integration approach predicted that compounds from E1, but not E2, modulate autophagy. To test it, HeLa cells were treated with the compounds, immunostained for the autophagosome marker LC3 and multiple parameters reporting diverse facets of autophagosomes were extracted (Table 3).

The compounds of sub-cluster E1, but not E2, had a strong effect on autophagy (Figure 3C,10A), thus validating the prediction from the integration analysis. Of the three compounds validated in intracellular mycobacterial killing, Nortriptyline and PE belong to sub-cluster E1 whereas Haloperidol belongs to E2. Consistent with the data of Figure 3C, Nortriptyline and PE, similar to Rapamycin or serum starvation (by HBSS), showed a strong effect on autophagy, whereas Haloperidol did not (Figure 10B,C). Human primary macrophages treated with Nortriptyline and PE resembled HeLa cells in showing an increase in both number and integral intensity of LC3 vesicles (Figure 10D,E), indicating that their effect of increasing autophagosomes is not cell type-restricted.

To determine whether the accumulation of autophagosomes is related to intracellular mycobacterial killing by Nortriptyline and PE, their IC50 values was first computed in the two assays. For both compounds, the IC50 values for increase in LC3 integral intensity correlated well with the decrease in *M. bovis* BCG-GFP (Figure 3D-F), arguing that the two phenotypes are linked. Second, if autophagy modulation is involved in intracellular mycobacterial killing, mycobacteria should be visualizable within autophagosomes. To test this, Mycobacteria-infected cells were analyzed by electron microscopy. Whereas in control cells mycobacteria resided within single-membrane phagosomes (Figure 4A), cells treated with Nortriptyline and PE clearly showed the presence of mycobacteria within autophagosomes, defined as vesicles containing multiple membrane whorls and delimited by double membrane (Figure 4B-F). Haloperidol treated cells, as expected, did not show this effect (Figure 11A).

Finally, the effect of an inhibitor of autophagy, 3-Methyl Adenine (3MA)(Seglen and Gordon, 1982) was tested. Upon treatment with Nortriptyline and PE in the presence of 3-MA, mycobacteria were not observed in autophagosomes but in mycobacterial phagosomes (Figure 11B-D). Additionally, 3-MA reverted the effect of Nortriptyline on mycobacterial survival in both CFU and image-based assays (not shown), arguing that the effect of the compounds on mycobacteria is related to autophagy.

**Example 6: Nortriptyline and PE modulate autophagy by different mechanisms**

Autophagy is a multi-step process consisting of autophagosome formation, maturation and eventual degradation by fusion with lysosomes (autophagolysosomes). The increase in autophagosomes mediated by Nortriptyline and PE could be either due to a higher rate of formation or a block in maturation and degradation (autophagic flux) (Klionsky et al., 2008). To distinguish between these possibilities, two different approaches were used. First, it was tested whether blockers of lysosomal degradation, e.g. inhibitors of acidification (BafilomycinAor Chloroquine) that inhibit autophagic flux could have an additive effect with the compounds on autophagosome accumulation (Klionsky et al., 2008). HeLa cells were treated with Nortriptyline and PE in the presence or absence of BafilomycinA1, and LC3 lipidation (LC3-II band) was assessed by immunoblotting. Whereas both Nortriptyline and PE showed significant increase in the LC3-II band, additive effects of BafilomycinA1 were seen only upon Nortriptyline, but not PE treatment (Figure 12A). In addition, HeLa cells stably expressing LC3 fused to GFP (HeLa BAC LC3-GFP) under the control of the endogenous promoter were generated through BAC recombineering (Poser et al., 2008). The results (Figure 12B,C) confirmed that Nortriptyline, but not PE, had an additive effect with BafilomycinA1 and Chloroquine in increasing LC3-GFP vesicles.

Second, an established assay was used for measuring autophagic flux based on a tandem mRFP-GFP-LC3 construct (Kimura et al., 2007). Since GFP is quenched in the acidic environment of autophagolysosomes whereas mRFP remains stable, agents blocking autophagy flux display higher colocalization of mRFP and GFP compared to those inducing autophagy. HeLa cells stably expressing mRFP-GFP-LC3 (Settembre et al., 2011) were treated with the compounds and the co-localization between mRFP and GFP was quantified. Nortriptyline, similar to starvation-induced autophagy (HBSS), showed a low mRFP to GFP co-localisation, whereas PE, similar to BafilomycinA1 and Chloroquine, yielded higher co-localisation (Figure 5A,B). Similar results were obtained for the co-localisation of LC3-GFP with LBPA in HeLa-LC3-GFP cells (Figure 12D). Based on previous studies (Klionsky et al., 2008), these results can be interpreted as indicating that Nortriptyline induces autophagy whereas PE inhibits autophagic flux.

In principle, blockers of autophagy flux should not have a detrimental effect on mycobacteria. Indeed, inhibitors of autophagy flux that abrogate the function of lysosomes by blocking acidification did not affect the intensity of intracellular *M. bovis* BCG-GFP signal (Figure 12E). In order to determine whether PE has a similar effect on lysosomal pH, the intensity of the acidotropic dye Lysotracker red was quantified over time. Unlike classical blockers of autophagy flux, PE did not have a negative effect on the acidity of lysosomes, but quite the contrary, it significantly increased the acidity (Figure 5C). Moreover, this effect correlated with the presence of mycobacteria within the lysotracker red-positive vesicles (Figure 5C) and a concomitant reduction in bacterial intensity (Figure 5D).

The increased acidification by PE is in apparent contradiction with the inhibition of autophagic flux. To solve this discrepancy, HeLa cells stably expressing mRFP-GFP- -LC3 were treated with the compounds for different periods of time. If autophagy flux were completely blocked, the co-localisation between mRFP and GFP (corresponding to non-acidic autophagosomes) should increase with time, whereas if the flux were slowed-down then the co-localisation would be expected to decrease (i.e. autophagosomes become acidic). The co-localisation of mRFP and GFP increased over time in cells treated with BafilomycinA1 (Figure 5E) or Chloroquine (not shown). In contrast, in PE-treated cells the colocalization increased within the first hr but then progressively decreased (Figure 5E), showing that the autophagosomes acidify over time.

To further characterize the effects of Nortriptyline and PE on autophagy, it was investigated whether they could synergize with an established physiological inducer of autophagy, Interferon-gamma (IFN-$\gamma$) (Gutierrez et al., 2004; Matsuzawa et al., 2012). Infected cells were pre-activated with IFN-$\gamma$ and assessed the effect of the compound addition on mycobacterial survival. The results were striking (Figure 5F) and showed that Nortriptyline synergized strongly with IFN-$\gamma$, supporting its role in autophagy induction. PE instead exerted a much weaker effect, providing additional evidence that the two compounds interact with the autophagy machinery differently.

**Example 7: Haloperidol accelerates endolysosomal trafficking**

Haloperidol kills intracellular mycobacteria by a mechanism distinct from autophagy. Haloperidol belongs to sub-cluster E2 (Figure 3B). This sub-cluster is characterized by a strong alteration of the endocytic profile that can be summarized as an increase in EGF and Tfn content, increased co-localisation of EGF to Tfn (reduced sorting) and accumulation of EGF in large endosomes in the cell centre. In accordance with the design principles of the endocytic system (Collinet et al., 2010; Rink et al., 2005), the alterations observed argue for a faster degradation. Hence, it was hypothesized that Haloperidol could positively modulate endocytic trafficking along the degradative route. To test this hypothesis, the kinetics of transport of low density lipoprotein (LDL) from early to late endosomes was monitored, using Rab5 (Bucci et al., 1992) and LBPA (Kobayashi et al., 1998) as markers, respectively (Figure 13A). HeLa cells stably expressing GFP-Rab5 from the endogenous promoter (HeLa BAC GFP-Rab5c) (Foret et al., 2012) were treated with Haloperidol and allowed to internalize LDL for different times prior to immunostaining with anti-Apolipoprotein B (ApoB) (to detect LDL) and LBPA antibodies. Haloperidol significantly increased the intensity of LDL in late, but not early endosomes (Figure 6A,B). Similarly, LDL-containing late, but not early endosomes, were larger, more numerous and closer to the cell centre in Haloperidol-treated compared to control cells (Figure 13B-G). These results confirm the prediction that Haloperidol leads to acceleration of trafficking along the degradative pathway.

Phagosome maturation depends on the fusion with early, late endosomes and lysosomes (Desjardins et al., 1994; Fairn and Grinstein, 2012) and is regulated by the motility of endocytic organelles along the cytoskeleton and the recruitment of the membrane tethering-fusion machinery. Both processes could be enhanced by Haloperidol. To address this, the influence of Haloperidol on endosome motility was tested. Increasing concentration of Rab5 and displacement of early endosomes towards cell centre are predictive of progression from early to late endosomes (Del Conte-Zerial et al., 2008; Rink et al., 2005). Endosome motility was therefore quantified in cells stably expressing GFP-Rab5, as previously described (Rink et al., 2005), and the velocity toward the cell centre (minus-end microtubule directed) and cell periphery (plus-end) was computed as a function of GFP-Rab5 intensity. Haloperidol-treated cells showed a much higher motility of early endosomes harboring high levels of Rab5 and this enhancement was higher toward the cell centre than the cell periphery (Figure 6C,D). Next, the mean square displacement of endosomes was computed over time, an established way to characterize motility patterns (Caspi et al., 2002). In Haloperidol-treated cells, long, but not short, time-scale movements of GFP-Rab5 endosomes were significantly accelerated (Figure 6E). These data indicate that Haloperidol accelerates the centripetal movement of early endosomes.

Second, the hypothesis was tested that Haloperidol could lead to increased delivery of components of the membrane tethering and fusion machinery to the arrested mycobacterial phagosome. Mycobacterial phagosomes contain Rab5, but not Rab7 (Alix et al., 2011; Russell, 2001) and lack PI(3)P (Vergne et al., 2005), which mediates the recruitment of various Rab5 effectors, including the membrane tethering factor Early Endosomal Antigen 1 (EEA1) (Christoforidis et al., 1999; Simonsen et al., 1998). Therefore, mycobacterial phagosomes lack EEA1 (Fratti et al., 2001). If Haloperidol could restore such machinery, one would expect to see a small, transient increase in EEA1 on mycobacterial phagosomes. *M. bovis* BCG-infected cells were treated with Haloperidol for different times and the co-localization of mycobacteria with EEA1 and LBPA was quantified. Whereas in control cells, as expected, mycobacterial phagosomes were devoid of EEA1 at all times (Figure 7A, upper panels, 7B), Haloperidol-treated cells displayed a small, but significant, localization of EEA1 to mycobacterial phagosomes 20-30 minutes post-treatment (Figure 7A, lower panels, 7B). Such an effect was not observed in cells treated with Nortriptyline and PE (data not shown), indicating that it was specific for Haloperidol. Moreover, in Haloperidol-treated cells the co-localization of mycobacteria and LBPA increased with time (Figure 7C,D). Taken together, these data indicate that Haloperidol restores the delivery of critical components of the membrane tethering machinery to the mycobacterial phagosomes, thereby delivering mycobacteria to lysosomes.

**Example 8: Density-shift clustering algorithm**

First the dataset was transformed into a Gabriel graph where edges connect neighbour data points (nodes). Then the density estimation for each node was performed using a kernel function that was chosen according to the selected distance measure (as detailed below). The Gabriel graph was converted to directed acyclic weighted graph (WDAG) by assigning direction to edges such that they point towards the nodes with higher density estimate. In the ambiguous case of equal density estimates of two neighbour nodes, one node was randomly chosen and a small arbitrary value (0.0000001) was added to its density estimate. Edge weights were set proportional to the distance between nodes that they connect. The sinks of the directed graph (vertices with no out-coming edges) were marked as modes (local maxima) of density. Those data points became cluster centers, and the remaining data points were assigned to the cluster centres based on the shortest weighted path in the directed neighbourhood graph.

*Kernel Density Estimate*

Kernel density estimate of the multivariate probability density function $\hat{f}(x)$ of a random variable x from an independent and identically-distributed sample $x_1, x_2, .., x_n$ is given by

$$\hat{f}(x) = \sum_{i=0}^{n} K(D(x, x_i), h),$$

where K is a kernel function that depends on the distance D between two points and bandwidth h. Kernel function can be any function that fits the following criteria:

1) K is a function of distance $D(x, x_i)$ and is defined over the whole argument space;
2) K is monotonously non-increasing;
3) K has unity integral on the argument space.

The simplest case is the so-called flat kernel, where K equals constant when $D(x, x_i) < h$ and 0 otherwise. In this case, kernel density estimate simply is equal to the number of data points that fall into the hyper-sphere of radius h around the point x, normalized by the volume of this hyper-sphere. The bandwidth parameter h determines the degree of density smoothing. In each particular case, the choice of kernel function is primarily guided by the measure of distance between data points. In the case of Euclidian distance, a commonly chosen kernel is a multivariate Gaussian function in which the covariance matrix parameterizes the bandwidth.

In case of directional data, meaning that the similarity between two feature vectors is given by cosine of angle (uncentered Pearson correlation), von Mises-Fisher probability function may be used as a kernel.

$$p_{vMF}(\vec{x} \mid \vec{\mu}, k) = \frac{k^{d/2-1}}{(2\pi)^{d/2} I_{d/2-1}(k)} e^{k \cdot \cos\theta}, \text{ where } \cos\theta = \frac{\vec{x} \cdot \vec{\mu}}{\|x\| \cdot \|\mu\|}$$

Von Mises-Fisher probability distribution is an analogue of normal distribution for directional data and defines the probability of a random vector $\overline{x}$ that deviates from the central vector $\overline{\mu}$ by $\cos\theta$. The distribution has one concentration parameter K (kappa) that is analogous to inverse of variance in Gaussian. Hence $K$ defines the concentration (inverse bandwidth) of von Mises-Fisher kernel. The normalization constant is dependent on the modified Bessel function of the first kind $I$ of the order $\frac{d}{2} - 1$ , where d is the number of dimensions.

*Gabriel Neighbourhood graph reconstruction*

Gabriel graph is defined such that two points are connected by an edge if and only if the centre of the edge is closer to these points than to any other point in the set.
An optimized search procedure for Gabriel graph search is as follows:

1. For each data point $x_i$ in dataset *X:*

    1.1. Sort the data points in *X* in the ascending order of distance to $x_i$ using *quicksort* algorithm
    1.2. Initialize the set of Gabriel neighbors $G_{xi} = \emptyset$
    For each $x_j : j \neq i$ in the sorted list *X:*

        1.2.1. Construct the mid-point $m_{ij}$: $D(m_{ij}, x_i) = D(m_{ij}, x_j)$
        1.2.2. Find the nearest neighbor $x_{n_1}$ of mid-point $m_{ij}$ in the set of Gabriel neighbors $G_{x_i}$ discovered until now
        1.2.3. If $D(x_{n_1}, m_{ij}) > D(x_i, m_{ij})$ then find the nearest neighbor $x_{n_2}$ of mid-point $m_{ij}$ in the total dataset *X*
        1.2.4. If $D(x_{n_2}, m_{ij}) > D(x_i, m_{ij})$ then add $x_j$ to $x_j$ to $G_{x_i}$
        1.2.5. Go to next j

Then the Gabriel graph was filtered to remove spurious edges. For this the neighbours $x_i$ and $x_j$, that connected

by edge which distance of central point to the third point of set ($x_K$) is smaller than $\dfrac{1}{2}(1+\alpha) \cdot D(x_i, x_j)$ are filtered out. In these examples $\alpha$ was chosen equal 0.05.

In some cases the density modes of two clusters could occur almost on the edge of clusters and in multidimensional space they may happen to be Gabriel neighbours of each other through 'long-range' connections. These connections were filtered as well by taking intersection with sets of its nearest neighbours of an equal size. This procedure removes 'long-range' links that spans across empty regions of the feature space while keeping most of the links within the clusters.

*Density-shift clustering algorithm*

Density-shift according to the present invention is a clustering method that combines per-data point density estimate with a filtered Gabriel neighbourhood graph to perform robust mode seeking in the multidimensional space. The modes of density are identified as points that have density higher than any of their Gabriel neighbours. Other points are assigned to their respective modes following the discrete walk through a succession of Gabriel neighbours with monotonously increasing density estimate at each step. The algorithm details are as follows:

1. Choose appropriate distance function $D(x_i, x_j)$ and a kernel function $K(D(x_i, x_j), h)$ with a pre-set bandwidth h.
2. Build Gabriel neighbour graph on the data points with given distance $D(x_i, x_j)$;
3. Filter spurious edges from Gabriel graph: Gabriel neighbour nodes, $x_i$ and $x_j$, that are connected by the edge whose distance from the central point of the edge to the third node of the graph $x_k$ is smaller than

$$\frac{1}{2}(1+\alpha) \cdot D(x_i, x_j)$$ ,are filtered out. The parameter $\alpha$ was set to 0.05 for all clustering.

4. Filter 'long-range' edges from Gabriel graph: for every node of Gabriel graph, its set of Gabriel neighbours intersects with set nearest neighbours of an equal size. This procedure removes 'long-range' links that spans across empty regions of the feature space while keeping most of the links within the clusters;
5. For each point (node) $x_i$ in the dataset compute the kernel density estimate $\hat{f}(x_i)$
6. Convert the modified Gabriel graph into weighed directed acyclic graph (WDAG) by directing every edge [$x_i$, $x_j$] such that it points to nodes $x_j$ where $f(x_i) > \hat{f}(x_i)$. Edge weights are given by $K(D(x_i, x_j), h)$. In the ambiguous case of equal density estimates of neighbour nodes one node is randomly chosen and a small arbitrary value (0.0000001) is added to its density estimate.
7. The sink nodes of the WDAG , i.e. points with highest density among the neighbours are marked as a cluster centres.
8. Remaining data points are assigned to the cluster centres based on the shortest weighted path over WDAG.

*Pattern-specific score (PSS)*

Pattern-specific score (PSS) is a hit selection method that is based on a statistical model in which each measured feature vector $\vec{r}$ is a sum of two independent components.

$$\vec{r} = a\vec{\mu} + \vec{n}$$

One component is a specific phenotype component that follows along the central direction $\vec{\mu}$ of its class and has an unknown amplitude a. The other component is a random noise component $\vec{n}$ that is drawn from the zero-centered multivariate normal distribution with the covariance matrix $\Sigma$.

Given the fact that $\vec{r}$ is fixed, for every possible value of $\alpha$ there is a unique corresponding random noise vector $\vec{n}$. The likelihood of $\alpha$ can be expressed through the noise vector probability:

$$p(a)da = p(\vec{n})dn = p(\vec{r} - a\vec{\mu}) \cdot \mu \cdot da \propto N_{o,\Sigma}(\vec{r} - a\vec{\mu})da \propto e^{-\frac{1}{2}(\vec{r}-a\vec{\mu})^T \Sigma^{-1}(\vec{r}-a\vec{\mu})}da$$

where the covariance matrix $\Sigma$ can be estimated either from the repeated negative control measurements, or, if unavailable, from the total set of measurements.

Then the probability that the specific component $\vec{r}$ is above the threshold $\alpha_0$ is

$$p(a > a_0) = \frac{\int\limits_{a}^{+\infty} p(a)da}{\int\limits_{-\infty}^{+\infty} p(a)da}$$

The integration is performed numerically using the standard Newton-Cotes method.

In practice, the grouping of the profiles into classes may be obtained from a clustering that is based on uncentered Pearson correlation, so that the mean (or mode) directions of the clusters can be taken as estimates of central directions of classes. The parameters of noise distribution (covariance matrix $\Sigma$) can be estimated from a sufficient sample of negative control measurements.

To compare the performance of PSS and $\chi^2$ on EEA1 hit selection, the covariance matrix of noise $\Sigma$ from a set of negative control profiles was estimated and the same covariance matrix was used to compute both measures. $\chi^2$ score was based on cumulative probability function of $\chi^2$ distribution, under the assumption that negative control profiles are normally distributed. Thus, the squared Mahalonobis length of a negative control profile follows a $\chi^2$ distribution with degrees of freedom equal to number of dimensions d, according to the following formula:

$$P_{\chi^2}(r) = F_{\chi^2}(r^T \Sigma^{-1} r; d) \ .$$

In all examples $a_0$ in PSS formula was set to 0.

*Clustering*

Datasets were clustered with our own parallelized Java implementations of average-linkage Hierarchical clustering, K-means, mean-shift and Gabriel neighbourhood-based density-shift. Hierarchical clustering was implemented using 1 - cos $\alpha$ as a distance measure, following standard practice (Kittler, R. et al. Genome-scale RNAi profiling of cell division in human tissue culture cells. Nature cell biology 9, 140 1-12 (2007)). For K-means clustering with cosine similarity measure we implemented spherical K-means algorithm, where the data points and the cluster centroids are projected on a unity hyper-sphere by normalizing all vectors to have unity Euclidian length. Mean-shift was implemented following the description of Cheng (Cheng, Y. Mean shift, mode seeking, and clustering. IEEE Transactions on Pattern Analysis and Machine Intelligence 17, 790-799 (1995)), the clustering was stopped when minimal correlation of vectors between iterations was higher than 0.999 and the points that converged within 0.95 of each mode were grouped into clusters.

*Silhouette Index*

Cluster Silhouette index was computed following the algorithm provided in Rousseeuw, P. J. Silhouettes: A graphical aid to the interpretation and validation of cluster analysis. Journal of Computational and Applied Mathematics 20, 53-65 (1987), using 1-cos $\alpha$ as a distance measure. In brief, for every i-th data point in the data set, two values are computed: a(i), which is the average distance other data points in its own cluster and b(i), that is a minimum of average distance any other cluster. Then silhouette of the i-th data point s(i)=(b(i)-a(i))/max(a(i),b(i)) is a value that lies between -1 and 1, higher values indicating better fitting of the data point into its own cluster. The average of Silhouette values over the whole dataset is the index that expresses the overall quality of a given clustering.

*Selection of the clustering free parameters*

For hierarchical clustering and K-means we fixed the free parameter by selecting a single clustering level that corresponded to a maximum of average Silhouette index. In case of Transitivity clustering the parameter was fixed following the protocol described in Wittkop, T. et al. Comprehensive cluster analysis with Transitivity Clustering. Nature protocols 6, 285-95 (2011). The selection is based on the analysis of distribution of inter- and intra-cluster distances between data points. The original publication on affinity propagation states that the free parameter (self-correspondence) can be set either to median or minimum of the data point similarity in the dataset, depending whether the user wishes many or few clusters. Since we clustered data based on cosine similarity, minimal possible similarity in the dataset is -1.0, while the median is close to 0.0.

*Construction of pseudo-dendrogram plots*

Generally, the increase in free parameter setting in the compared algorithms is expected to yield more clusters. Although only Hierarchical clustering guarantees that the clusters of the next level are nested in the clusters of previous level of parameter setting, the clusters of the next level generally can be mapped in one-to-one or many-to-one fashion to the clusters of the previous level based on shared data points. Each cluster of the next level is mapped to a cluster of previous level in members of which it has the highest enrichment (lowest hypergeometric p-value). By recursive mapping of consecutive levels we obtain a tree that is then visualized as a dendrogram, where clusters are represented with boxes of width proportional to the cluster size.

*Comparison to the gold standard classification*

Given the clustering and the gold standard classification, for each class one cluster was selected that contained the highest number of the given class members and amounted to at least 50% of the class. If there was no cluster that contained more than 50% of the class, the class was considered to be undetected. The classification performance was computed as the fraction of data points that were correctly assigned to their class based on the established cluster-class mapping.

*Interaction enrichment*

Clustering of RNAi phenotypes is expected to group together genes that participate in the same functional modules. According to the definition of Hartwell and co-workers (Hartwell, L. H., Hopfield, J. J., Leibler, S. & Murray, A. W. From molecular to modular cell biology. Nature 402, C47-52 (1999)), a functional module is characterized by extensive physical interaction of the components. We designed a simple way to cross-validate the gene phenotypic clustering based on this definition by quantifying how much the gene clusters are enriched in protein-protein interactions. The human protein-protein interaction data was obtained from STRING 9.0 database (see the world wide web at string-db.org), where we extracted the interactions annotated as 'experimental' and 'databases' and having confidence index of at least 0.7. As an enrichment measure we took minus logarithm of $p_{value}$ of the standard hypergeometric test that quantifies how likely is it to observe by chance a given or a higher number of interactions between selected protein pairs (sample), given the total number of interactions between all possible pairs of proteins in the dataset (population). The sample included protein pairs where both phenotypic profiles were grouped in the same cluster and appeared within the top 10th percentile of profiles in this clustering, ranked by a given scoring measure.

Gene *annotation enrichment analysis*

The significance of enrichment of a cluster in a labelled subset of the total dataset was assessed using the standard hypergeometric test. Gene Ontology annotation was downloaded from the official website (see the world wide web at geneontology.org). The tests were performed without accounting for term interdependency or correcting for multiple hypothesis testing.

**References**

Alix, E., Mukherjee, S., and Roy, C.R. (2011). Subversion of membrane transport pathways by vacuolar pathogens. J Cell Biol 195, 943-952.

Armstrong, J.A., and Hart, P.D. (1971). Response of cultured macrophages to Mycobacterium tuberculosis, with observations on fusion of lysosomes with phagosomes. J Exp Med 134, 713-740.

Bucci, C., Parton, R.G., Mather, I.H., Stunnenberg, H., Simons, K., Hoflack, B., and Zerial, M. (1992). The small GTPase rab5 functions as a regulatory factor in the early endocytic pathway. Cell 70, 715-728.

Behar, S.M., Divangahi, M., and Remold, H.G. (2010). Evasion of innate immunity by Mycobacterium tuberculosis: is death an exit strategy? Nat Rev Microbiol 8, 668-674.

Caspi, A., Granek, R., and Elbaum, M. (2002). Diffusion and directed motion in cellular transport. Phys Rev E Stat Nonlin Soft Matter Phys 66, 011916.

Chen, M., Gan, H., and Remold, H.G. (2006). A mechanism of virulence: virulent Mycobacterium tuberculosis strain H37Rv, but not attenuated H37Ra, causes significant mitochondrial inner membrane disruption in macrophages leading to necrosis. J Immunol 176, 3707-3716.

Christoforidis, S., McBride, H.M., Burgoyne, R.D., and Zerial, M. (1999). The Rab5 effector EEA1 is a core component of endosome docking. Nature 397, 621-625.

Collinet, C., Stoter, M., Bradshaw, C.R., Samusik, N., Rink, J.C., Kenski, D., Habermann, B., Buchholz, F., Henschel, R., Mueller, M.S., et al. (2010). Systems survey of endocytosis by multiparametric image analysis. Nature 464, 243-249.

Del Conte-Zerial, P., Brusch, L., Rink, J.C., Collinet, C., Kalaidzidis, Y., Zerial, M., and Deutsch, A. (2008). Membrane identity and GTPase cascades regulated by toggle and cut-out switches. Mol Syst Biol 4, 206.

Desjardins, M., Huber, L.A., Parton, R.G., and Griffiths, G. (1994). Biogenesis of phagolysosomes proceeds through a sequential series of interactions with the endocytic apparatus. J Cell Biol 124, 677-688.

Dhandayuthapani, S., Via, L.E., Thomas, C.A., Horowitz, P.M., Deretic, D., and Deretic, V. (1995). Green fluorescent protein as a marker for gene expression and cell biology of mycobacterial interactions with macrophages. Mol Microbiol 17, 901-912.

Dyer, M.D., Murali, T.M., and Sobral, B.W. (2008). The landscape of human proteins interacting with viruses and other pathogens. PLoS Pathog 4, e32.

Eggert, U.S., Kiger, A.A., Richter, C., Perlman, Z.E., Perrimon, N., Mitchison, T.J., and Field, C.M. (2004). Parallel chemical genetic and genome-wide RNAi screens identify cytokinesis inhibitors and targets. PLoS Biol 2, e379.

Fairn, G.D., and Grinstein, S. (2012). How nascent phagosomes mature to become phagolysosomes. Trends Immunol.

Foret, L., Dawson, J.E., Villasenor, R., Collinet, C., Deutsch, A., Brusch, L., Zerial, M., Kalaidzidis, Y., and Julicher, F. (2012). A general theoretical framework to infer endosomal network dynamics from quantitative image analysis. Curr Biol 22, 1381-1390.

Fratti, R.A., Backer, J.M., Gruenberg, J., Corvera, S., and Deretic, V. (2001). Role of phosphatidylinositol 3-kinase and Rab5 effectors in phagosomal biogenesis and mycobacterial phagosome maturation arrest. J Cell Biol 154, 631-644.

Galluzzi, L., Aaronson, S.A., Abrams, J., Alnemri, E.S., Andrews, D.W., Baehrecke, E.H., Bazan, N.G., Blagosklonny, M.V., Blomgren, K., Borner, C., et al. (2009). Guidelines for the use and interpretation of assays for monitoring cell death in higher eukaryotes. Cell Death Differ 16, 1093-1107.

Gutierrez, M.G., Master, S.S., Singh, S.B., Taylor, G.A., Colombo, M.I., and Deretic, V. (2004). Autophagy is a defense mechanism inhibiting BCG and Mycobacterium tuberculosis survival in infected macrophages. Cell 119, 753-766.

Huang, R., Southall, N., Wang, Y., Yasgar, A., Shinn, P., Jadhav, A., Nguyen, D.T., and Austin, C.P. (2011). The NCGC pharmaceutical collection: a comprehensive resource of clinically approved drugs enabling repurposing and chemical genomics. Sci Transl Med 3, 80ps16.

Imming, P., Sinning, C., and Meyer, A. (2006). Drugs, their targets and the nature and number of drug targets. Nat Rev Drug Discov 5, 821-834.

Jayachandran, R., Sundaramurthy, V., Combaluzier, B., Mueller, P., Korf, H., Huygen, K., Miyazaki, T., Albrecht, I., Massner, J., and Pieters, J. (2007). Survival of mycobacteria in macrophages is mediated by coronin 1-dependent activation of calcineurin. Cell 130, 37-50.

Jayaswal, S., Kamal, M.A., Dua, R., Gupta, S., Majumdar, T., Das, G., Kumar, D., and Rao, K.V. (2010). Identification of host-dependent survival factors for intracellular Mycobacterium tuberculosis through an siRNA screen. PLoS Pathog 6, e1000839.

Karnovsky, M. (1971). Use of ferrocyanide-reduced osmium tetroxide in electron microscopy.. In Proceedings of the Eleventh Annual Meeting of the American Society for Cell Biology.

Keane, J., Remold, H.G., and Kornfeld, H. (2000). Virulent Mycobacterium tuberculosis strains evade apoptosis of infected alveolar macrophages. J Immunol 164, 2016-2020.

Kimura, S., Noda, T., and Yoshimori, T. (2007). Dissection of the autophagosome maturation process by a novel reporter protein, tandem fluorescent-tagged LC3. Autophagy 3, 452-460.

Klionsky, D.J., Abeliovich, H., Agostinis, P., Agrawal, D.K., Aliev, G., Askew, D.S., Baba, M., Baehrecke, E.H., Bahr, B.A., Ballabio, A., et al. (2008). Guidelines for the use and interpretation of assays for monitoring autophagy in higher eukaryotes. Autophagy 4, 151-175.

Kobayashi, T., Stang, E., Fang, K.S., de Moerloose, P., Parton, R.G., and Gruenberg, J. (1998). A lipid associated with the antiphospholipid syndrome regulates endosome structure and function. Nature 392, 193-197.

Koul, A., Herget, T., Klebl, B., and Ullrich, A. (2004). Interplay between mycobacteria and host signalling pathways. Nat Rev Microbiol 2, 189-202.

Kuijl, C., Savage, N.D., Marsman, M., Tuin, A.W., Janssen, L., Egan, D.A., Ketema, M., van den Nieuwendijk, R.,

van den Eeden, S.J., Geluk, A., et al. (2007). Intracellular bacterial growth is controlled by a kinase network around PKB/AKT1. Nature 450, 725-730.

Kumar, D., Nath, L., Kamal, M.A., Varshney, A., Jain, A., Singh, S., and Rao, K.V. (2010). Genome-wide analysis of the host intracellular network that regulates survival of Mycobacterium tuberculosis. Cell 140, 731-743.

Laplante, M., and Sabatini, D.M. (2012). mTOR signaling in growth control and disease. Cell 149, 274-293.

Matsuzawa, T., Kim, B.H., Shenoy, A.R., Kamitani, S., Miyake, M., and Macmicking, J.D. (2012). IFN-gamma elicits macrophage autophagy via the p38 MAPK signaling pathway. J Immunol 189, 813-818.

Parvati K., Prakasa Rao B.S., and Mariya Das M.. (2008). Image Segmentation Using Gray-Scale Morphology and Marker-Controlled Watershed Transformation. Discrete Dynamics in Nature and Society 2008.

Pieters, J. (2008). Mycobacterium tuberculosis and the macrophage: maintaining a balance. Cell Host Microbe 3, 399-407.

Ponpuak, M., Davis, A.S., Roberts, E.A., Delgado, M.A., Dinkins, C., Zhao, Z., Virgin, H.W.t., Kyei, G.B., Johansen, T., Vergne, I., et al. (2010). Delivery of cytosolic components by autophagic adaptor protein p62 endows autophagosomes with unique antimicrobial properties. Immunity 32, 329-341.

Poser, I., Sarov, M., Hutchins, J.R., Heriche, J.K., Toyoda, Y., Pozniakovsky, A., Weigl, D., Nitzsche, A., Hegemann, B., Bird, A.W., et al. (2008). BAC TransgeneOmics: a high-throughput method for exploration of protein function in mammals. Nat Methods 5, 409-415.

Regenthal, R., Krueger, M., Koeppel, C., and Preiss, R. (1999). Drug levels: therapeutic and toxic serum/plasma concentrations of common drugs. J Clin Monit Comput 15, 529-544.

Rink, J., Ghigo, E., Kalaidzidis, Y., and Zerial, M. (2005). Rab conversion as a mechanism of progression from early to late endosomes. Cell 122, 735-749.

Roerdink, A.M.a.J.B.T.M. (2000). The watershed transform: definitions, algorithms, and parallelization strategies. Fundamenta Informaticae 41, 187-228.

Rossi, M., Munarriz, E.R., Bartesaghi, S., Milanese, M., Dinsdale, D., Guerra-Martin, M.A., Bampton, E.T., Glynn, P., Bonanno, G., Knight, R.A., et al. (2009). Desmethylclomipramine induces the accumulation of autophagy markers by blocking autophagic flux. J Cell Sci 122, 3330-3339.

Russell, D.G. (2001). Mycobacterium tuberculosis: here today, and here tomorrow. Nat Rev Mol Cell Biol 2, 569-577.

Schmid, S.L., and Cullis, P.R. (1998). Membrane sorting. Endosome marker is fat not fiction. Nature 392, 135-136.

Schwegmann, A., and Brombacher, F. (2008). Host-directed drug targeting of factors hijacked by pathogens. Sci Signal 1, re8.

Seglen, P.O., and Gordon, P.B. (1982). 3-Methyladenine: specific inhibitor of autophagic/lysosomal protein degradation in isolated rat hepatocytes. Proc Natl Acad Sci U S A 79, 1889-1892.

Settembre, C., Di Malta, C., Polito, V.A., Garcia Arencibia, M., Vetrini, F., Erdin, S., Erdin, S.U., Huynh, T., Medina, D., Colella, P., et al. (2011). TFEB links autophagy to lysosomal biogenesis. Science 332, 1429-1433.

Shubin, H., Sherson, J., Pennes, E., Glaskin, A., and Sokmensuer, A. (1958). Prochlorperazine (compazine) as an aid in the treatment of pulmonary tuberculosis. Antibiotic Med Clin Ther 5, 305-309.

Simonsen, A., Lippe, R., Christoforidis, S., Gaullier, J.M., Brech, A., Callaghan, J., Toh, B.H., Murphy, C., Zerial, M., and Stenmark, H. (1998). EEA1 links PI(3)K function to Rab5 regulation of endosome fusion. Nature 394, 494-498.

Swinney, D.C., and Anthony, J. (2011). How were new medicines discovered? Nat Rev Drug Discov 10, 507-519.

Vergne, I., Chua, J., Lee, H.H., Lucas, M., Belisle, J., and Deretic, V. (2005). Mechanism of phagolysosome biogenesis block by viable Mycobacterium tuberculosis. Proc Natl Acad Sci U S A 102, 4033-4038.

Zschocke, J., Zimmermann, N., Berning, B., Ganal, V., Holsboer, F., and Rein, T. (2011). Antidepressant drugs diversely affect autophagy pathways in astrocytes and neurons--dissociation from cholesterol homeostasis. Neuropsychopharmacology 36, 1754-1768.

Tables:

**Table 1:** Description and grouping of parameters used in mycobacteria high content screen (see also Figure 1)

| Parameter groups | | Number | Descriptor |
|---|---|---|---|
| Bacteria / infection parameters | Number related | 1 | Number of bacteria within infected cell Mean |
| | | 2 | Number of bacteria within infected cell Median |
| | | 3 | Number of bacteria within infected cell Mode |
| | | 4 | Total number bacteria per image |
| | | 5 | Number of bacteria per cell Mean |
| | | 6 | Number of bacteria per cell Median |
| | | 7 | Number of bacteria per cell Mode |
| | | 8 | Total number of bacteria in infected cells |
| | | 9 | Number Infected Cells |
| | Intensity related | 10 | Integral Vesicle Intensity of bacteria within infected cell |
| | | 11 | Mean Intensity (M. bovis BCG-GFP), Non-weighted Median |
| | | 12 | Mean Intensity (M. bovis BCG-GFP), Weighted (intensity) |
| | | 13 | Mean Intensity (M. bovis BCG-GFP), Weighted (integral intensity) |
| | | 14 | Mean Integral Intensity (M. bovis BCG-GFP), Non-weighted Median |
| | | 15 | Mean Integral Intensity (M. bovis BCG-GFP), Weighted (intensity) |
| | | 16 | Mean Integral Intensity (M. bovis BCG-GFP), Weighted (integral intensity) |
| | Shape related | 17 | Mean Area (M. bovis BCG-GFP), Non-weighted Median |
| | | 18 | Mean Area (M. bovis BCG-GFP), Weighted (intensity) |
| | | 19 | Mean Area (M. bovis BCG-GFP), Weighted (integral intensity) |
| Host cell parameters | Morphology | 20 | Area |
| | | 21 | Compactness |
| | | 22 | Dispersion |
| | | 23 | Eccentricity |
| | | 24 | Solidity |
| | | 25 | Extent |
| | Toxicity indicators | 26 | Nucleus Size Median |
| | | 27 | Nucleus Size Median (weighted) |
| | | 28 | Nucleus Size Mean |
| | | 29 | Number of NucleiIntensity (M. bovis BCG-GFP), Non-weighted Median |
| | | 30 | Number of Cells |

**Table 2:** Description of the morphological parameters describing bacterial shape (see also Figure 9A)

| Parameter | Description |
| --- | --- |
| a. Median: Area | Median area of bacteria estimated at half height of the fitting functions. |
| b. Mean: Area: Weighted (Mean Intensity) | Same as parameter a., but the mean area weighted by bacterial mean intensity is calculated. |
| c. Mean: Area: Weighted (Integral Intensity) | Same as parameter a., but the mean area weighted by bacterial integral intensity is calculated. |
| d. Median: Elongation | Median elongation of bacteria estimated at half height of the fitting functions. |
| e. Mean: Elongation: Weighted (Mean Intensity) | Same as parameter d., but the mean area weighted by bacterial mean intensity is calculated. |
| f. Mean: Elongation: Weighted (Integral Intensity) | Same as parameter d., but the mean area weighted by bacterial integral intensity is calculated. |
| g. Mean: Bacteria area | Mean object area measured on the contour of the segmented bacterial. |
| h. Mean: Bacteria perimeter | Mean object perimeter measured on the contour of the segmented bacteria. |
| i. Mean: Bacteria compactness | Mean object compactness measured on the contour of the segmented bacteria. Compactness is a measure of region non-smoothness, defined as the ratio between the actual area and the area a circle with the same perimeter would have. |
| j. Mean: bacteria dispersion | Mean object dispersion measured on the contour of the segmented bacteria. Dispersion is a measure of region irregularity, defined as the ratio of the major chord length (maximum circle enclosing the region) to the area. |
| k. Mean: Bacteria relative dispersion | Mean object relative dispersion measured on the contour of the segmented bacteria. Relative dispersion is a measure of region spreading, defined as the ratio of the maximal region radius and the minimal one. |
| l. Mean: Bacteria elongation | Mean object elongation measured on the contour of the segmented bacteria. Elongation is another measure of region Irregularity, defined as the ratio of the actual perimeter and the perimeter a circle with the same area would have. |
| m. Mean: Bacteria eccentricity | Mean object eccentricity measured on the contour of the segmented bacteria. Eccentricity is a measure of the elongation of the best fitting ellipse to the region, defined as $e = \sqrt{1 - \left(\frac{b}{a}\right)^2}$, where a is the semi-major axis and b the semi-minor axis of the best fitting ellipse (estimated through the region eigenvalues). |
| n. Mean: Bacteria solidity | Mean object solidity measured on the contour of the segmented bacteria. Solidity is a measure of region tightness, defined as the ratio between the region area and the area of the convex hull of the same region. |
| o. Mean: Bacteria extent | Mean object extent measured on the contour of the segmented bacteria. Extent is a measure of region extension defined as the ratio between the area of the minimal rectangle (bounding box) that encloses and the actual region area. |

**Autophagy high content assay**

| Parameter groups | | Parameter description |
|---|---|---|
| LC3 vesicle parameters | Number | Number of LC3 vesicles |
| | Intensity | Total Integral Intensity of LC3 vesicles |
| | | Mean Integral Intensity of LC3 vesicles |
| | | Mean Integral Intensity of LC3 vesicles, weighted by volume |
| | | Mean Intensity of LC3 vesicle |
| | | Mean Intensity of LC3 vesicle, Weighted By Volume |
| | Area | Mean Area of LC3 vesicles |
| | | Mean Area of LC3 vesicles, Weighted By Vol |
| | Position | Distance to Nucleus from LC3 vesicles |
| | | Distance to Nucleus from LC3 vesicles, Weighted by Volume |
| Cellular morphology parameters | | Cell Number |
| | | Cell Area |
| | | Cell Perimeter |
| | | Cell Compactness |
| | | Cell Dispersion |
| | | Cell Elongation |

**Endocytosis high content assay**

| Parameter groups | | Parameter description |
|---|---|---|
| EGF / Tfn parameters | Number | Number of EGF / Tfn |
| | Intensity | Total intensity (EGF/Tfn) |
| | | Integral Intensity of EGF / Tfn vesicles |
| | | Mean Integral Intensity of EGF / Tfn vesicles, weighted by integral intensity |
| | | Mean Integral Intensity of EGF / Tfn vesicles, weighted by intensity |
| | | Mean Integral Intensity of EGF / Tfn vesicles |
| | | Mean Intensity of EGF / Tfn vesicles, weighted by integral intensity |
| | | Mean Intensity of EGF / Tfn vesicles, weighted by Intensity |
| | | Mean Intensity of EGF / Tfn vesicles |
| | Area | Mean Area of EGF / Tfn vesicles, weighted by integral intensity |
| | | Mean Area of EGF / Tfn vesicles, weighted by intensity |
| | | Mean Area of EGF / Tfn vesicles |
| | Position | Distance to Nucleus of EGF / Tfn vesicles, weighted by intensity |
| | | Distance to Nucleus of EGF / Tfn vesicles, weighted by integral intensity |
| | | Distance to Nucleus of EGF / Tfn vesicles |
| | Co-localisation | Co-localisation of EGF to Tfn |
| | | Co-localisation Co-localisation of Tfn to EGF |

**Table 3:** Autophagy and endocytosis parameters (see also Figure 3)

**Table 4:** Compounds from cluster M5 and their pattern-specific score (PSS)

| Compounds | PSS |
|---|---|
| 12A-HYDROXY-9-DEMETHYLMUNDUSERONE-8-CARBOXYLIC ACID | 0.838688 |
| 3;7-EPOXYCARYOPHYLLAN-6-OL | 0.719354 |
| ACEPHATE | 0.657407 |
| ALBIZZIINE | 0.538698 |
| ALVERINE CITRATE | 1 |
| AMITRIPTYLINE HYDROCHLORIDE | 1 |
| AMOXAPINE | 0.99999 |
| ANTHRALIN | 0.701373 |
| APIGENIN TRIACETATE | 0.413001 |
| ARACHIDONIC ACID | 0.936708 |
| ARCAINE SULFATE | 0.768613 |
| ARTEMISININ | 0.495906 |
| ARTHONIOIC ACID | 0.81887 |
| ATENOLOL | 0.683277 |
| ATRACTYLOSIDE POTASSIUM | 0.667684 |
| AZELASTINE HYDROCHLORIDE | 0.980104 |
| BAICALEIN | 0.928898 |
| BENZAMIL HYDROCHLORIDE | 0.963588 |
| BENZTROPINE | 0.999999 |
| BEPRIDIL HYDROCHLORIDE | 1 |
| BERGENIN | 0.807155 |
| BETULIN | 0.924371 |
| BUTAMBEN | 0.580649 |
| CARPROFEN | 0.825904 |
| CARYLOPHYLLENE OXIDE | 0.578179 |
| CEFADROXIL | 0.671358 |
| CEFDITORIN PIVOXIL | 0.862599 |
| CHAULMOOGRIC ACID; ETHYL ESTER | 0.724781 |
| CHLOROCRESOL | 0.887429 |
| CHLORPROMAZINE | 0.999694 |
| CHOLEST-5-EN-3-ONE | 0.701001 |
| CITICOLINE | 0.50781 |
| CLEMASTINE | 1 |
| CLOFOCTOL | 0.941259 |
| CLOMIPHENE CITRATE | 0.999764 |
| CLOMIPRAMINE HYDROCHLORIDE | 0.999993 |
| CLOPERASTINE HYDROCHLORIDE | 0.999994 |

(continued)

| Compounds | PSS |
|---|---|
| CYCLOBENZAPRINE HYDROCHLORIDE | 0.999999 |
| D;L-THREO-3-HYDROXYASPARTIC ACID | 0.800381 |
| DEOXYGUANOSINE | 0.589927 |
| DESIPRAMINE HYDROCHLORIDE | 1 |
| DEXTROMETHORPHAN HYDROBROMIDE | 0.999996 |
| DICYCLOMINE HYDROCHLORIDE | 0.99985 |
| DIENESTROL | 0.592275 |
| DIPHENYLPYRALINE HYDROCHLORIDE | 0.992221 |
| DOXEPIN HYDROCHLORIDE | 0.984494 |
| DROFENINE HYDROCHLORIDE | 1 |
| ENOXOLONE | 0.42336 |
| ETHOPROPAZINE HYDROCHLORIDE | 1 |
| ETHOXYQUIN | 0.793273 |
| FENDILINE HYDROCHLORIDE | 1 |
| FLUOROURACIL | 0.775835 |
| FLUOXETINE | 0.999614 |
| FLUPHENAZINE HYDROCHLORIDE | 1 |
| FORMONONETIN | 0.522543 |
| GLUCITOL-4-GUCOPYANOSIDE | 0.883553 |
| H89 | 0.963554 |
| HALOPERIDOL | 0.996028 |
| HEDERAGENIN | 0.62298 |
| HEXETIDINE | 0.961727 |
| HIERACIN | 0.316411 |
| HYGROMYCIN B | 1 |
| IMIPRAMINE HYDROCHLORIDE | 0.999999 |
| IRIGENIN TRIMETHYL ETHER | 0.790056 |
| KETOCONAZOLE | 0.831512 |
| KETOTIFEN FUMARATE | 0.991791 |
| LOPERAMIDE HYDROCHLORIDE | 0.999945 |
| MAPROTILINE HYDROCHLORIDE | 0.999816 |
| MEBHYDROLIN NAPHTHALENESULFONATE | 0.999885 |
| MECLIZINE HYDROCHLORIDE | 0.916186 |
| MENTHYLBENZOATE | 0.491647 |
| METERGOLINE | 0.98639 |
| METHIOTHEPIN MALEATE | 0.994122 |
| N;N-DIMETHYLAMILORIDE | 0.597645 |
| NALBUPHINE HYDROCHLORIDE | 0.832264 |

(continued)

| Compounds | PSS |
|---|---|
| N-METHYL (-)EPHEDRINE | 0.893618 |
| NORSTICTIC ACID | 0.531188 |
| NORTRIPTYLINE | 0.999856 |
| OLMESARTAN MEDOXOMIL | 0.564798 |
| ORPHENADRINE CITRATE | 0.873333 |
| PEMPIDINE TARTRATE | 0.847039 |
| PENTOBARBITAL | 0.57003 |
| PERPHENAZINE | 0.999998 |
| PHYSOSTIGMINE SULFATE | 0.750539 |
| PIMETHIXENE MALEATE | 1 |
| PROCHLORPERAZINE EDISYLATE | 0.999981 |
| PROMAZINE HYDROCHLORIDE | 0.999996 |
| PROMETHAZINE HYDROCHLORIDE | 0.999995 |
| PROMETRYN | 0.879264 |
| PROPAFENONE HYDROCHLORIDE | 0.999961 |
| PYRVINIUM PAMOATE | 0.855694 |
| QUINALIZARIN | 0.815755 |
| ROFECOXIB | 0.719237 |
| RUTILANTINONE | 0.97813 |
| SERTRALINE HYDROCHLORIDE | 1 |
| SMILAGENIN | 0.719768 |
| SPAGLUMIC ACID | 0.686382 |
| SULOCTIDIL | 0.999917 |
| SUPROFEN METHYL ESTER | 0.628898 |
| TAMOXIFEN CITRATE | 0.999925 |
| TELITHROMYCIN | 0.795677 |
| THIAMPHENICOL | 0.810423 |
| THIOTEPA | 0.887964 |
| THIOTHIXENE | 0.998498 |
| TOTAROL ACETATE | 0.790397 |
| TRIFLUOPERAZINE HYDROCHLORIDE | 0.999991 |
| TRIFLUPROMAZINE HYDROCHLORIDE | 0.990837 |
| TRIHEXYPHENIDYL HYDROCHLORIDE | 0.999994 |
| TRIMEPRAZINE TARTRATE | 1 |
| TRIMIPRAMINE MALEATE | 1 |
| TUBOCURARINE CHLORIDE | 0.794363 |
| URSOCHOLANIC ACID | 0.819147 |
| VERATRIDINE | 0.541782 |

(continued)

| Compounds | PSS |
|---|---|
| XANTHOPTERIN | 0.322306 |
| XYLAZINE | 0.901593 |

**Table 5:** List of compounds from the endocytosis clusters E1 and E2

| Compounds from endocytosis cluster E1 | Compounds from endocytosis cluster E2 |
|---|---|
| ALBIZZIINE | 3;7-EPOXYCARYOPHYLLAN-6-OL |
| ALVERINE CITRATE | ACEPHATE |
| AMITRIPTYLINE HYDROCHLORIDE | APIGENIN TRIACETATE |
| AMOXAPINE | ARTHONIOIC ACID |
| ANTHRALIN | ATENOLOL |
| ARACHIDONIC ACID | ATRACTYLOSIDE POTASSIUM |
| ARCAINE SULFATE | BENZAMIL HYDROCHLORIDE |
| ARTEMISININ | BERGENIN |
| BAICALEIN | CARPROFEN |
| BETULIN | CEFDITORIN PIVOXIL |
| BUTAMBEN | CHAULMOOGRIC ACID; ETHYL ESTER |
| CEFADROXIL | CHLOROCRESOL |
| CHLORPROMAZINE | CLOPERASTINE HYDROCHLORIDE |
| CHOLEST-5-EN-3-ONE | DEOXYGUANOSINE |
| CITICOLINE | DIENESTROL |
| CLOFOCTOL | FLUOROURACIL |
| CLOMIPHENE CITRATE | FLUOXETINE |
| CLOMIPRAMINE HYDROCHLORIDE | FLUPHENAZINE HYDROCHLORIDE |
| CYCLOBENZAPRINE HYDROCHLORIDE | GLUCITOL-4-GUCOPYANOSIDE |
| DESIPRAMINE HYDROCHLORIDE | HALOPERIDOL |
| DEXTROMETHORPHAN HYDROBROMIDE | HEDERAGENIN |
| DIPHENYLPYRALINE HYDROCHLORIDE | IRIGENIN TRIMETHYL ETHER |
| DOXEPIN HYDROCHLORIDE | KETOTIFEN FUMARATE |
| ETHOPROPAZINE HYDROCHLORIDE | METHIOTHEPIN MALEATE |
| ETHOXYQUIN | N;N-DIMETHYLAMILORIDE |
| FORMONONETIN | NALBUPHINE HYDROCHLORIDE |
| HEXETIDINE | NORSTICTIC ACID |
| HYGROMYCIN B | OLMESARTAN MEDOXOMIL |
| IMIPRAMINE HYDROCHLORIDE | PEMPIDINE TARTRATE |
| KETOCONAZOLE | PENTOBARBITAL |
| LOPERAMIDE HYDROCHLORIDE | PHYSOSTIGMINE SULFATE |
| MAPROTILINE HYDROCHLORIDE | PIMETHIXENE MALEATE |

(continued)

| Compounds from endocytosis cluster E1 | Compounds from endocytosis cluster E2 |
| --- | --- |
| MEBHYDROLIN NAPHTHALENESULFONATE | PROMAZINE HYDROCHLORIDE |
| MECLIZINE HYDROCHLORIDE | PROMETHAZINE HYDROCHLORIDE |
| MENTHYLBENZOATE | PROMETRYN |
| METERGOLINE | PYRVINIUM PAMOATE |
| NORIHIPTYLINE | QUINALIZARIN |
| PERPHENAZINE | RUTILANTINONE |
| PROCHLORPERAZINE EDISYLATE | SPAGLUMIC ACID |
| PROPAFENONE HYDROCHLORIDE | SUPROFEN METHYL ESTER |
| ROFECOXIB | THIAMPHENICOL |
| SERTRALINE HYDROCHLORIDE | THIOTEPA |
| SMILAGENIN | THIOTHIXENE |
| TAMOXIFEN CITRATE | TOTAROL ACETATE |
| TELITHROMYCIN | TRIFLUOPERAZINE HYDROCHLORIDE |
| TRIMEPRAZINE TARTRATE | TRIFLUPROMAZINE HYDROCHLORIDE |
| TRIMIPRAMINE MALEATE | TRIHEXYPHENIDYL HYDROCHLORIDE |
| XANTHOPTERIN | TUBOCURARINE CHLORIDE |
| | URSOCHOLANIC ACID |
| | VERATRIDINE |
| | XYLAZINE |

**Claims**

1.  A compound capable of killing mycobacteria by activating host cellular mechanisms for use in treating tuberculosis, wherein the compound is selected from the group consisting of nortriptyline, haloperidol and prochlorperazine edisylate (PE).

2.  A pharmaceutical composition comprising as the single active agent the compound according to claim 1, and optionally a pharmaceutically acceptable carrier.

3.  The compound according to claim 1 or the pharmaceutical composition according to claim 2, having admixed thereto or associated in a separate container one or more agents selected from the group consisting of antibiotic compounds, arginine, vitamin D and IFN-γ.

4.  A method of treating tuberculosis comprising administering a pharmaceutically effective amount of a compound capable of killing mycobacteria by activating host cellular mechanisms to a subject in need thereof, wherein the compound is selected from the group consisting of nortriptyline, haloperidol and prochlorperazine edisylate (PE).

5.  The method according to claim 4, further comprising the administration of one or more agents selected from the group consisting of antibiotic compounds, arginine, vitamin D and IFN-γ.

6.  A method of identifying a lead compound for the treatment of tuberculosis, the method comprising the steps of:

    (a) incubating cells infected with mycobacteria with a test compound;
    (b) staining the cells with (a) detectable marker probe(s) or introducing into the cell a detectable marker probe;
    (c) obtaining a multi-parametric phenotypic profile of host cell parameters and bacterial parameters; and

(d) clustering the multi-parametric phenotypic profile by using a modified Gabriel Neighbourhood and density estimate algorithm, which comprises:

(i) computing multi-dimensional Gabriel neighbourhood graph over dataset, where nodes correspond to phenotypic profiles and edges determine the neighbourhood of nodes;
(ii) filtering spurious as well as 'long-range' edges from the Gabriel graph;
(iii) computing the density estimate for each node of the Gabriel graph using a kernel function, wherein the kernel function is chosen according to the selected multi-parametric profile dissimilarity measure;
(iv) converting the modified Gabriel graph into weighted directed acyclic graph (WDAG) by assigning directionality to edges such that their point from nodes with lower density estimate to nodes with higher density estimate and edge weights are set proportional to the distances (dissimilarity) between neighbouring nodes, or, in the ambiguous case of equal density estimate of two neighbour nodes, one node is randomly chosen and a small arbitrary value (0.0000001) is added to its density estimate;
(v) marking the nodes of the graph with no out-coming edges, ie. nodes having local maxima of density, as cluster centres; and
(vi) assigning the remaining nodes to the cluster centers based on the shortest weighted path in the directed neighbourhood graph;

wherein an alteration in bacterial parameters and a lack or substantial lack of alteration of host cell parameters in the presence of the test compound is indicative of a compound suitable as a lead compound for the treatment of tuberculosis.

7. The method according to claim 6, wherein the cells are infected with mycobacteria that express a fluorescent protein marker.

8. The method according to claim 6 or 7, wherein the bacterial parameters are selected from the group consisting of parameters related to the number of bacteria, parameters related to signal intensity of the bacteria and parameters related to the shape of the bacteria.

9. The method according to any one of claims 6 to 8, wherein the host cell parameters are selected from the group consisting of parameters related to the morphology of the cell and parameters indicative of toxicity.

10. The method according to any one of claims 6 to 9, wherein the bacterial parameters comprise at least

(i) number of bacteria per cell (mean);
(ii) total number of bacteria (per image);
(iii) integral vesicle intensity of bacteria within infected cell;
(iv) mean intensity of bacteria (non-weighted median);
(v) mean area of bacteria (non-weighted median); and
(vi) number of infected cells;

and wherein the host cell parameters comprise at least

(a) number of cells.

11. The method of any one of claims 6 to 10, further comprising optimizing the pharmacological properties of the test compound identified as lead compound.

12. The method of claim 11, wherein the optimization comprises modifying the test compound identified as lead compound to achieve:

i) modified spectrum of activity, organ specificity, and/or
ii) improved potency, and/or
iii) decreased toxicity (improved therapeutic index), and/or
iv) decreased side effects, and/or
v) modified onset of therapeutic action, duration of effect, and/or
vi) modified pharmacokinetic parameters (resorption, distribution, metabolism and excretion), and/or
vii) modified physico-chemical parameters (solubility, hygroscopicity, colour, taste, odour, stability, state), and/or

viii) improved general specificity, organ/tissue specificity, and/or
ix) optimised application form and route
by

(a) esterification of carboxyl groups, or
(b) esterification of hydroxyl groups with carboxylic acids, or
(c) esterification of hydroxyl groups to, e.g. phosphates, pyrophosphates or sulfates or hemi-succinates, or
(d) formation of pharmaceutically acceptable salts, or
(e) formation of pharmaceutically acceptable complexes, or
(f) synthesis of pharmacologically active polymers, or
(g) introduction of hydrophilic moieties, or
(h) introduction/exchange of substituents on aromates or side chains, change of substituent pattern, or
(i) modification by introduction of isosteric or bioisosteric moieties, or
(j) synthesis of homologous compounds, or
(k) introduction of branched side chains, or
(l) conversion of alkyl substituents to cyclic analogues, or
(m) derivatisation of hydroxyl groups to ketales, acetales, or
(n) N-acetylation to amides, phenylcarbamates, or
(o) synthesis of Mannich bases, imines, or
(p) transformation of ketones or aldehydes to Schiffs bases, oximes, acetales, ketales, enolesters, oxazolidines, thiazolidines

or combinations thereof.

13. A method of identifying the cellular pathway targeted by a test compound, the method comprising

(a) treating a cell with the test compound and obtaining a multi-parametric phenotypic profile of the cell after said treatment;
(b) providing a phenotypic profile obtained for the same cell type by introducing directed genetic perturbations to target genes of said cell;
(c) comparing the multi-parametric phenotypic profile obtained in (a) with the phenotypic profile provided in (b) and identifying the genes whose perturbation leads to an identical or substantially identical phenotypic profile as the test compound; and
(d) identifying the cellular pathways involving the genes identified in (c).

14. The method of claim 13, wherein the directed genetic perturbations to target genes of the cell are introduced by use of RNAi.

15. The method of claim 14, wherein the RNAi-mediated genetic perturbation is a genome-wide RNAi screen.

**A**

High content chemical screen to identify modulators
of intracellular mycobacterial survival

↓

Quantitative multiparametric image analysis (QMPIA)

↓

Clustering of multiple parameters,
defining cluster of interest

↓

Validation of selected compounds
for intracellular mycobacterial killing

↓

Comparison of quantitative multiparametric data
with genome wide screen for endocytosis
(through an intermediate screen)

↓

Identification of cellular processes influenced by
compounds

↓

Validation of the identified cellular processes

Figure 1

**B**

a

10 µm

DAPI/Cell Mask Blue
*M. bovis* BCG-GFP

b  Macrophage segmentation

Intensity
1000
300
100

Y (um)  120  130  140  150  160

c  *M. bovis* BCG segmentation

Intensity
1000
300
100
136

Y (um)  127  130  133

c

| Cluster ID | Cluster size | *M. bovis* BCG parameters | | | | | | | | | | | | | | | | | | | Host parameters | | | | | | | | | | | Z score |
| | | Number | | | | | | | | | Intensity | | | | | | | Size | | | Morphology | | | | | | Toxicity | | | | | |
| | | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 | 27 | 28 | 29 | 30 | |
| M.1 | 1034 | | | | | | | | | | | | | | | | | | | | | | | | | | | | | | | | +4 |
| M.2 | 317 | | | | | | | | | | | | | | | | | | | | | | | | | | | | | | | | |
| M.3 | 316 | | | | | | | | | | | | | | | | | | | | | | | | | | | | | | | | 0 |
| M.4 | 147 | | | | | | | | | | | | | | | | | | | | | | | | | | | | | | | | |
| M.5 | 115 | | | | | | | | | | | | | | | | | | | | | | | | | | | | | | | | -4 |
| M.6 | 69 | | | | | | | | | | | | | | | | | | | | | | | | | | | | | | | | |

**Figure 1 continued**

**D**

Figure 1 continued

Figure 2

**F**

**G**

*M. bovis* BCG-GFP with LBPA

D (9676 cells)
H (2453 cells)
N (1923 cells)
PE (2312 cells)

**Figure 2 continued**

Figure 3

**Figure 3 continued**

**Figure 4**

**D**

**E**

**F**

Figure 4 continued

Figure 5

**B**

ChQ(103 cells)
Baf (90 cells)
PE (117 cells)
DMSO(148 cells)
N (90 cells)
Hb (108 cells)

Colocalisation threshold
Colocalisation of mRFP and GFP signals

**C**

Total LTR

Bacterial LTR

PE

PE

DMSO

Time (hours)

**Figure 5 continued**

**D**

**E**

**Figure 5 continued**

Figure 5 continued

Figure 6

Figure 6 continued

**A**

**B**

**Figure 7**

**C**

**D**

## *M. bovis* BCG with LBPA

**Figure 7 continued**

**A**

**B**

MSD library (n=2000)    M5 cluster (n=115)

11 %    5.2 %

Alamar blue (n=114)

44.7 %

□ antibacterial

Figure 8

C

D

**Figure 8 continued**

**E**

**F**

**Figure 8 continued**

Figure 9

**C**

**D**

*M. bovis* BCG-GFP    Lysotracker red    DAPI

**Figure 9 continued**

E

F

NORTRIPTYLINE    AMITRIPTYLINE

**Figure 9 continued**

**G**

Mycobacteria assay

**H**

Endocytosis assay

**Figure 9 continued**

I

**Figure 9 continued**

A

**Figure 10**

**B**

**C**

Figure 10 continued

**D**

**DAPI    LC3**

**E**

**Figure 10 continued**

**Figure 11**

**Figure 11 continued**

**Figure 12**

**Figure 12 continued**

**D**

## Colocalisation LC3-GFP with LBPA

PE (n=846)
Baf (n=908)
N (n=799)
D (n=756)
HBSS (n=772)

**E**

**Figure 12 continued**

**F**

### Colocalisation *M. bovis* BCG-GFP with lysotracker red

N (847 cells)
H89 (977 cells)
PE (507 cells)
D (523 cells)

Colocalisation coefficient

Colocalisation threshold

**Figure 12 continued**

**A**

Net Cargo flow
Cell periphery

Minus end

Plus end

Cell center

Rab5

Rab conversion

LBPA

**Early endosome**
vesicles containing
LDL and Rab5 but NOT LBPA

Late endosome,
**Lysosome**
Vesicles containing
LDL and LBPA but NOT Rab5

**Figure 13**

**B**

**C**

Figure 13 continued

**D**

**E**

Figure 13 continued

**F**

**G**

Figure 13 continued

Europäisches
Patentamt

European
Patent Office

Office européen
des brevets

## EUROPEAN SEARCH REPORT

Application Number

EP 13 15 5069

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2012/154944 A2 (STC UNM [US]; DERETIC VOJO [US]; CASTILLO ELISEO [US]; BRADFUTE STEVEN) 15 November 2012 (2012-11-15) * claims 22-47, 53, 68-69 * * examples 5-6 * ----- | 1-5 | INV. A61K31/135 A61K31/4515 A61K31/5415 A61P31/06 G01N33/569 |
| X | MEINDL W: "ANTIMYKOBAKTERIELLE ANTIHISTAMINIKA//ANTIMYCOBACTERIAL ANTIHISTAMINICS", ARCHIV DER PHARMAZIE, WILEY VERLAG, WEINHEIM, vol. 322, no. 8, 1 January 1989 (1989-01-01), pages 493-497, XP008023938, ISSN: 0365-6233 * table 1; compound 16 * ----- | 1-5 | |
| X | S. RAMON-GARCIA ET AL: "Synergistic Drug Combinations for Tuberculosis Therapy Identified by a Novel High-Throughput Screen", ANTIMICROBIAL AGENTS AND CHEMOTHERAPY, vol. 55, no. 8, 16 May 2011 (2011-05-16), pages 3861-3869, XP055059891, ISSN: 0066-4804, DOI: 10.1128/AAC.00474-11 * tables 2-5; compound haloperidol and bromperidol * * page 3867, right-hand column, last paragraph * ----- -/-- | 1-5 | TECHNICAL FIELDS SEARCHED (IPC) A61K G01N |

~~The present search report has been drawn up for all claims~~

| Place of search | Date of completion of the search | Examiner |
|---|---|---|
| Munich | 23 April 2013 | Renard, Delphine |

EPO FORM 1503 03.82 (P04C01)

Europäisches Patentamt

European Patent Office

Office européen des brevets

**EUROPEAN SEARCH REPORT**

Application Number

EP 13 15 5069

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | SHUBIN H ET AL: "Prochlorperazine (compazine) as an aid in the treatment of pulmonary tuberculosis.", ANTIBIOTIC MEDICINE & CLINICAL THERAPY MAY 1958, vol. 5, no. 5, May 1958 (1958-05), pages 305-309, XP009168870, ISSN: 0570-3107 * Results and discussion * | 1-5 | |
| X | MADRID ET AL: "Synthesis and antitubercular activity of phenothiazines with reduced binding to dopamine and serotonin receptors", BIOORGANIC & MEDICINAL CHEMISTRY LETTERS, PERGAMON, ELSEVIER SCIENCE, GB, vol. 17, no. 11, 10 May 2007 (2007-05-10), pages 3014-3017, XP022068384, ISSN: 0960-894X, DOI: 10.1016/J.BMCL.2007.03.064 * table 1; compounds CPZ-TFP * | 1-5 | |
| X | JAMES C SACCHETTINI ET AL: "Drugs versus bugs: in pursuit of the persistent predator Mycobacterium tuberculosis", NATURE REVIEWS. MICROBIOLOGY, NATURE PUBLISHING GROUP, GB, vol. 6, no. 1, 1 June 2008 (2008-06-01), pages 41-52, XP009168873, ISSN: 1740-1526 * page 50, paragraph * * pages 42-47, paragraph Drug-discovery methods * | 1-5 | TECHNICAL FIELDS SEARCHED (IPC) |

-/--

~~The present search report has been drawn up for all claims~~

| Place of search | Date of completion of the search | Examiner |
|---|---|---|
| Munich | 23 April 2013 | Renard, Delphine |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

Application Number

EP 13 15 5069

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2010/215730 A1 (GUY DAVID [US] ET AL) 26 August 2010 (2010-08-26) * example 1 * * claims 1-12 * * paragraph [0073] * | 2,3 | |
| X | US 2006/039869 A1 (WERMELING DANIEL [US] ET AL) 23 February 2006 (2006-02-23) * paragraphs [0035] - [0037] * * page 1; claims 1-2, 10-14 * | 2,3 | |
| A | Y. LIN ET AL: "Identification of antituberculosis agents that target ribosomal protein interactions using a yeast two-hybrid system", PROCEEDINGS OF THE NATIONAL ACADEMY OF SCIENCES, vol. 109, no. 43, 8 October 2012 (2012-10-08), pages 17412-17417, XP055060094, ISSN: 0027-8424, DOI: 10.1073/pnas.1110271109 * compound T766 * * the whole document * | 1-5 | |

TECHNICAL FIELDS SEARCHED (IPC)

~~The present search report has been drawn up for all claims~~

| Place of search | Date of completion of the search | Examiner |
|---|---|---|
| Munich | 23 April 2013 | Renard, Delphine |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

Europäisches
Patentamt

European
Patent Office

Office européen
des brevets

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

1-5

☐ The present supplementary European search report has been drawn up for those parts of the European patent application which relate to the invention first mentioned in the claims (Rule 164 (1) EPC).

Europäisches
Patentamt

European
Patent Office

Office européen
des brevets

**LACK OF UNITY OF INVENTION
SHEET B**

The Search Division considers that the present European patent application does not comply with the requirements of unity of invention and relates to several inventions or groups of inventions, namely:

1. claims: 1-5

 Nortriptyline, haloperidol or prochlorperazine edisylate for use in the treatment of tuberculosis
 ---

2. claims: 6-12

 Method to identify a compound for use in the treatment of tuberculosis
 ---

3. claims: 13-15

 Method for identifying the cellular pathway targeted by a test compound
 ---

## ANNEX TO THE EUROPEAN SEARCH REPORT
## ON EUROPEAN PATENT APPLICATION NO.

EP 13 15 5069

This annex lists the patent family members relating to the patent documents cited in the above-mentioned European search report.
The members are as contained in the European Patent Office EDP file on
The European Patent Office is in no way liable for these particulars which are merely given for the purpose of information.

23-04-2013

| Patent document cited in search report | | Publication date | Patent family member(s) | | Publication date |
|---|---|---|---|---|---|
| WO 2012154944 | A2 | 15-11-2012 | NONE | | |
| US 2010215730 | A1 | 26-08-2010 | NONE | | |
| US 2006039869 | A1 | 23-02-2006 | US 2006039869 A1 | | 23-02-2006 |
| | | | WO 2006023497 A2 | | 02-03-2006 |

EPO FORM P0459

EP 2 767 275 A1

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 3438991 A
- GB 895309 A
- BE 577977
- US 2902484 A

**Non-patent literature cited in the description**

- **PETERS ; HENNION.** *J. Med. Chem.,* 1964, vol. 7 (3), 390-392
- **OOSTHUIZEN et al.** *Journal of Psychopharmacology,* 2001, vol. 15 (4), 251-5
- **SCHÖN et al.** *Eur Respir J,* 2003, vol. 21, 483-8
- **SCHON et al.** *Tuberculosis (Edinb,* September 2011, vol. 91 (5), 370-7
- **COUSSENS et al.** *PNAS,* 2012, vol. 109 (38), 15449-54
- **BRENK, R. et al.** Lessons Learnt from Assembling Screening Libraries for Drug Discovery for Neglected Diseases. *ChemMedChem,* 2008, vol. 3, 435-444
- **QUINN J.R. et al.** Developing a Drug-like Natural Product Library. *J. Nat. Prod.,* 2008, vol. 71, 464-468
- **COLLINET et al.** *Nature,* 2010
- **GABRIEL, K. R. ; SOKAL, R. R.** A new statistical approach to geographic variation analysis. *Systematic Zoology,* 1969, vol. 18 (3), 259-270
- **MARK NIXON ; ALBERTO S AGUADO.** Feature Extraction & Image Processing for Computer Vision
- **KUBINYI.** Hausch-Analysis and Related Approaches. VCH Verlag, 1992
- **HOLZGRABE ; BECHTOLD.** *Deutsche Apotheker Zeitung,* 2000, vol. 140 (8), 813-823
- **R. FISHER.** On the probable error of correlation coefficient deduced from a small sample set. *Metron,* 1921, vol. 1, 3-32
- **R. FISHER.** Frequency Distribution of the Values of the Correlation Coefficient in Samples from an Indefinitely Large Population. *Biometrika,* 1915, vol. 10 (4), 507-521
- **MAHALANOBIS, P. C.** On the generalised distance in statistics. *Proceedings of the National Institute of Sciences of India,* 1936, vol. 2 (1), 49-55, http://en.wikipedia.org/wiki/Mahalanobis_distance
- **KITTLER, R. et al.** Genome-scale RNAi profiling of cell division in human tissue culture cells. *Nature cell biology,* 2007, vol. 9 (140), 1-12
- **CHENG, Y.** Mean shift, mode seeking, and clustering. *IEEE Transactions on Pattern Analysis and Machine Intelligence,* 1995, vol. 17, 790-799
- **ROUSSEEUW, P. J. SILHOUETTES.** A graphical aid to the interpretation and validation of cluster analysis. *Journal of Computational and Applied Mathematics,* 1987, vol. 20, 53-65
- **WITTKOP, T. et al.** Comprehensive cluster analysis with Transitivity Clustering. *Nature protocols,* 2011, vol. 6, 285-95
- **HARTWELL, L. H. ; HOPFIELD, J. J. ; LEIBLER, S. ; MURRAY, A. W.** From molecular to modular cell biology. *Nature,* 1999, vol. 402, C47-52
- **ALIX, E. ; MUKHERJEE, S. ; ROY, C.R.** Subversion of membrane transport pathways by vacuolar pathogens. *J Cell Biol,* 2011, vol. 195, 943-952
- **ARMSTRONG, J.A. ; HART, P.D.** Response of cultured macrophages to Mycobacterium tuberculosis, with observations on fusion of lysosomes with phagosomes. *J Exp Med,* 1971, vol. 134, 713-740
- **BUCCI, C. ; PARTON, R.G. ; MATHER, I.H. ; STUNNENBERG, H. ; SIMONS, K. ; HOFLACK, B. ; ZERIAL, M.** The small GTPase rab5 functions as a regulatory factor in the early endocytic pathway. *Cell,* 1992, vol. 70, 715-728
- **BEHAR, S.M. ; DIVANGAHI, M. ; REMOLD, H.G.** Evasion of innate immunity by Mycobacterium tuberculosis: is death an exit strategy?. *Nat Rev Microbiol,* 2010, vol. 8, 668-674
- **CASPI, A. ; GRANEK, R. ; ELBAUM, M.** Diffusion and directed motion in cellular transport. *Phys Rev E Stat Nonlin Soft Matter Phys,* 2002, vol. 66, 011916
- **CHEN, M. ; GAN, H. ; REMOLD, H.G.** A mechanism of virulence: virulent Mycobacterium tuberculosis strain H37Rv, but not attenuated H37Ra, causes significant mitochondrial inner membrane disruption in macrophages leading to necrosis. *J Immunol,* 2006, vol. 176, 3707-3716
- **CHRISTOFORIDIS, S. ; MCBRIDE, H.M. ; BURGOYNE, R.D. ; ZERIAL, M.** The Rab5 effector EEA1 is a core component of endosome docking. *Nature,* 1999, vol. 397, 621-625

- **COLLINET, C. ; STOTER, M. ; BRADSHAW, C.R. ; SAMUSIK, N. ; RINK, J.C. ; KENSKI, D. ; HABERMANN, B. ; BUCHHOLZ, F. ; HENSCHEL, R. ; MUELLER, M.S. et al.** Systems survey of endocytosis by multiparametric image analysis. *Nature,* 2010, vol. 464, 243-249
- **DEL CONTE-ZERIAL, P. ; BRUSCH, L. ; RINK, J.C. ; COLLINET, C. ; KALAIDZIDIS, Y. ; ZERIAL, M. ; DEUTSCH, A.** Membrane identity and GTPase cascades regulated by toggle and cut-out switches. *Mol Syst Biol,* 2008, vol. 4, 206
- **DESJARDINS, M. ; HUBER, L.A. ; PARTON, R.G. ; GRIFFITHS, G.** Biogenesis of phagolysosomes proceeds through a sequential series of interactions with the endocytic apparatus. *J Cell Biol,* 1994, vol. 124, 677-688
- **DHANDAYUTHAPANI, S. ; VIA, L.E. ; THOMAS, C.A. ; HOROWITZ, P.M. ; DERETIC, D. ; DERETIC, V.** Green fluorescent protein as a marker for gene expression and cell biology of mycobacterial interactions with macrophages. *Mol Microbiol,* 1995, vol. 17, 901-912
- **DYER, M.D. ; MURALI, T.M. ; SOBRAL, B.W.** The landscape of human proteins interacting with viruses and other pathogens. *PLoS Pathog,* 2008, vol. 4, e32
- **EGGERT, U.S. ; KIGER, A.A. ; RICHTER, C. ; PERLMAN, Z.E. ; PERRIMON, N. ; MITCHISON, T.J. ; FIELD, C.M.** Parallel chemical genetic and genome-wide RNAi screens identify cytokinesis inhibitors and targets. *PLoS Biol,* 2004, vol. 2, e379
- **FAIRN, G.D. ; GRINSTEIN, S.** How nascent phagosomes mature to become phagolysosomes. *Trends Immunol.,* 2012
- **FORET, L. ; DAWSON, J.E. ; VILLASENOR, R. ; COLLINET, C. ; DEUTSCH, A. ; BRUSCH, L. ; ZERIAL, M. ; KALAIDZIDIS, Y. ; JULICHER, F.** A general theoretical framework to infer endosomal network dynamics from quantitative image analysis. *Curr Biol,* 2012, vol. 22, 1381-1390
- **FRATTI, R.A. ; BACKER, J.M. ; GRUENBERG, J. ; CORVERA, S. ; DERETIC, V.** Role of phosphatidylinositol 3-kinase and Rab5 effectors in phagosomal biogenesis and mycobacterial phagosome maturation arrest. *J Cell Biol,* 2001, vol. 154, 631-644
- **GALLUZZI, L. ; AARONSON, S.A. ; ABRAMS, J. ; ALNEMRI, E.S. ; ANDREWS, D.W. ; BAEHRECKE, E.H. ; BAZAN, N.G. ; BLAGOSKLONNY, M.V. ; BLOMGREN, K. ; BORNER, C. et al.** Guidelines for the use and interpretation of assays for monitoring cell death in higher eukaryotes. *Cell Death Differ,* 2009, vol. 16, 1093-1107
- **GUTIERREZ, M.G. ; MASTER, S.S. ; SINGH, S.B. ; TAYLOR, G.A. ; COLOMBO, M.I. ; DERETIC, V.** Autophagy is a defense mechanism inhibiting BCG and Mycobacterium tuberculosis survival in infected macrophages. *Cell,* 2004, vol. 119, 753-766
- **HUANG, R. ; SOUTHALL, N. ; WANG, Y. ; YASGAR, A. ; SHINN, P. ; JADHAV, A. ; NGUYEN, D.T. ; AUSTIN, C.P.** The NCGC pharmaceutical collection: a comprehensive resource of clinically approved drugs enabling repurposing and chemical genomics. *Sci Transl Med,* 2011, vol. 3 (80), 16
- **IMMING, P. ; SINNING, C. ; MEYER, A.** Drugs, their targets and the nature and number of drug targets. *Nat Rev Drug Discov,* 2006, vol. 5, 821-834
- **JAYACHANDRAN, R. ; SUNDARAMURTHY, V. ; COMBALUZIER, B. ; MUELLER, P. ; KORF, H. ; HUYGEN, K. ; MIYAZAKI, T. ; ALBRECHT, I. ; MASSNER, J. ; PIETERS, J.** Survival of mycobacteria in macrophages is mediated by coronin 1-dependent activation of calcineurin. *Cell,* 2007, vol. 130, 37-50
- **JAYASWAL, S. ; KAMAL, M.A. ; DUA, R. ; GUPTA, S. ; MAJUMDAR, T. ; DAS, G. ; KUMAR, D. ; RAO, K.V.** Identification of host-dependent survival factors for intracellular Mycobacterium tuberculosis through an siRNA screen. *PLoS Pathog,* 2010, vol. 6, e1000839
- **KARNOVSKY, M.** Use of ferrocyanide-reduced osmium tetroxide in electron microscopy. *Proceedings of the Eleventh Annual Meeting of the American Society for Cell Biology,* 1971
- **KEANE, J. ; REMOLD, H.G. ; KORNFELD, H.** Virulent Mycobacterium tuberculosis strains evade apoptosis of infected alveolar macrophages. *J Immunol,* 2000, vol. 164, 2016-2020
- **KIMURA, S. ; NODA, T. ; YOSHIMORI, T.** Dissection of the autophagosome maturation process by a novel reporter protein, tandem fluorescent-tagged LC3. *Autophagy,* 2007, vol. 3, 452-460
- **KLIONSKY, D.J. ; ABELIOVICH, H. ; AGOSTINIS, P. ; AGRAWAL, D.K. ; ALIEV, G. ; ASKEW, D.S. ; BABA, M. ; BAEHRECKE, E.H. ; BAHR, B.A. ; BALLABIO, A. et al.** Guidelines for the use and interpretation of assays for monitoring autophagy in higher eukaryotes. *Autophagy,* 2008, vol. 4, 151-175
- **KOBAYASHI, T. ; STANG, E. ; FANG, K.S. ; DE MOERLOOSE, P. ; PARTON, R.G. ; GRUENBERG, J.** A lipid associated with the antiphospholipid syndrome regulates endosome structure and function. *Nature,* 1998, vol. 392, 193-197
- **KOUL, A. ; HERGET, T. ; KLEBL, B. ; ULLRICH, A.** Interplay between mycobacteria and host signalling pathways. *Nat Rev Microbiol,* 2004, vol. 2, 189-202
- **KUIJL, C. ; SAVAGE, N.D. ; MARSMAN, M. ; TUIN, A.W. ; JANSSEN, L. ; EGAN, D.A. ; KETEMA, M. ; VAN DEN NIEUWENDIJK, R. ; VAN DEN EEDEN, S.J. ; GELUK, A. et al.** Intracellular bacterial growth is controlled by a kinase network around PKB/AKT1. *Nature,* 2007, vol. 450, 725-730

- **KUMAR, D. ; NATH, L. ; KAMAL, M.A. ; VARSH-NEY, A. ; JAIN, A. ; SINGH, S. ; RAO, K.V.** Genome-wide analysis of the host intracellular network that regulates survival of Mycobacterium tuberculosis. *Cell,* 2010, vol. 140, 731-743
- **LAPLANTE, M. ; SABATINI, D.M.** mTOR signaling in growth control and disease. *Cell,* 2012, vol. 149, 274-293
- **MATSUZAWA, T. ; KIM, B.H. ; SHENOY, A.R. ; KAMITANI, S. ; MIYAKE, M. ; MACMICKING, J.D.** IFN-gamma elicits macrophage autophagy via the p38 MAPK signaling pathway. *J Immunol,* 2012, vol. 189, 813-818
- **PARVATI K. ; PRAKASA RAO B.S. ; MARIYA DAS M.** Image Segmentation Using Gray-Scale Morphology and Marker-Controlled Watershed Transformation. *Discrete Dynamics in Nature and Society,* 2008
- **PIETERS, J.** Mycobacterium tuberculosis and the macrophage: maintaining a balance. *Cell Host Microbe,* 2008, vol. 3, 399-407
- **PONPUAK, M. ; DAVIS, A.S. ; ROBERTS, E.A. ; DELGADO, M.A. ; DINKINS, C. ; ZHAO, Z. ; VIRGIN, H.W.T. ; KYEI, G.B. ; JOHANSEN, T. ; VERGNE, I. et al.** Delivery of cytosolic components by autophagic adaptor protein p62 endows autophagosomes with unique antimicrobial properties. *Immunity,* 2010, vol. 32, 329-341
- **POSER, I. ; SAROV, M. ; HUTCHINS, J.R. ; HERICHE, J.K. ; TOYODA, Y. ; POZNIAKOVSKY, A. ; WEIGL, D. ; NITZSCHE, A. ; HEGEMANN, B. ; BIRD, A.W. et al.** BAC TransgeneOmics: a high-throughput method for exploration of protein function in mammals. *Nat Methods,* 2008, vol. 5, 409-415
- **REGENTHAL, R. ; KRUEGER, M. ; KOEPPEL, C. ; PREISS, R.** Drug levels: therapeutic and toxic serum/plasma concentrations of common drugs. *J Clin Monit Comput,* 1999, vol. 15, 529-544
- **RINK, J. ; GHIGO, E. ; KALAIDZIDIS, Y. ; ZERIAL, M.** Rab conversion as a mechanism of progression from early to late endosomes. *Cell,* 2005, vol. 122, 735-749
- **ROERDINK, A.M.A.J.B.T.M.** The watershed transform: definitions, algorithms, and parallelization strategies. *Fundamenta Informaticae,* 2000, vol. 41, 187-228
- **ROSSI, M. ; MUNARRIZ, E.R. ; BARTESAGHI, S. ; MILANESE, M. ; DINSDALE, D. ; GUERRA-MARTIN, M.A. ; BAMPTON, E.T. ; GLYNN, P. ; BONANNO, G. ; KNIGHT, R.A. et al.** Desmethylclomipramine induces the accumulation of autophagy markers by blocking autophagic flux. *J Cell Sci,* 2009, vol. 122, 3330-3339
- **RUSSELL, D.G.** Mycobacterium tuberculosis: here today, and here tomorrow. *Nat Rev Mol Cell Biol,* 2001, vol. 2, 569-577
- **SCHMID, S.L. ; CULLIS, P.R.** Membrane sorting. Endosome marker is fat not fiction. *Nature,* 1998, vol. 392, 135-136
- **SCHWEGMANN, A. ; BROMBACHER, F.** Host-directed drug targeting of factors hijacked by pathogens. *Sci Signal,* 2008, vol. 1, re8
- **SEGLEN, P.O. ; GORDON, P.B.** 3-Methyladenine: specific inhibitor of autophagic/lysosomal protein degradation in isolated rat hepatocytes. *Proc Natl Acad Sci U S A,* 1982, vol. 79, 1889-1892
- **SETTEMBRE, C. ; DI MALTA, C. ; POLITO, V.A. ; GARCIA ARENCIBIA, M. ; VETRINI, F. ; ERDIN, S. ; ERDIN, S.U. ; HUYNH, T. ; MEDINA, D. ; COLELLA, P. et al.** TFEB links autophagy to lysosomal biogenesis. *Science,* 2011, vol. 332, 1429-1433
- **SHUBIN, H. ; SHERSON, J. ; PENNES, E. ; GLASKIN, A. ; SOKMENSUER, A.** Prochlorperazine (compazine) as an aid in the treatment of pulmonary tuberculosis. *Antibiotic Med Clin Ther,* 1958, vol. 5, 305-309
- **SIMONSEN, A. ; LIPPE, R. ; CHRISTOFORIDIS, S. ; GAULLIER, J.M. ; BRECH, A. ; CALLAGHAN, J. ; TOH, B.H. ; MURPHY, C. ; ZERIAL, M. ; STENMARK, H.** EEA1 links PI(3)K function to Rab5 regulation of endosome fusion. *Nature,* 1998, vol. 394, 494-498
- **SWINNEY, D.C. ; ANTHONY, J.** How were new medicines discovered?. *Nat Rev Drug Discov,* 2011, vol. 10, 507-519
- **VERGNE, I. ; CHUA, J. ; LEE, H.H. ; LUCAS, M. ; BELISLE, J. ; DERETIC, V.** Mechanism of phagolysosome biogenesis block by viable Mycobacterium tuberculosis. *Proc Natl Acad Sci U S A,* 2005, vol. 102, 4033-4038
- **ZSCHOCKE, J. ; ZIMMERMANN, N. ; BERNING, B. ; GANAL, V. ; HOLSBOER, F. ; REIN, T.** Antidepressant drugs diversely affect autophagy pathways in astrocytes and neurons--dissociation from cholesterol homeostasis. *Neuropsychopharmacology,* 2011, vol. 36, 1754-1768